(19) Europäisches Patentamt
European Patent Office
Office européen des brevets

(11) EP 2 019 093 A1

(12) EUROPEAN PATENT APPLICATION
published in accordance with Art. 153(4) EPC

(43) Date of publication:
**28.01.2009 Bulletin 2009/05**

(21) Application number: **07743622.8**

(22) Date of filing: **18.05.2007**

(51) Int Cl.:
***C07D 233/64*** (2006.01) ***A61K 31/4164*** (2006.01)
***A61K 31/4178*** (2006.01) ***A61K 31/454*** (2006.01)
***A61K 31/513*** (2006.01) ***A61P 25/28*** (2006.01)
***C07D 249/06*** (2006.01) ***C07D 249/08*** (2006.01)
***C07D 401/04*** (2006.01) ***C07D 401/12*** (2006.01)
***C07D 403/10*** (2006.01) ***C07D 405/12*** (2006.01)

(86) International application number:
**PCT/JP2007/060187**

(87) International publication number:
**WO 2007/135969 (29.11.2007 Gazette 2007/48)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **19.05.2006 JP 2006140479**

(71) Applicant: **Eisai R&D Management Co., Ltd.**
**Tokyo 112-8088 (JP)**

(72) Inventors:
- **KIMURA, Teiji**
**Tsukuba-shi, Ibaraki 3002635 (JP)**
- **KAWANO, Koki**
**Tsukuba-shi, Ibaraki 3002635 (JP)**
- **DOI, Eriko**
**Tsukuba-shi, Ibaraki 3002635 (JP)**
- **KITAZAWA, Noritaka**
**Tsukuba-shi, Ibaraki 3002635 (JP)**
- **MIYAGAWA, Takehiko**
**Tsukuba-shi, Ibaraki 3002635 (JP)**
- **SATO, Nobuaki**
**Tsukuba-shi, Ibaraki 3002635 (JP)**
- **KANEKO, Toshihiko**
**Tsukuba-shi, Ibaraki 3002635 (JP)**
- **SHIN, Kogyoku**
**Tsukuba-shi, Ibaraki 3002635 (JP)**
- **ITO, Koichi**
**Tsukuba-shi, Ibaraki 3002635 (JP)**
- **TAKAISHI, Mamoru**
**Tsukuba-shi, Ibaraki 3002635 (JP)**
- **SASAKI, Takeo**
**Tsukuba-shi, Ibaraki 3002635 (JP)**
- **HAGIWARA, Hiroaki**
**Tsukuba-shi, Ibaraki 3002635 (JP)**

(74) Representative: **HOFFMANN EITLE**
**Patent- und Rechtsanwälte**
**Arabellastrasse 4**
**81925 München (DE)**

# (54) UREA TYPE CINNAMIDE DERIVATIVE

(57) Disclosed is a compound represented by the formula (I) below or a pharmacologically acceptable salt thereof. Also disclosed is a use of the compound or salt as a pharmaceutical product.

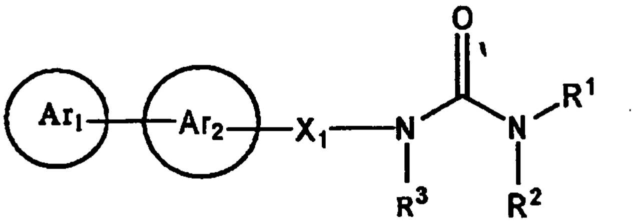 (I)

(In the formula, $Ar_1$ represents an imidazolyl group which may be substituted with a $C_{1-6}$ alkyl group; $Ar_2$ represents a phenyl group which may be substituted with a $C_{1-6}$ alkoxy group; $X_1$ represents a single bond; $R^1$ and $R^2$ respectively

EP 2 019 093 A1

represent a $C_{1-6}$ alkyl group or the like which may be substituted with a substituent such as a 5- to 14-membered aromatic heterocyclic group; and $R^3$ represents a hydrogen atom or the like.)

## Description

### TECHNICAL FIELD

The present invention relates to a pharmaceutical, more particularly, to an amyloid-β (hereinafter referred to as Aβ) production inhibitor effective for treatment of a neurodegenerative disease caused by Aβ such as Alzheimer's disease or Down's syndrome.

### BACKGROUND ART

Alzheimer's disease is a disease characterized by degeneration and loss of neurons as well as formation of senile plaques and neurofibrillary degeneration. Currently, Alzheimer's disease is treated only with symptomatic treatment using a symptom improving agent typified by an acetylcholinesterase inhibitor, and a fundamental remedy to inhibit progression of the disease has not yet been developed. It is necessary to develop a method for controlling the cause of the onset of pathology in order to create a fundamental remedy for Alzheimer's disease.
It is assumed that Aβ-proteins as metabolites of amyloid precursor proteins (hereinafter referred to as APP) are highly involved in degeneration and loss of neurons and onset of symptoms of dementia (see Non-Patent Documents 1 and 2, for example). An Aβ-protein has, as main components, Aβ40 consisting of 40 amino acids and Aβ42 with two amino acids added at the C-terminal. The Aβ40 and Aβ42 are known to have high aggregability (see Non-Patent Document 3, for example) and to be main components of senile plaques (see Non-Patent Documents 3, 4 and 5, for example). Further, it is known that the Aβ40 and Aβ42 are increased by mutation in APP and presenilin genes which is observed in familial Alzheimer's disease (see Non-Patent Documents 6, 7 and 8, for example). Accordingly, a compound that reduces production of Aβ40 and Aβ42 is expected as a progression inhibitor or prophylactic agent for Alzheimer's disease.
Aβ is produced by cleaving APP by β-secretase and subsequently by γ-secretase. For this reason, attempts have been made to create γ-secretase and β-secretase inhibitors in order to reduce Aβ production. Many of these secretase inhibitors already known are, for example, peptides and peptide mimetics such as L-685,458 (see Non-Patent Document 9, for example) and LY-411575 (see Non-Patent Documents 10, 11 and 12, for example).


Non-Patent Document 1: Klein WL, and seven others, Alzheimer's disease-affected brain: Presence of oligomeric Aβ ligands (ADDLs) suggests a molecular basis for reversible memory loss, Proceeding National Academy of Science USA 2003, Sep 2;100(18), p.10417-10422.
Non-Patent Document 2: Nitsch RM, and sixteen others, Antibodies against β-amyloid slow cognitive decline in Alzheimer's disease, Neuron, 2003, May 22;38, p.547-554.
Non-Patent Document 3: Jarrett JT, and two others, The carboxy terminus of the β amyloid protein is critical for the seeding of amyloid formation: Implications for the pathogenesis of Alzheimers' disease, Biochemistry, 1993, 32(18), p.4693-4697.
Non-Patent Document 4: Glenner GG, and another, Alzheimer's disease: initial report of the purification and characterization of a novel cerebrovascular amyloid protein, Biochemical and biophysical research communications, 1984, May 16, 120(3), p.885-890.
Non-Patent Document 5: Masters CL, and five others, Amyloid plaque core protein in Alzheimer disease and Down syndrome, Proceding National Academy of Science USA, 1985 Jun, 82(12), p.4245-4249.
Non-Patent Document 6: Gouras GK, and eleven others, Intraneuronal Aβ42 accumulation in human brain, American Journal of Pathology, 2000, Jan, 156(1), p.15-20.
Non-Patent Document 7: Scheuner D, and twenty others, Secreted amyloid β-protein similar to that in the senile plaques of Alzheimer's disease is increased in vivo by the presenilin 1 and 2 and APP mutations linked to familial Alzheimer's disease, Nature Medicine, 1996, Aug, 2(8), p.864-870.
Non-Patent Document 8: Forman MS, and four others, Differential effects of the swedish mutant amyloid precursor protein on β-amyloid accumulation and secretion in neurons and nonneuronal cells, The Journal of Biological Chemistry, 1997, Dec 19, 272(51), p.32247-32253.
Non-Patent Document 9: Shearman MS, and nine others, L-685,458, an Aspartyl Protease Transition State Mimic, Is a Potent Inhibitor of Amyloid β-Protein Precursor γ-Secretase Activity, Biochemistry, 2000, Aug 1, 39(30), p. 8698-8704.
Non-Patent Document 10: Shearman MS, and six others, Catalytic Site-Directed γ-Secretase Complex Inhibitors Do Not Discriminate Pharmacologically betweeen Notch S3 and β-APP Clevages, Biochemistry, 2003, Jun 24, 42 (24), p.7580-7586.
Non-Patent Document 11: Lanz TA, and three others, Studies of Aβ pharmacodynamics in the brain, cerebrospinal fluid, and plasma in young (plaque-free) Tg2576 mice using the γ-secretase inhibitor N2-[(2S)-2-(3,5-difluorophenyl)-2-hydroxyethanoyl]-N1-[(7S)-5-methyl-6-oxo-6,7-dihydro-5H-dibenzo[b,d]azepin-7-yl]-L-alaninamide (LY-411575),

The journal of pharmacology and experimental therapeutics, 2004, Apr, 309(1), p.49-55.
Non-Patent Document 12: Wong GT, and twelve others, Chronic treatment with the γ-secretase inhibitor LY-411,575 inhibits β-amyloid peptide production and alters lymphopoiesis and intestinal cell differentiation, The journal of biological chemistry, 2004, Mar 26, 279(13), p.12876-12882.

DISCLOSURE OF THE INVENTION

As described above, a compound that inhibits production of Aβ40 and Aβ42 from APP has been expected as a therapeutic or prophylactic agent for a disease caused by Aβ which is typified by Alzheimer's disease. However, a nonpeptidic compound having high efficacy which inhibits production of Aβ40 and Aβ42 has not yet been known. Accordingly, there is a need for a novel low-molecular-weight compound that inhibits production of Aβ40 and Aβ42.
As a result of extensive studies, the present inventors have found a nonpeptidic cinnamide compound that inhibits production of Aβ40 and Aβ42 from APP for the first time, and thus found a prophylactic or therapeutic agent for a disease caused by Aβ which is typified by Alzheimer's disease. This finding has led to the accomplishment of the present invention.
Specifically, the present invention relates to:

1) A compound represented by the formula (I):

[Formula 1]

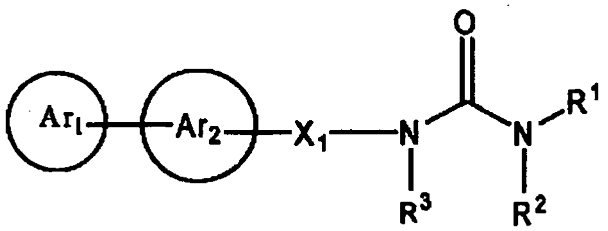

(I)

or a pharmacologically acceptable salt thereof,
wherein $Ar_1$ represents an imidazolyl group or a triazolyl group which may be substituted with 1 to 3 substituents selected from Substituent Group A1 shown below;
$Ar_2$ represents a pyridinyl group or a phenyl group which may be substituted with 1 to 3 substituents selected from Substituent Group A2 shown below;
$X_1$ represents a single bond, a C1-6 alkylene group which may be substituted with 1 to 3 substituents selected from Substituent Group A3 shown below or a C2-6 alkenylene group which may be substituted with 1 to 3 substituents selected from Substituent Group A3 shown below;

(1) $R^1$ and $R^2$ are the same or different and each represent a group selected from Substituent Group A4 shown below; or
(2) $R^1$ and $R^2$, together with a nitrogen atom to which they are bonded, form a 5- to 11-membered heterocyclic group which may be substituted with 1 to 4 substituents selected from Substituent Group A4 and is represented by the formula (II):

[Formula 2]

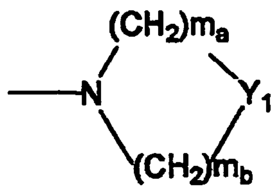

(II)

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-,

(10) $-CR^5=CR^6-$ (wherein $R^5$ and $R^6$ are the same or different and each represent a substituent selected from Substituent Group A4 shown below), (11) a single bond or (12) $>C=CR^{13}R^{14}$ (wherein $R^{13}$ and $R^{14}$ are the same or different and each represent a substituent selected from Substituent Group A4 shown below); and $m_a$ and $m_b$ each represent an integer of 0 to 4; and $R^3$ represents a group selected from Substituent Group A4 shown below, or represents, together with $-N-CO-N-R^2$, a 5- to 8-membered ring group

[Substituent Group A1: (1) a hydrogen atom, (2) a halogen atom and (3) a C1-6 alkyl group (wherein the C1-6 alkyl group may be substituted with 1 to 3 substituents selected from the group consisting of a halogen atom, a hydroxyl group, a cyano group, a C1-6 alkoxy group, a C3-8 cycloalkyl group and a C1-6 alkylcarbonyl group);

Substituent Group A2: (1) a hydrogen atom, (2) a halogen atom, (3) a cyano group and (4) a C1-6 alkoxy group (wherein the C1-6 alkoxy group may be substituted with 1 to 3 substituents selected from the group consisting of a halogen atom, a cyano group, a C1-6 alkoxy group, a C2-6 alkenyl group, a C2-6 alkynyl group and a C3-8 cycloalkyl group);

Substituent Group A3: (1) a hydrogen atom and (2) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4));

Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

2) The compound or pharmacologically acceptable salt thereof according to 1) above, wherein $Ar_1$ is an imidazolyl group or a triazolyl group which may be substituted with 1 or 2 substituents selected from the group consisting of (1) a hydrogen atom and (2) a C1-6 alkyl group (wherein the C1-6 alkyl group may be substituted with 1 to 3 halogen atoms);

3) The compound or pharmacologically acceptable salt thereof according to 2) above, wherein $Ar_1$ is an imidazolyl group which may be substituted with a C1-6 alkyl group;

4) The compound or pharmacologically acceptable salt thereof according to 3) above, wherein $Ar_1$ is an imidazolyl group which may be substituted with a methyl group;

5) The compound or pharmacologically acceptable salt thereof according to 1) above, wherein $Ar_2$ is a phenyl group which may be substituted with 1 to 3 substituents selected from the group consisting of (1) a hydrogen atom, (2) a halogen atom, (3) a cyano group, (4) a C1-6 alkoxy group (wherein the C1-6 alkoxy group may be substituted with 1 to 3 substituents selected from a C2-6 alkenyl group, a C2-6 alkynyl group and a C3-8 cycloalkyl group), (5) a C2-6 alkenyloxy group and (6) a C2-6 alkynyloxy group;

6) The compound or pharmacologically acceptable salt thereof according to 5) above, wherein $Ar_2$ is a phenyl group which may be substituted with 1 to 3 substituents selected from the group consisting of (1) a hydrogen atom, (2) a

halogen atom, (3) a cyano group and (4) a C1-6 alkoxy group;

7) The compound or pharmacologically acceptable salt thereof according to 6) above, wherein $Ar_2$ is a phenyl group which may be substituted with a C1-6 alkoxy group;

8) The compound or pharmacologically acceptable salt thereof according to 7) above, wherein $Ar_2$ is a phenyl group which may be substituted with a methoxy group;

9) The compound or pharmacologically acceptable salt thereof according to 1) above, wherein $X_1$ is a C1-6 alkylene group;

10) The compound or pharmacologically acceptable salt thereof according to 1) above, wherein $X_1$ is a C2-6 alkenylene group;

11) The compound or pharmacologically acceptable salt thereof according to 1) above, wherein $X_1$ is a single bond;

12) The compound or pharmacologically acceptable salt thereof according to 1) above, wherein $R^1$ and $R^2$ are the same or different and each represent a group selected from Substituent Group A4 shown below [Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

13) The compound or pharmacologically acceptable salt thereof according to 12) above, wherein $R^1$ and $R^2$ are the same or different and each represent a group selected from Substituent Group A5 shown below [Substituent Group A5: (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A6, a 5- to 14-membered aromatic heterocyclic group which may be substituted with 1 to 3 substituents selected from Substituent Group A6, a 5-to 14-membered non-aromatic heterocyclic group which may be substituted with 1 to 3 substituents selected from Substituent Group A6 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A6 or a 5- to 14-membered aromatic heterocyclic group which may be substituted with 1 to 3 substituents selected from Substituent Group A6)), (3) a 6- to 14-membered aromatic hydrocarbon ring group which may be substituted with 1 to 3 substituents selected from Substituent Group A6, (4) a 5- to 14-membered aromatic heterocyclic group which may be substituted with 1 to 3 substituents selected from Substituent Group A6 and (5) -X-$A^2$ (wherein X and $A^2$ are as defined above). Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino

group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above)];

14) The compound or pharmacologically acceptable salt thereof according to 13) above, wherein $R^1$ and $R^2$ are the same or different and each represent (1) a hydrogen atom or (2) a C1-6 alkyl group (wherein the C1-6 alkyl group is a hydrogen atom, a C3-8 cycloalkyl group, a C3-8 cycloalkoxy group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7 or -O-$A^4$ (wherein $A^4$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7))

[Substituent Group A7: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A7, (9) -CO-$A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic heterocyclic group which may be substituted with 1 to 3 substituents selected from Substituent Group A7];

15) The compound or pharmacologically acceptable salt thereof according to 1) above, wherein $R^1$ and $R^2$, together with a nitrogen atom to which they are bonded, are a 5- to 11-membered heterocyclic group which may be substituted with 1 to 4 substituents selected from Substituent Group A4 shown below and is represented by the formula (II):

[Formula 3]

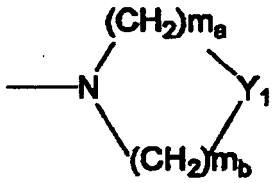
(II)

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ each represent a substituent selected from Substituent Group A4 shown below), (11) a single bond or (12) $>C=CR^{13}R^{14}$ (wherein $R^{13}$ and $R^{14}$ are the same or different and each represent a substituent selected from Substituent Group A4 shown below); and $m_a$ and $m_b$ are the same or different and each represent an integer of 0 to 4

[Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22)

a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

16) The compound or pharmacologically acceptable salt thereof according to 15) above, wherein the 5- to 11-membered heterocyclic group is a piperidinyl group, a pyrrolidinyl group, an azepinyl group, an azocanyl group, a piperazinyl group, a 1,4-diazepanyl group, a morpholinyl group or a thiomorpholinyl group;

17) The compound or pharmacologically acceptable salt thereof according to 16) above, wherein $R^1$ and $R^2$, together with a nitrogen atom to which are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 3 substituents selected from the group consisting of (1) a hydrogen atom, (2) a halogen atom, (3) a hydroxyl group, (4) a formyl group, (5) a hydroxyimino group, (6) a C1-6 alkoxyimino group, (7) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A6 shown below and a 5- to 14-membered aromatic heterocyclic group which may be substituted with 1 to 3 substituents selected from Substituent Group A6 shown below), (8) a 6- to 14-membered aromatic hydrocarbon ring group which may be substituted with 1 to 3 substituents selected from Substituent Group A6 shown below, (9) a 5- to 14-membered aromatic heterocyclic group which may be substituted with 1 to 3 substituents selected from Substituent Group A6 shown below, (10) -O-$A^2$ (wherein $A^2$ represents a 6- to 14-membered aromatic hydrocarbon ring group which may be substituted with 1 to 3 substituents selected from Substituent Group A6 shown below or a 5- to 14-membered aromatic heterocyclic group which may be substituted with 1 to 3 substituents selected from Substituent Group A6 shown below), (11) - CO-$A^2$ (wherein $A^2$ is as defined above) and (12) =CH-$A^2$ (wherein $A^2$ is as defined above)

[Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above)];

18) The compound or pharmacologically acceptable salt thereof according to 17) above, wherein $R^1$ and $R^2$, together with a nitrogen atom to which they are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 4 substituents selected from the group consisting of (1) a hydrogen atom, (2) a halogen atom, (3) a hydroxyl group, (4) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A8 shown below), (5) a 6- to 14-membered aromatic hydrocarbon ring group which may be substituted with 1 to 3 substituents selected from Substituent Group A8 shown below, (6) a 5- to 14-membered aromatic heterocyclic group which may be substituted with 1 to 3 substituents selected from Substituent Group A8 shown below, (7) -O-$A^6$ (wherein $A^6$ represents a 6- to 14-membered aromatic hydrocarbon ring group which may be substituted with 1 to 3 substituents selected from Substituent Group A8 shown below) and (8) =CH-$A^6$ (wherein $A^6$ is as defined above) [Substituent Group A8: (1) a hydrogen atom, (2) a halogen atom, (3) a C1-6 alkyl group (wherein the C1-6 alkyl group may be substituted with 1 to 5 halogen atoms), (4) a C1-6 alkoxy group and (5) a 6- to 14-membered aromatic hydrocarbon

ring group];

19) The compound or pharmacologically acceptable salt thereof according to 12) above, wherein $R^1$ is $-X_{21}-X_{22}-Ar_3$ (wherein $X_{21}$ represents 1) a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group) and a 5- to 14-membered non-aromatic heterocyclic group which may be substituted with 1 to 3 substituents selected from Substituent Group A6) or 2) a single bond; $X_{22}$ represents a single bond, an imino group which may be substituted with a substituent selected from Substituent Group A6, -O- or -S-; and $Ar_3$ represents a 6- to 14-membered aromatic hydrocarbon which may be substituted with 1 to 3 substituents selected from Substituent Group A6 or a 5- to 14-membered aromatic heterocyclic group which may be substituted with 1 to 3 substituents selected from Substituent Group A6)

[Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and $-O-A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) $-CO-A^3$ (wherein $A^3$ is as defined above)];

20) The compound or pharmacologically acceptable salt thereof according to 19) above, wherein $R^1$ is $-X_{21a}-X_{22a}-Ar_{3a}$ (wherein $X_{21a}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms) and a 5- to 14-membered non-aromatic heterocyclic group which may be substituted with 1 to 3 substituents selected from Substituent Group A7); $X_{22a}$ represents a single bond or an oxygen atom; and $Ar_{3a}$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)

[Substituent Group A7: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A7, (9) $-CO-A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic heterocyclic group which may be substituted with 1 to 3 substituents selected from Substituent Group A7];

21) The compound or pharmacologically acceptable salt thereof according to 20) above, wherein $Ar_{3a}$ is a 6- to 14-membered aromatic hydrocarbon ring group selected from the group consisting of a phenyl group, a naphthyl group and a fluorenyl group or a 5- to 14-membered aromatic heterocyclic group selected from the group consisting of a thienyl group, a pyridinyl group, a quinolinyl group, an isoquinolinyl group, an indolyl group, a benzothiazolyl group,

a benzoxazolyl group and a furyl group, which may be substituted with 1 to 3 substituents selected from Substituent Group A7 [Substituent Group A7: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic heterocyclic group which may be substituted with 1 to 3 substituents selected from Substituent Group A7];

22) The compound or pharmacologically acceptable salt thereof according to 1) above, wherein $R^1$ is a 6-to 14-membered non-aromatic hydrocarbon ring group or a 5- to 14-membered non-aromatic heterocyclic group represented by the formula (III):

[Formula 4]

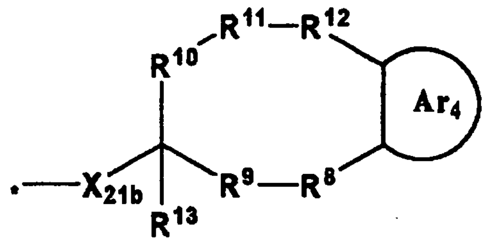

(III)

wherein $R^8$ to $R^{12}$ are the same or different and each represent 1) a single bond, 2) -CO-, 3) a methylene group which may be substituted with 1 or 2 substituents selected from Substituent Group A4, 4) -O-, 5) an imino group which may have a substituent selected from Substituent Group A4 or 6) -S-; $R^{13}$ represents a substituent selected from Substituent Group A9 shown below; $Ar_4$ represents a 6- to 14-membered aromatic hydrocarbon ring group which may be substituted with 1 to 3 substituents selected from Substituent Group A4 shown below or a 5- to 14-membered aromatic heterocyclic group which may be substituted with 1 to 3 substituents selected from Substituent Group A4 shown below; and $X_{21b}$ represents a C1-6 alkylene group [Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above);

Substituent Group A9: (1) a hydrogen atom, (2) a halogen atom, (3) a hydroxyl group, (4) a cyano group, (5) a C3-8 cycloalkyl group, (6) a C2-6 alkenyl group, (7) a C2-6 alkynyl group, (8) a C2-6 alkenyloxy group, (9) a C2-6 alkynyloxy group, (10) a C3-8 cycloalkoxy group, (11) a C3-8 cycloalkylthio group, (12) a C1-6 alkylcarbonyl group, (13) a C1-6

alkylthio group, (14) a C1-6 alkylsulfinyl group, (15) a C3-8 cycloalkylsulfinyl group, (16) a C1-6 alkylsulfonyl group, (17) a C3-8 cycloalkylsulfonyl group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4 and (25) a 5- to 14-membered non-aromatic heterocyclic group which may be substituted with 1 to 3 substituents selected from Substituent Group A4];

23) The compound or pharmacologically acceptable salt thereof according to 22) above, wherein $Ar_4$ is a phenyl group or a 5- to 14-membered aromatic heterocyclic group selected from the group consisting of a pyridinyl group, a pyrimidinyl group, a pyrazinyl group, a thienyl group, an oxazolyl group, a pyrrolyl group, a thiazolyl group and a furyl group, which may be substituted with 1 to 3 substituents selected from the group consisting of a halogen atom, a C1-6 alkyl group (wherein the C1-6 alkyl group may be substituted with 1 to 3 substituents selected from the group consisting of a halogen atom and a C1-6 alkyl group), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A6, a 5- to 14-membered aromatic heterocyclic group which may be substituted with 1 to 3 substituents selected from Substituent Group A6, a 5-to 14-membered non-aromatic heterocyclic group which may be substituted with 1 to 3 substituents selected from Substituent Group A6 and -CO-$A^2$ (wherein $A^2$ represents a 6- to 14-membered aromatic hydrocarbon ring group which may be substituted with 1 to 3 substituents selected from Substituent Group A6 shown below or a 5- to 14-membered aromatic heterocyclic group which may be substituted with 1 to 3 substituents selected from Substituent Group A6 shown below) [Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above)];

24) The compound or pharmacologically acceptable salt thereof according to 23) above, wherein $R^{13}$ is a phenyl group or a pyridinyl group which may be substituted with 1 to 3 substituents selected from Substituent Group A6 [Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above)];

25) The compound or pharmacologically acceptable salt thereof according to 24) above, wherein $R^1$ is an indanyl

group, an azaindanyl group, a tetrahydronaphthyl group, an azatetrahydronaphthyl group, a chromanyl group, an azachromanyl group, a tetrahydrobenzofuranyl group or a tetrahydrobenzothienyl group, which may be substituted with 1 to 3 substituents selected from the group consisting of (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 halogen atoms or C1-6 alkyl groups), (7) a C1-6 alkoxy group (wherein the C1-6 alkoxy group may be substituted with 1 to 3 halogen atoms), (8) an amino group (wherein the amino group may be substituted with a C1-6 alkyl group optionally having 1 to 5 halogen atoms) and (9) a 5- to 14-membered non-aromatic heterocyclic group;

26) The compound or pharmacologically acceptable salt thereof according to 1) above, wherein $R^3$ is a substituent selected from Substituent Group A4;

27) The compound or pharmacologically acceptable salt thereof according to 26) above, wherein $R^3$ is (1) a hydrogen atom or (2) C1-6 alkyl (wherein the C1-6 alkyl group may be substituted with a 6- to 14-membered aromatic hydrocarbon ring group which may be substituted with 1 to 3 substituents selected from Substituent Group A4 or a 5- to 14-membered aromatic heterocyclic group which may be substituted with 1 to 3 substituents selected from Substituent Group A4;

28) The compound or pharmacologically acceptable salt thereof according to 27) above, wherein $R^3$ is (1) a hydrogen atom or (2) C1-6 alkyl;

29) A medicine comprising the compound or pharmacologically acceptable salt thereof according to any one of 1) to 28) above as an active ingredient;

30) A prophylactic or therapeutic agent for a disease caused by Amyloid-β, comprising the compound or pharmacologically acceptable salt thereof according to any one of 1) to 28) above as an active ingredient; and 31) The prophylactic or therapeutic agent according to 30) above, wherein the disease caused by amyloid-β is Alzheimer's disease, dementia, Down's syndrome or amyloidosis.

The compound of the general formula (I) or pharmacologically acceptable salt thereof according to the present invention and the prophylactic or therapeutic agent for a disease caused by Aβ according to the present invention are novel inventions that have not yet been described in any documents.

Meanings of symbols, terms and the like used in the present specification will be explained and the present invention will be described in detail below.

In the present specification, a structural formula of a compound may represent a certain isomer for convenience. However, the present invention includes all isomers and isomer mixtures such as geometric isomers which can be generated from the structure of a compound, optical isomers based on asymmetric carbon, stereoisomers and tautomers. The present invention is not limited to the description of a chemical formula for convenience and may include any one of the isomers or mixtures thereof. Accordingly, the compound of the present invention may have an asymmetric carbon atom in the molecule and exist as an optically active compound or racemate, and the present invention includes each of the optically active compound and the racemate without limitations. Although crystal polymorphs of the compound may be present, the compound is not limited thereto as well and may be present as a single crystal form or a mixture of single crystal forms. The compound may be an anhydride or hydrate.

The "disease caused by Aβ" refers to a wide variety of diseases such as Alzheimer's disease (see Klein WL, and seven others, Alzheimer's disease-affected brain: Presence of oligomeric Aβ ligands (ADDLs) suggests a molecular basis for reversible memory loss, Proceding National Academy of Science USA, 2003, Sep 2, 100(18), p.10417-10422; Nitsch RM, and sixteen others, Antibodies against β-amyloid slow cognitive decline in Alzheimer's disease, Neuron, 2003, May 22, 38(4), p.547-554: Jarrett JT, and two others, The carboxy terminus of the β amyloid protein is critical for the seeding of amyloid formation: Implications for the pathogenesis of Alzheimers' disease, Biochemistry, 1993, May 11, 32(18), p.4693-4697; Glenner GG, and another, Alzheimer's disease; initial report of the purification and characterization of a novel cerebrovascular amyloid protein, Biochemical and biophysical research communications, 1984, May 16, 120(3), p.885-890; Masters CL, and six others, Amyloid plaque core protein in Alzheimer disease and Down syndrome, Proceeding National Academy of Science USA, 1985, June, 82(12), p.4245-4249; Gouras GK, and eleven others, Intraneuronal Aβ42 accumulation in human brain, American journal of pathology, 2000, Jan, 156(1), p.15-20; Scheuner D, and twenty others, Secreted amyloid β-protein similar to that in the senile plaques of Alzheimer's disease is increased in vivo by the presenilin 1 and 2 and APP mutations linked to familial Alzheimer's disease, Nature Medicine, 1996, Aug, 2(8),p.864-870; Forman MS, and four others, Differential effects of the swedish mutant amyloid precursor protein on β-amyloid accumulation and secretion in neurons and nonneuronal cells, The journal of biological chemistry, 1997, Dec 19, 272(51), p.32247-32253, for example), senile dementia (see Blass JP, Brain metabolism and brain disease: Is metabolic deficiency the proximate cause of Alzheimer dementia? Journal of Neuroscience Research, 2001, Dec 1, 66 (5), p.851-856, for example), frontotemporal dementia (see Evin G, and eleven others, Alternative transcripts of presenilin-1 associated with frontotemporal dementia, Neuroreport, 2002, Apr 16, 13(5), p.719-723, for example), Pick's disease (see Yasuhara O, and three others, Accumulation of amyloid precursor protein in brain lesions of patients with Pick

disease, Neuroscience Letters, 1994, Apr 25, 171(1-2), p.63-66, for example), Down's syndrome (see Teller JK, and ten others, Presence of soluble amyloid β-peptide precedes amyloid plaque formation in Down's syndrome, Nature Medicine, 1996, Jan, 2(1), p.93-95;Tokuda T, and six others, Plasma levels of amyloid β proteins Aβ1-40 and Aβ1-42 (43) are elevated in Down's syndrome, Annals of Neurology, 1997, Feb, 41(2), p.271-273, for example), cerebral angiopathy (see Hayashi Y, and nine others, Evidence for presenilin-1 involvement in amyloid angiopathy in the Alzheimer's disease-affected brain, Brain Research, 1998, Apr 13, 789(2), p.307-314; Barelli H, and fifteen others, Characterization of new polyclonal antibodies specific for 40 and 42 amino acid-long amyloid β peptides: their use to examine the cell biology of presenilins and the immunohistochemistry of sporadic Alzheimer's disease and cerebral amyloid angiopathy cases, Molecular Medicine, 1997, Oct, 3(10), p.695-707; Calhoun ME, and ten others, Neuronal overexpression of mutant amyloid precursor protein results in prominent deposition of cerebrovascular amyloid, Proceeding National Academy of Science USA, 1999, Nov 23, 96(24), p.14088-14093; Dermaut B, and ten others, Cerebral amyloid angiopathy is a pathogenic lesion in Alzheimer's Disease due to a novel presenilin-1 mutation, Brain, 2001, Dec, 124(12), p. 2383-2392, for example), hereditary cerebral hemorrhage with amyloidosis (Dutch type) (see Cras P, and nine others, Presenile Alzheimer dementia characterized by amyloid angiopathy and large amyloid core type senile plaques in the APP 692Ala-->Gly mutation, Acta Neuropathologica(Berl), 1998, Sep, 96(3), p.253-260; Herzig MC, and fourteen others, Aβ is targeted to the vasculature in a mouse model of hereditary cerebral hemorrhage with amyloidosis, Nature Neuroscience, 2004, Sep, 7(9), p.954-960; van Duinen SG, and five others, Hereditary cerebral hemorrhage with amyloidosis in patients of Dutch origin is related to Alzheimer disease, Proceeding National Academy of Science USA, 1987, Aug, 84(16), p.5991-5994; Levy E, and eight others, Mutation of the Alzheimer's disease amyloid gene in hereditary cerebral hemorrhage, Dutch type, Science, 1990, Jun 1, 248(4959), p.1124-1126, for example), cognitive impairment (see Laws SM, and seven others, Association between the presenilin-1 mutation Glu318Gly and complaints of memory impairment, Neurobiology of Aging, 2002, Jan-Feb, 23(1), p.55-58, for example), dysmnesia and learning disability (see Vaucher E, and five others, Object recognition memory and cholinergic parameters in mice expressing human presenilin 1 transgenes, Experimental Neurology, 2002 Jun, 175(2), p.398-406; Morgan D, and fourteen others, Aβ peptide vaccination prevents memory loss in an animal model of Alzheimer's disease, Nature, 2000 Dec 21-28, 408(6815), p.982-985; Moran PM, and three others, Age-related learning deficits in transgenic mice expressing the 751-amino acid isoform of human β-amyloid precursor protein, Proceeding National Academy of Science USA, 1995, June 6, 92(12), p.5341-5345, for example), amyloidosis, cerebral ischemia (see Laws SM, and seven others, Association between the presenilin-1 mutation Glu318Gly and complaints of memory impairment, Neurobiology of Aging, 2002, Jan-Feb, 23(1), p.55-58; Koistinaho M, and ten others, β-amyloid precursor protein transgenic mice that harbor diffuse Aβ deposits but do not form plaques show increased ischemic vulnerability: Role of inflammation, Proceeding National Academy of Science USA, 2002, Feb 5, 99(3), p.1610-1615; Zhang F, and four others, Increased susceptibility to ischemic brain damage in transgenic mice overexpressing the amyloid precursor protein, The journal of neuroscience, 1997, Oct 15, 17(20), p. 7655-7661, for example), vascular dementia (see Sadowski M, and six others, Links between the pathology of Alzheimer's disease and vascular dementia, Neurochemical Research, 2004, Jun, 29(6), p.1257-1266, for example), ophthalmoplegia (see O'Riordan S, and seven others, Presenilin-1 mutation(E280G), spastic paraparesis, and cranial MRI white-matter abnormalities, Neurology, 2002, Oct 8, 59(7), p.1108-1110, for example), multiple sclerosis (see Gehrmann J, and four others, Amyloid precursor protein (APP) expression in multiple sclerosis lesions, Glia, 1995, Oct, 15(2), p.141-51; Reynolds WF, and six others, Myeloperoxidase polymorphism is associated with gender specific risk for Alzheimer's disease, Experimental Neurology, 1999, Jan, 155(1), p.31-41, for example), head injury, skull injury (see Smith DH, and four others, Protein accumulation in traumatic brain injury, NeuroMolecular Medicine, 2003, 4(1-2), p.59-72, for example), apraxia (see Matsubara-Tsutsui M, and seven others, Molecular evidence of presenilin 1 mutation in familial early onset dementia, American journal of Medical Genetics, 2002, Apr 8, 114(3), p.292-298, for example), prion disease, familial amyloid neuropathy, triplet repeat disease (see Kirkitadze MD, and two others, Paradigm shifts in Alzheimer's disease and other neurodegenerative disorders: the emerging role of oligomeric assemblies, Journal of Neuroscience Research, 2002, Sep 1, 69(5), p.567-577; Evert BO, and eight others, Inflammatory genes are upreglulated in expanded ataxin-3-expressing cell lines and spinocerebellar ataxia type 3 brains, The Journal of Neuroscience, 2001, Aug 1, 21(15), p. 5389-5396; Mann DM, and another, Deposition of amyloid(A4) protein within the brains of persons with dementing disorders other than Alzheimer's disease and Down's syndrome, Neuroscience Letters, 1990, Feb 5, 109(1-2), p.68-75, for example), Parkinson's disease (see Primavera J, and four others, Brain accumulation of amyloid-β in Non-Alzheimer Neurodegeneration, Jornal of Alzheimer's Disease, 1999, Oct, 1(3), p.183-193, for example), Lewy body dementia (see Giasson BI, and two others, Interactions of amyloidogenic proteins. NeuroMolecular Medicine, 2003, 4(1-2), p.49-58; Masliah E, and six others, β-amyloid peptides enhance α-synuclein accumulation and neuronal deficits in a trancgenic mouse model linking Alzheimer's disease and Parkinson's disease, Proceeding National Academy of Science USA, 2001, Oct 9, 98(21), p.12245-12250; Barrachina M, and six others, Amyloid-β deposition in the cerebral cortex in Dementia with Lewy bodies is accompanied by a relative increase in AβPP mRNA isoforms containing the Kunitz protease inhibitor, Neurochemistry International, 2005, Feb, 46(3), p.253-260; Primavera J, and four others, Brain accumulation of amyloid-β in Non-Alzheimer Neurodegeneration, Jornal of Alzheimer's Disease, 1999, Oct, 1(3), p.183-193, for example), parkinsonism-dementia complex (see Schmidt ML, and six others, Amyloid plaques in Guam amyotrophic lateral sclerosis/ parkinsonism-dementia complex contain species of Aβ similar to those found in the amyloid plaques of Alzheimer's disease and pathological aging, Acta Neuropathologica (Berl), 1998, Feb, 95(2), p.117-122; Ito H, and three others, Demonstration of β amyloid protein-containing neurofibrillary tangles in parkinsonism-dementia complex on Guam, Neuropathology and applied neurobiology, 1991, Oct, 17(5), p. 365-373, for example), frontotemporal dementia and parkinsonism linked to chromosome 17 (see Rosso SM, and three others, Coexistent tau andamyloid pathology in hereditary frontotemporal dementia with tau mutations, Annals of the New York academy of sciences, 2000, 920, p. 115-119, for example), argyrophilic grain dementia (see Tolnay M, and four others, Low amyloid(Aβ) plaque load and relative predominance of diffuse plaques distinguish argyrophilic grain disease from Alzheimer's disease, Neuropathology and applied neurobiology, 1999, Aug, 25(4), p.295-305, for example), Niemann-Pick disease (see Jin LW, and three others, Intracellular accumulation of amyloidogenic fragments of amyloid-β precursor protein in neurons with Niemann-Pick type C defects is associated with endosomal abnormalities, American Journal-of Pathology, 2004, Mar, 164(3), p. 975-985, for example), amyotrophic lateral sclerosis (see Sasaki S, and another, Immunoreactivity of β-amyloid precursor protein in amyotrophic lateral sclerosis, Acta Neuropathologica(Berl), 1999, May, 97(5), p.463-468; Tamaoka A, and four others, Increased amyloid β protein in the skin of patients with amyotrophic lateral sclerosis, Journal of neurology, 2000, Aug, 247(8), p.633-635; Hamilton RL, and another, Alzheimer disease pathology in amyotrophic lateral sclerosis, Acta Neuropathologica, 2004, Jun, 107(6), p.515-522; Turner BJ, and six others, Brain P-amyloidaccumulation in transgenic mice expressing mutant superoxide dismutase 1, Neurochemical Research, 2004, Dec, 29(12), p.2281-2286, for example), hydrocephalus (see Weller RO, Pathology of cerebrospinal fluid and interstitial fluid of the CNS: Significance for Alzheimer disease, prion disorders and multiple sclerosis, Journal of Neuropathology and Experimental Neurology, 1998, Oct, 57(10), p.885-894; Silverberg GD, and four others, Alzheimer's disease, normal-pressure hydrocephalus, and senescent changes in CSF circulatory physiology: a hypothesis, Lancet neurology, 2003, Aug, 2(8), p.506-511; Weller RO, and three others, Cerebral amyloid angiopathy: Accumulation of Aβ in interstitial fluid drainage pathways in Alzheimer's disease, Annals of the New York academy of sciences, 2000, Apr, 903, p.110-117; Yow HY, and another, A role for cerebrovascular disease in determining the pattern of β-amyloid deposition in Alzheimer's disease, Neurology and applied neurobiology, 2002, 28, p.149; Weller RO, and four others, Cerebrovasculardisease is a major factor in the failure of elimination of Aβ from the aging human brain, Annals of the New York academy of sciences, 2002, Nov, 977, p.162-168, for example), paraparesis (see O'Riordan S, and seven others, Presenilin-1 mutation(E280G), spastic paraparesis, and cranial MRI white-matter abnormalities, Neurology, 2002, Oct 8, 59(7), p.1108-1110; Matsubara-Tsutsui M, and seven others, Molecular evidence of presenilin 1 mutation in familial early onset dementia, American journal of Medical Genetics, 2002, Apr 8, 114(3), p.292-298; Smith MJ, and eleven others, Variable phenotype of Alzheimer's disease with spastic paraparesis, Annals of Neurology, 2001, 49(1), p.125-129; Crook R, and seventeen others, A variant of Alzheimer's disease with spastic pararesis and unusual plaques due to deletion of exon 9 of presenilin 1, Nature Medicine, 1998, Apr;4(4), p.452-455, for example), progressive supranuclear palsy (see Barrachina M, and six others, Amyloid-β deposition in the cerebral cortex in Dementia with Lewy bodies is accompanied by a relative increase in AβPP mRNA isoforms containing the Kunitz protease inhibitor, Neurochemistry International, 2005, Feb, 46(3), p.253-260; Primavera J, and four others, Brain accumulation of amyloid-β in Non-Alzheimer Neurodegeneration, Jornal of Alzheimer's Disease, 1999, Oct, 1(3), p.183-193, for example), intracerebral hemorrhage (see Atwood CS, and three others, Cerebrovascular requirement for sealant, anti-coagulant and remodeling molecules that allow for the maintenance of vascular integrity and blood supply, Brain Research Reviews, 2003, Sep, 43(1), p.164-78; Lowenson JD, and two others, Protein aging: Extracellular amyloid formation and intracellular repair, Trends in cardiovascular medicine, 1994, 4 (1), p. 3-8, for example), convulsion (see Singleton AB, and thirteen others, Pathology of early-onset Alzheimer's disease cases bearing the Thr113-114ins presenilin-1 mutation, Brain, 2000, Dec, 123(Pt12), p.2467-2474, for example), mild cognitive impairment (see Gattaz WF, and four others, Platelet phospholipase A2 activity in Alzheimer's disease and mild cognitive impairment, Journal of Neural Transmission, 2004, May, 111(5), p.591-601; Assini A, and fourteen others, Plasma levels of amyloid β-protein 42 are increased in women with mild cognitive impariment, Neurology, 2004, Sep 14, 63(5), p. 828-831, for example), arteriosclerosis (see De Meyer GR, and eight others, Platelet phagocytosis and processing of β-amyloid precursor protein as a mechanism of macrophage activation in atherosclerosis, Circulation Research, 2002, Jun 14, 90(11), p.1197-1204, for example).

The "6- to 14-membered cyclic aromatic hydrocarbon ring group", "5- to 14-membered aromatic heterocyclic group", "6- to 14-membered non-aromatic hydrocarbon ring group" and "5- to 14-membered non-aromatic heterocyclic group" in the formula (I) which are contained in the therapeutic or prophylactic agent for a disease caused by Aβ according to the present invention have the following meanings.

The "6- to 14-membered cyclic aromatic hydrocarbon ring group" refers to a monocyclic, bicyclic or tricyclic aromatic hydrocarbon group having 6 to 14 carbon atoms. Preferable examples of the group include 6- to 14-membered monocyclic, bicyclic or tricyclic aromatic hydrocarbon groups such as a phenyl group, an indenyl group, a naphthyl group, an azulenyl group, a heptalenyl group, a biphenyl group, a fluorenyl group, a phenalenyl group, a phenanthrenyl group and an anthracenyl group.

The "5- to 14-membered aromatic heterocyclic group" refers to a monocyclic, bicyclic or tricyclic aromatic heterocyclic group having 5 to 14 carbon atoms. Preferable examples of the group include (1) nitrogen-containing aromatic heterocyclic groups such as a pyrrolyl group, a pyridyl group, a pyridazinyl group, a pyrimidinyl group, a pyrazinyl group, a pyrazolinyl group, an imidazolyl group, an indolyl group, an isoindolyl group, an indolizinyl group, a purinyl group, an indazolyl group, a quinolyl group, an isoquinolyl group, a quinolizinyl group, a phthalazinyl group, a naphthyridinyl group, a quinoxalinyl group, a quinazolinyl group, a cinnolinyl group, a pteridinyl group, an imidazotriazinyl group, a pyrazinopyridazinyl group, an acridinyl group, a phenanthridinyl group, a carbazolyl group, a perimidinyl group, a phenanthrolinyl group and a phenacyl group, (2) sulfur-containing aromatic heterocyclic groups such as a thienyl group and a benzothienyl group, (3) oxygen-containing aromatic heterocyclic groups such as a furyl group, a pyranyl group, a cyclopentapyranyl group, a benzofuranyl group and an isobenzofuranyl group and (4) aromatic heterocyclic groups containing two or more hetero atoms selected from the group consisting of a nitrogen atom, a sulfur atom and an oxygen atom such as a thiazolyl group, an isothiazolyl group, a benzothiazolinyl group, a benzothiadiazolyl group, a phenothiazinyl group, an isoxazolyl group, a furazanyl group, a phenoxazinyl group, a pyrazoloxazolyl group, an imidazothiazolyl group, a thienofuryl group, a furopyrrolyl group and a pyridooxazinyl group.

The "6- to 14-membered non-aromatic hydrocarbon ring group" refers to a cyclic aliphatic hydrocarbon group having 6 to 14 carbon atoms. Examples of the group include cyclic aliphatic hydrocarbon groups having 6 to 14 carbon atoms such as a cyclopropyl group, a cyclobutyl group, a cyclopentyl group, a cyclohexyl group, a cycloheptyl group, a cyclooctyl group, a spiro[3.4]octanyl group, a decanyl group, an indanyl group, a 1-acenaphthenyl group, a cyclopentacyclooctenyl group, a benzocyclooctenyl group, an indenyl group, a tetrahydronaphthyl group, a 6,7,8,9-tetrahydro-5H-benzocycloheptenyl group and a 1,4-dihydronaphthalenyl group.

The "5- to 14-membered non-aromatic heterocyclic group" 1) has 5 to 14 ring-forming atoms, 2) contains 1 to 5 hetero atoms such as a nitrogen atom, -O- or -S- in the ring-forming atoms, and 3) may contain one or more carbonyl groups, double bonds or triple bonds in the ring, and refers not only to a 5-to 14-membered non-aromatic monocyclic heterocyclic group but also to a saturated heterocyclic group condensed with an aromatic hydrocarbon ring group or a saturated hydrocarbon ring group or saturated heterocyclic group condensed with an aromatic heterocyclic group. Specific examples of the 5- to 14-membered non-aromatic heterocyclic group include an azetidinyl ring, a pyrrolidinyl ring, a piperidinyl ring, an azepanyl ring, an azocanyl ring, a tetrahydrofuranyl ring, a tetrahydropyranyl ring, a morpholinyl ring, a thiomorpholinyl ring, a piperazinyl ring, a thiazolidinyl ring, a dioxanyl ring, an imidazolinyl ring, a thiazolinyl ring, a 1,2-benzopyranyl ring, an isochromanyl ring, a chromanyl ring, an indolinyl ring, an isoindolinyl ring, an azaindanyl group, an azatetrahydronaphthyl group, an azachromanyl group, a tetrahydrobenzofuranyl group, a tetrahydrobenzothienyl group, a 2,3,4,5-tetrahydrobenzo[b]thienyl group, a 3,4-dihydro-2H-benzo[b][1,4]dioxepinyl group, an indan-1-onyl group, a 6,7-dihydro-5H-cyclopentapyrazinyl group, a 6,7-dihydro-5H-[1]pyridinyl group, a 6,7-dihydro-5H-[1]pyridinyl group, a 5,6-dihydro-4H-cyclopenta[b]thienyl group, a 4,5,6,7-tetrahydrobenzo[b]thienyl group, a 3,4-dihydro-2H-naphthale-1-onyl group, a 2,3-dihydro-isoindol-1-onyl group, a 3,4-dihydro-2H-isoquinolin-1-onyl group and a 3,4-dihydro-2H-benzo[1,4]oxapinyl group.

Substituent Group A1, Substituent Group A2, Substituent Group A3, Substituent Group A4, Substituent Group A5, Substituent Group A6, Substituent Group A7, Substituent Group A8 and Substituent Group A9 refer to the following groups.

Substituent Group A1 refers to (1) a hydrogen atom, (2) a halogen atom and (3) a C1-6 alkyl group (wherein the C1-6 alkyl group may be substituted with 1 to 3 substituents selected from the group consisting of a halogen atom, a hydroxyl group, a cyano group, a C1-6 alkoxy group, a C3-8 cycloalkyl group and a C1-6 alkylcarbonyl group).

Substituent Group A2 refers to (1) a hydrogen atom, (2) a halogen atom, (3) a cyano group and (4) a C1-6 alkoxy group (wherein the C1-6 alkoxy group may be substituted with 1 to 3 substituents selected from the group consisting of a halogen atom, a cyano group, a C1-6 alkoxy group, a C2-6 alkenyl group, a C2-6 alkynyl group and a C3-8 cycloalkyl group).

Substituent Group A3 refers to (1) a hydrogen atom and (2) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6-to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4)).

Substituent Group A4 refers to (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 to 2 substituents selected from Substituent Group A4, (21) a carbamoyl group which may be substituted with 1 to 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

Substituent Group A5 refers to (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A6, a 5- to 14-membered aromatic heterocyclic group which may be substituted with 1 to 3 substituents selected from Substituent Group A6, a 5- to 14-membered non-aromatic heterocyclic group which may be substituted with 1 to 3 substituents selected from Substituent Group A6 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A6 or a 5-to 14-membered aromatic heterocyclic group which may be substituted with 1 to 3 substituents selected from Substituent Group A6)), (3) a 6- to 14-membered aromatic hydrocarbon ring group which may be substituted with 1 to 3 substituents selected from Substituent Group A6, (4) a 5- to 14-membered aromatic heterocyclic group which may be substituted with 1 to 3 substituents selected from Substituent Group A6 and (5) -X-$A^2$ (wherein X and $A^2$ are as defined above).

Substituent Group A6 refers to (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6-to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above).

Substituent Group A7 refers to (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A7, (9) -CO-$A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic

heterocyclic group which may be substituted with 1 to 3 substituents selected from Substituent Group A7.

Substituent Group A8 refers to (1) a hydrogen atom, (2) a halogen atom, (3) a C1-6 alkyl group (wherein the C1-6 alkyl group may be substituted with 1 to 5 halogen atoms), (4) a C1-6 alkoxy group and (5) a 6- to 14-membered aromatic hydrocarbon ring group.

Substituent Group A9 refers to (1) a hydrogen atom, (2) a halogen atom, (3) a hydroxyl group, (4) a cyano group, (5) a C3-8 cycloalkyl group, (6) a C2-6 alkenyl group, (7) a C2-6 alkynyl group, (8) a C2-6 alkenyloxy group, (9) a C2-6 alkynyloxy group, (10) a C3-8 cycloalkoxy group, (11) a C3-8 cycloalkylthio group, (12) a C1-6 alkylcarbonyl group, (13) a C1-6 alkylthio group, (14) a C1-6 alkylsulfinyl group, (15) a C3-8 cycloalkylsulfinyl group, (16) a C1-6 alkylsulfonyl group, (17) a C3-8 cycloalkylsulfonyl group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4 and (25) a 5- to 14-membered non-aromatic heterocyclic group which may be substituted with 1 to 3 substituents selected from Substituent Group A4.

The "halogen atom" refers to a fluorine atom, a chlorine atom, a bromine atom, an iodine atom or the like and is preferably a fluorine atom, a chlorine atom or a bromine atom.

The "C1-6 alkyl group" refers to an alkyl group having 1 to 6 carbon atoms. Preferable examples of the group include linear or branched alkyl groups such as a methyl group, an ethyl group, an n-propyl group, an i-propyl group, an n-butyl group, an i-butyl group, a tert-butyl group, an n-pentyl group, an i-pentyl group, a neopentyl group, an n-hexyl group, a 1-methylpropyl group, an 1,2-dimethylpropyl group, a 1-ethylpropyl group, a 1-methyl-2-ethylpropyl group, a 1-ethyl-2-methylpropyl group, a 1,1,2-trimethylpropyl group, a 1-methylbutyl group, a 2-methylbutyl group, a 1,1-dimethylbutyl group, a 2,2-dimethylbutyl group, a 2-ethylbutyl group, a 1,3-dimethylbutyl group, a 2-methylpentyl group and a 3-methylpentyl group.

The "C1-6 alkoxy group" refers to an alkyl group having 1 to 6 carbon atoms in which a hydrogen atom is replaced by an oxygen atom. Preferable examples of the group include a methoxy group, an ethoxy group, an n-propoxy group, an i-propoxy group, an n-butoxy group, an i-butoxy group, a sec-butoxy group, a tert-butoxy group, an n-pentoxy group, an i-pentoxy group, a sec-pentoxy group, a tert-pentoxy group, an n-hexoxy group, an i-hexoxy group, a 1,2-dimethylpropoxy group, a 2-ethylpropoxy group, a 1-methyl-2-ethylpropoxy group, a 1-ethyl-2-methylpropoxy group, a 1,1,2-trimethylpropoxy group, a 1,1,2-trimethylpropoxy group, a 1,1-dimethylbutoxy group, a 2,2-dimethylbutoxy group, a 2-ethylbutoxy group, a 1,3-dimethylbutoxy group, a 2-methylpentoxy group, a 3-methylpentoxy group and a hexyloxy group.

The "C1-6 alkylsulfonyl group" refers to an alkyl group having 1 to 6 carbon atoms in which one hydrogen atom is replaced by a sulfonyl group. Preferable examples of the group include a methanesulfonyl group and an ethanesulfonyl group.

The "amino group which may be substituted with a C1-6 alkyl group" refers to an amino group which may be substituted with an alkyl group having 1 to 6 carbon atoms. Preferable examples of the group include an amino group, a methylamino group, an ethylamino group, a propylamino group and a dimethylamino group.

The "C2-6 alkenyl group" refers to an alkenyl group having 2 to 6 carbon atoms. Preferable examples of the group include linear or branched alkenyl groups such as a vinyl group, an allyl group, a 1-propenyl group, an isopropenyl group, a 1-buten-1-yl group, a 1-buten-2-yl group, a 1-buten-3-yl group, a 2-buten-1-yl group and a 2-buten-2-yl group.

The "C2-6 alkynyl group" refers to an alkynyl group having 2 to 6 carbon atoms. Preferable examples of the group include linear or branched alkynyl groups such as an ethynyl group, a 1-propynyl group, a 2-propynyl group, a butynyl group, a pentynyl group and a hexynyl group.

The "C3-8 cycloalkyl group" refers to a cyclic alkyl group having 3 to 8 carbon atoms. Preferable examples of the group include a cyclopropyl group, a cyclobutyl group, a cyclopentyl group, a cyclohexyl group, a cycloheptyl group and a cyclooctyl group.

The "C1-6 alkylthio group" refers to an alkyl group having 1 to 6 carbon atoms in which one hydrogen atom is replaced by a sulfur atom. Preferable examples of the group include a methylthio group, an ethylthio group, an n-propylthio group, an i-propylthio group, an n-butylthio group, an i-butylthio group, a tert-butylthio group, an n-pentylthio group, an i-pentylthio group, a neopentylthio group, an n-hexylthio group and a 1-methylpropylthio group.

The "C1-6 alkylsulfinyl group" refers to an alkyl group having 1 to 6 carbon atoms in which one hydrogen atom is replaced by a sulfinyl group. Preferable examples of the group include a methylsulfinyl group, an ethylmethylsulfinyl group, an n-propylsulfinyl group, an i-propylsulfinyl group, an n-butylsulfinyl group, an i-butylsulfinyl group, a tert-butylsulfinyl group, an n-pentylsulfinyl group, an i-pentylsulfinyl group, a neopentylsulfinyl group, an n-hexylsulfinyl group and

a 1-methylpropylsulfinyl group.

The "C1-6 alkylcarbonyl group" refers to an alkyl group having 1 to 6 carbon atoms in which one hydrogen atom is replaced by a carbonyl group. Preferable examples of the group include an acetyl group, a propionyl group and a butyryl group.

The "C3-8 cycloalkoxy group" refers to a cyclic alkyl group having 3 to 8 carbon atoms in which one hydrogen atom is replaced by an oxygen atom. Preferable examples of the group include a cyclopropoxy group, a cyclobutoxy group, a cyclopentoxy group, a cyclohexoxy group, a cycloheptyloxy group and a cyclooctyloxy group.

The "C3-8 cycloalkylthio group" refers to a cyclic alkyl group having 3 to 8 carbon atoms in which one hydrogen atom is replaced by a sulfur atom. Preferable examples of the group include a cyclopropylthio group, a cyclobutylthio group, a cyclopentylthio group, a cyclohexylthio group, a cycloheptylthio group and a cyclooctylthio group.

The "C1-6 alkoxyimino group" refers to an imino group in which a hydrogen atom is replaced by a C1-6 alkoxy group. Preferable examples of the group include a methoxyimino group and an ethoxyimino group.

The "C2-6 alkenyloxy group" refers to an alkenyl group having 2 to 6 carbon atoms in which one hydrogen atom is replaced by an oxygen atom. Preferable examples of the group include linear or branched alkenyloxy groups such as a vinyloxy group, an allyloxy group, a 1-propenyloxy group, an isopropenyloxy group, a 1-buten-1-yloxy group, a 1-buten-2-yloxy group, a 1-buten-3-yloxy group, a 2-buten-1-yloxy group and a 2-buten-2-yloxy group.

The "C2-6 alkynyloxy group" refers to an alkynyl group having 2 to 6 carbon atoms in which one hydrogen atom is replaced by an oxygen atom. Preferable examples of the group include linear or branched alkynyloxy groups such as an ethynyloxy group, a 1-propynyloxy group, a 2-propynyloxy group, a butynyloxy group, a pentynyloxy group and a hexynyloxy group.

The "C3-8 cycloalkylsulfinyl group" refers to a cyclic alkyl group having 3 to 8 carbon atoms in which one hydrogen atom is replaced by a sulfinyl group. Preferable examples of the group include a cyclopropylsulfinyl group, a cyclobutylsulfinyl group, a cyclopentylsulfinyl group, a cyclohexylsulfinyl group, a cycloheptylsulfinyl group and a cyclooctylsulfinyl group.

The "C3-8 cycloalkylsulfonyl group" refers to a cyclic alkyl group having 3 to 8 carbon atoms in which one hydrogen atom is replaced by a sulfonyl group. Preferable examples of the group include a cyclopropylsulfonyl group, a cyclobutylsulfonyl group, a cyclopentylsulfonyl group, a cyclohexylsulfonyl group, a cycloheptylsulfonyl group and a cyclooctylsulfonyl group.

Preferable examples of the "hydroxyl group having a protecting group" include a methoxymethyl ether group, a tetrahydropyranyl ether group, a tert-butyl ether group, an allyl ether group, a benzoate group, an acetate group, a formate group, a crotonate group, a p-phenylbenzoate group, a pivaloate group, a tert-butyldimethylsilyl group, a tert-butyldiphenylsilyl group, a trityl group and a benzyl group.

A preferable example of the C1-6 alkoxy group in the "C1-6 alkoxy group (wherein the C1-6 alkoxy group may be substituted with 1 to 5 halogen atoms or the adjacent C1-6 alkoxy groups, together with a carbon atom to which they are bonded, may form a cyclic group)" is 1 to 5 halogen atoms; alternatively, the adjacent C1-6 alkoxy groups, together with a carbon atom to which they are bonded, may form a cyclic group. The phrase "the adjacent C1-6 alkoxy groups, together with a carbon atom to which they are bonded, may form a cyclic group" refers to a methylenedioxy group or an ethylenedioxy group, for example. Such a group is specifically represented by the following formula, for example.

[Formula 5]

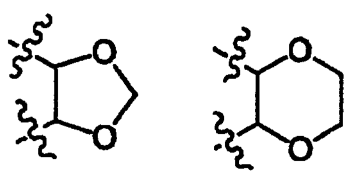

Next, the compound of the formula (I) of the present invention will be described.

In the compound of the formula (I) or pharmacologically acceptable salt thereof,

$Ar_1$ is preferably an imidazolyl group or a triazolyl group which may be substituted with 1 to 3 substituents selected from Substituent Group A1,

$Ar_1$ is more preferably an imidazolyl group or a triazolyl group which may be substituted with 1 or 2 substituents selected from the group consisting of a hydrogen atom and a C1-6 alkyl group (wherein the C1-6 alkyl group may be substituted with 1 to 3 halogen atoms),

$Ar_1$ is still more preferably an imidazolyl group which may be substituted with a C1-6 alkyl group, and
$Ar_1$ is most preferably an imidazolyl group which may be substituted with a methyl group.

In the compound of the formula (I) or pharmacologically acceptable salt thereof,
$Ar_2$ is preferably a pyrimidinyl group or a phenyl group which may be substituted with 1 to 3 substituents selected from Substituent Group A2 shown below,
$Ar_2$ is more preferably a phenyl group which may be substituted with 1 to 3 substituents selected from the group consisting of a hydrogen atom, a halogen atom, a cyano group, a C1-6 alkoxy group (wherein the C1-6 alkoxy group may be substituted with 1 to 3 substituents selected from the group consisting of a C2-6 alkenyl group, a C2-6 alkynyl group and a C3-8 cycloalkyl group), a C2-6 alkenyloxy group and a C2-6 alkynyloxy group, and
$Ar_2$ is still more preferably a phenyl group which may be substituted with 1 to 3 substituents selected from the group consisting of a hydrogen atom, a halogen atom, a cyano group and a C1-6 alkoxy group,
$Ar_2$ is yet more preferably a phenyl group which may be substituted with a C1-6 alkoxy group, and
$Ar_2$ is most preferably a phenyl group which may be substituted with a methoxy group.

In the compound of the formula (I) or pharmacologically acceptable salt thereof,
$X_1$ is preferably a C1-6 alkylene group, a C2-6 alkenylene group or a single bond, and
$X_1$ is more preferably a single bond.

In the compound of the formula (I) or pharmacologically acceptable salt thereof,
preferably, $R^1$ and $R^2$ are the same or different and each represent a group selected from Substituent Group A4,
more preferably, $R^1$ and $R^2$ are the same or different and each represent a group selected from Substituent Group A5, and
most preferably, $R^1$ and $R^2$ are the same or different and each represent a hydrogen atom or a C1-6 alkyl group (wherein the C1-6 alkyl group is a hydrogen atom, a C3-8 cycloalkyl group, a C3-8 cycloalkoxy group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7 and -O-$A^4$ (wherein $A^4$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)).

In the compound of the formula (I) or pharmacologically acceptable salt thereof,
preferably, $R^1$ and $R^2$, together with a nitrogen atom to which they are bonded, form a 5- to 11-membered heterocyclic group represented by the formula (II), and
more preferably, the 5- to 11-membered heterocyclic group is a piperidinyl group, a pyrrolidinyl group, an azepinyl group, an azocanyl group, a piperazinyl group, a 1,4-diazepanyl group, a morpholinyl group or a thiomorpholinyl group.

In the compound of the formula (I),
the substituent for the 5- to 11-membered heterocyclic group represented by the formula (II) which is formed by $R^1$ and $R^2$ together with a nitrogen atom to which they are bonded is preferably 1 to 4 substituents selected from Substituent Group A4,
the substituent for the 5- to 11-membered heterocyclic group represented by the formula (II) which is formed by $R^1$ and $R^2$ together with a nitrogen atom to which they are bonded is more preferably 1 to 4 substituents selected from the group consisting of a hydrogen atom, a halogen atom, a hydroxyl group, a formyl group, a hydroxyimino group, a C1-6 alkoxyimino group, a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A6 and a 5- to 14-membered aromatic heterocyclic group which may be substituted with 1 to 3 substituents selected from Substituent Group A6), a 6- to 14-membered aromatic hydrocarbon ring group which may be substituted with 1 to 3 substituents selected from Substituent Group A6, a 5-to 14-membered aromatic heterocyclic group which may be substituted with 1 to 3 substituents selected from Substituent Group A6, -O-$A^2$ (wherein $A^2$ represents a 6-to 14-membered aromatic hydrocarbon ring group which may be substituted with 1 to 3 substituents selected from Substituent Group A6 or a 5- to 14-membered aromatic heterocyclic group which may be substituted with 1 to 3 substituents selected from Substituent Group A6), -CO-$A^2$ (wherein $A^2$ is as defined above) and =CH-$A^2$ (wherein $A^2$ is as defined above), and
the substituent for the 5- to 11-membered heterocyclic group represented by the formula (II) which is formed by $R^1$ and $R^2$ together with a nitrogen atom to which they are bonded is most preferably 1 to 4 substituents selected from the group consisting of a hydrogen atom, a halogen atom, a hydroxyl group, a C1-6 alkyl group (wherein the C1-6 alkyl group may be substituted with 1 to 3 hydroxyl groups or 1 to 3 substituents selected from a 6- to 14-membered aromatic hydrocarbon ring group which may be substituted with 1 to 3 substituents selected from Substituent Group A8), a 6- to 14-membered aromatic hydrocarbon ring group which may be substituted with 1 to 3 substituents selected from Substituent Group A8, a 5- to 14-membered aromatic heterocyclic group which may be substituted with 1 to 3 substituents selected from

Substituent Group A8, -O-$A^6$ (wherein $A^6$ represents a 6- to 14-membered aromatic hydrocarbon ring group which may be substituted with 1 to 3 substituents selected from Substituent Group A8) and =CH-$A^6$ (wherein $A^6$ is as defined above).

In the compound of formula (I) or pharmacologically acceptable salt thereof, $R^1$ is preferably $-X_{21}-X_{22}-Ar_3$ (wherein $X_{21}$ represents 1) a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group) and a 5- to 14-membered non-aromatic heterocyclic group which may be substituted with 1 to 3 substituents selected from Substituent Group A6) or 2) a single bond; $X_{22}$ represents a single bond, an imino group which may be substituted with a substituent selected from Substituent Group A6, -O- or -S-; and $Ar_3$ represents a 6- to 14-membered aromatic hydrocarbon which may be substituted with 1 to 3 substituents selected from Substituent Group A6 or a 5- to 14-membered aromatic heterocyclic group which may be substituted with 1 to 3 substituents selected from Substituent Group A6).

In the compound of the formula (I) or pharmacologically acceptable salt thereof, $R^1$ is more preferably $-X_{21a}-X_{22a}-Ar_{3a}$ (wherein $X_{21a}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms) and a 5- to 14-membered non-aromatic heterocyclic group which may be substituted with 1 to 3 substituents selected from Substituent Group A7); $X_{22a}$ represents a single bond or an oxygen atom; and $Ar_{3a}$ represents a 6-to 14-membered aromatic hydrocarbon ring group which may be substituted with 1 to 3 substituents selected from Substituent Group A7 or a 5-to 14-membered aromatic heterocyclic group which may be substituted with 1 to 3 substituents selected from Substituent Group A7).

In the compound of the formula (I) or pharmacologically acceptable salt thereof,
preferably, when $R^1$ represents $-X_{21}-X_{22}-Ar_3$,
$Ar_3$ is a 6- to 14-membered aromatic hydrocarbon group which may be substituted with 1 to 3 substituents selected from Substituent Group A6 or a 5-to 14-membered aromatic heterocyclic group which may be substituted with 1 to 3 substituents selected from Substituent Group A6,
more preferably, when $R^1$ represents $-X_{21a}-X_{22a}-Ar_{3a}$,
$Ar_{3a}$ is a 6- to 14-membered aromatic hydrocarbon group which may be substituted with 1 to 3 substituents selected from Substituent Group A7 or a 5-to 14-membered aromatic heterocyclic group which may be substituted with 1 to 3 substituents selected from Substituent Group A7, and
most preferably, when $R^1$ represents $-X_{21a}-X_{22a}-Ar_{3a}$,
$Ar_{3a}$ is a 6- to 14-membered aromatic hydrocarbon ring group selected from the group consisting of a phenyl group, a naphthyl group and a fluorenyl group or a 5- to 14-membered aromatic heterocyclic group selected from the group consisting of a thienyl group, a pyridinyl group, a quinolinyl group, an isoquinolinyl group, an indolyl group, a benzothiazolyl group, a benzoxazolyl group and a furyl group, which may be substituted with 1 to 3 substituents selected from Substituent Group A7.

In the compound of the formula (I) or pharmacologically acceptable salt thereof,
when $R^1$ represents a 6- to 14-membered non-aromatic hydrocarbon ring group or a 5- to 14-membered non-aromatic heterocyclic group represented by the formula (III),
$Ar_4$ is preferably a 6- to 14-membered aromatic hydrocarbon ring group which may be substituted with 1 to 3 substituents selected from Substituent Group A4 or a 5- to 14-membered aromatic heterocyclic group which may be substituted with 1 to 3 substituents selected from Substituent Group A4, and
$Ar_4$ is more preferably a 5- to 14-membered aromatic heterocyclic group selected from the group consisting of a phenyl group, a pyridyl group, a pyrimidinyl group, a pyrazinyl group, a thienyl group, an oxazolyl group, a pyrrolynyl group, a thiazolydinyl group and a furyl group, which may be substituted with 1 to 3 substituents selected from the group consisting of a halogen atom, a C1-6 alkyl group (wherein the C1-6 alkyl group may be substituted with 1 to 3 substituents selected from the group consisting of a halogen atom and a C1-6 alkyl group), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A6, a 5- to 14-membered aromatic heterocyclic group which may be substituted with 1 to 3 substituents selected from Substituent Group A6, a 5-to 14-membered non-aromatic heterocyclic group which may be substituted with 1 to 3 substituents selected from Substituent Group A6 and -CO-$A^2$ (wherein $A^2$ represents a 6- to 14-membered aromatic hydrocarbon ring group which may be substituted with 1 to 3 substituents selected from Substituent Group A6 shown below or a 5- to 14-membered aromatic heterocyclic group which may be substituted with 1 to 3 substituents selected from Substituent Group A6 shown below).

In the compound of the formula (I) or pharmacologically acceptable salt thereof, $R^1$ is preferably a 6- to 14-membered non-aromatic hydrocarbon ring group or a 5- to 14-membered non-aromatic heterocyclic group represented by the formula (III), and

$R^1$ is more preferably an indanyl group, an azaindanyl group, a tetrahydronaphthyl group, an azatetrahydronaphthyl group, a chromanyl group, an azachromanyl group, a tetrahydrobenzofuranyl group or a tetrahydrobenzothienyl group, which may be substituted with 1 to 3 substituents selected from the group consisting of a halogen atom, a hydroxyl group, a cyano group, a C3-8 cycloalkyl group, a C3-8 cycloalkoxy group, a C1-6 alkyl group (wherein the C1-6 alkyl group may be substituted with 1 to 3 halogen atoms or C1-6 alkyl groups), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms) and a 5- to 14-membered non-aromatic heterocyclic group.

**[0056]** - Methods for preparing the compound of the general formula (I) of the present invention will be described below. The compound represented by the general formula (I):

[Formula 6]

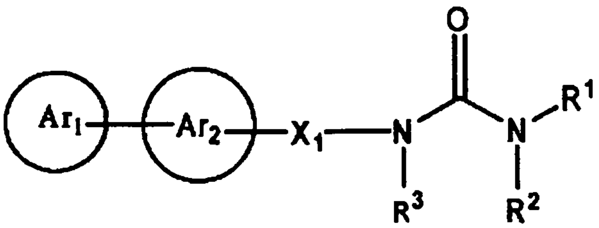

(I)

wherein $Ar_1$, $Ar_2$, $R^1$, $R^2$, $R^3$ and $X_1$ are as defined above, is synthesized according to a method such as the following General Preparation Method 1 to General Preparation Method 4, for example.

[General Preparation Method 1]

Typically used General Preparation Method 1 for the compound of the general formula (I) of the present invention will be described below.

[Formula 7]

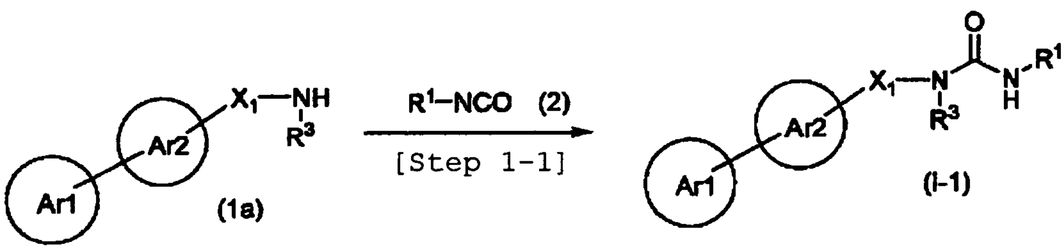


(1a) (I-1)

In the formula, $Ar_1$, $Ar_2$, $R^1$ and $X_1$ are as defined above; and $R^3$ represents a group selected from Substituent Group

A4 shown above.

The above General Preparation Method 1 is an example of a method for preparing the compound of the general formula (I-1) comprising reacting an amine compound (1a) with an isocyanate compound (2) by addition reaction in Step 1-1.

[Preparation of compound of general formula (I-1)]

The compound of the general formula (I-1) can be prepared by reacting an amine compound (1a) with an isocyanate compound (2) according to Step 1-1. Specifically, Step 1-1 varies according to the starting material and is not particularly limited insofar as the conditions are similar to those in this reaction. A known method described in many documents may be used for the reaction (see Shin Jikken Kagaku Koza (New Courses in Experimental Chemistry), vol. 14, Yuki Gosei (Organic Synthesis) [II], edited by The Chemical Society of Japan, Maruzen Co., Ltd., August 2005, p. 428-429, for example). Preferably, 1.0 to 10.0 equivalents of the isocyanate compound (2) with respect to the amine compound (1a) is stirred usually in an inert solvent in the presence or absence of 1.0 to 100.0 equivalents of a base with respect to the amine compound (1a), for example. The base used varies according to the starting material and is not particularly limited. Both an inorganic base and an organic base are effectively used. Preferable examples of the inorganic salt include hydroxides, hydrides, carbonates and bicarbonates of alkali metals and alkali earth metals. Potassium carbonate, sodium carbonate, sodium hydroxide, potassium hydroxide, sodium bicarbonate, potassium bicarbonate and the like are particularly preferable. The organic base is preferably a tertiary amine such as triethylamine. The solvent used is not particularly limited insofar as it does not inhibit the reaction and allows the starting material to be dissolved therein to a certain extent. Preferable examples of the solvent include ether solvents such as ethyl ether, diisopropyl ether, dimethoxyethane, tetrahydrofuran and dioxane; halogenated solvents such as dichloromethane, dichloroethane and chloroform; aromatic solvents such as toluene, chlorobenzene and xylene; acetonitrile, N,N-dimethylformamide, acetone, methyl ethyl ketone, dimethyl sulfoxide and water. These may be used single or in a mixture. Toluene, acetonitrile, dichloromethane, chloroform and the like are particularly preferable. The reaction temperature must be a temperature that can complete the reaction without promoting formation of an undesirable by-product, and is preferably -20°C to 200°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Preparation of amine compound (1a)]

[Formula 8]

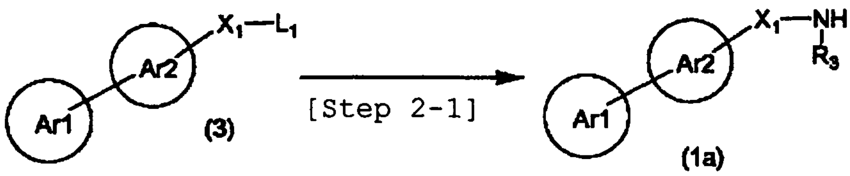


In the formula, $Ar_1$, $Ar_2$ and $X_1$ are as defined above; $L_1$ represents a hydroxyl group, a halogen atom, a nitro group, a nitrile group, an oxime group, an azide group, an amide group or a carbonyl group; and $R^3$ represents a group selected from Substituent Group A4 shown above.

The amine compound (1a) can be prepared from a compound (3) according to Step 2-1. Specifically, Step 2-1 varies according to the starting material and is not particularly limited insofar as the conditions are similar to those in this reaction. A method known to a person skilled in the art may be used for the reaction. Examples of the method include i) a method of converting the compound (3), wherein $L_1$ represents a hydroxyl group or a halogen atom, into the amine compound by a known technique, ii) a method of converting the compound (3), wherein $L_1$ represents a nitro group, a nitrile group, an oxime group, an azide group or an amide group, by a known reduction reaction and iii) a method of converting the compound (3), wherein $L_1$ represents a carbonyl group, by a known reductive amination reaction.

In the method i), the conversion can be performed by a method described in many known documents. For example, the amine compound (1a) is preferably obtained from the corresponding alcohol compound (3) by the Mitsunobu

method (see O. Mitsunobu, "Synthesis", 1981, p.1, for example) or from the alkyl halide compound (3) by the Gabriel method (see M.M.S. Gibson et al., "Angew. Chem.", 1968, vol.80, p.986, for example). In the Mitsunobu method, the desired amine compound (1a) can be efficiently obtained by two-stage reaction in which the compound (3) is condensed with preferably 1.0 to 3.0 equivalents of an imide compound such as phthalimide with respect to the alcohol compound (3) in the presence of preferably 1.0 to 3.0 equivalents of triphenylphosphine and 1.0 to 3.0 equivalents of dialkyl azodicarboxylate with respect to the alcohol compound (3), for example, and is then treated with 1.0 to 3.0 equivalents of hydrazine, for example. The reaction temperature must be a temperature that can complete the reaction without promoting formation of an undesirable by-product. Preferably, the temperature is, for example, ice-cold temperature to 100°C for the first-stage condensation with an imide compound and is, for example, 50°C to 100°C for the second-stage hydrazine treatment. The solvent used in this reaction varies according to the starting material and the condensing agent used, and is not particularly limited insofar as it does not inhibit the reaction and allows the starting material to be dissolved therein to a certain extent. Diethyl ether or tetrahydrofuran is preferable for the first-stage reaction, for example, and methanol or ethanol is preferable for the second-stage reaction, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization. In the Gabriel method, preferably, the desired amine compound (1a) can be efficiently obtained by two-stage reaction in which the corresponding alkyl halide compound (3) is condensed with an imide compound by a technique known to a person skilled in the art and is then treated with, preferably, for example, 1.0 to 3.0 equivalents of hydrazine. The reaction temperature must be a temperature that can complete the reaction without promoting formation of an undesirable by-product. Preferably, the temperature is, for example, ice-cold temperature to 100°C for the first-stage condensation with an imide compound and is, preferably, for example, 50 to 100°C for the second-stage hydrazine treatment. The solvent used in this reaction varies according to the starting material and the condensing agent used, and is not particularly limited insofar as it does not inhibit the reaction and allows the starting material to be dissolved therein to a certain extent. Diethyl ether, tetrahydrofuran or N, N-dimethylformamide is preferable for the first-stage reaction, for example, and methanol or ethanol is preferable for the second-stage reaction, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

In the method ii), a reduction reaction described in many known documents may be used (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., February, 1978, p. 1333-1341, for example). Preferably, the desired amine compound can be obtained by a catalytic reduction method using a metal catalyst or a reduction method using a metal hydride, for example.

The catalytic reduction method is preferably, for example, performed in a hydrogen atmosphere at normal pressure to 100 atm. Preferable examples of the metal catalyst used in this reaction include platinum, platinum oxide, platinum black, Raney nickel and palladium-carbon. The weight ratio of the metal catalyst to the compound (3) is 1 to 100%, for example, and preferably 1 to 50%, for example. The amount of the metal catalyst used may be appropriately increased and reduced. The solvent used in this reaction varies according to the starting material, and is not particularly limited insofar as it does not inhibit the reaction and allows the starting material to be dissolved therein to a certain extent. Preferable examples of the solvent include methanol, ethanol, diethyl ether, tetrahydrofuran, methylene chloride, chloroform and ethyl acetate. An acidic substance such as acetic acid or hydrochloric acid may be appropriately added in order to make the reaction efficiently proceed. The reaction temperature must be a temperature that can complete the reaction without promoting formation of an undesirable by-product. The temperature is preferably room temperature to 100°C, for example, and more preferably room temperature to 50°C, for example. Under preferable reaction conditions, the reduction reaction is completed in 1 to 24 hours.

In the reduction method using a metal hydride, preferably, the desired amine compound (1a) can be efficiently obtained using lithium aluminum hydride or diborane. The amount of the metal hydride used is preferably 1.0 to 100.0 equivalents, for example, and more preferably 1.0 to 10.0 equivalents, for example, with respect to the compound (3) and may be appropriately increased and reduced.

The solvent used in this reaction varies according to the starting material, and is not particularly limited insofar as it does not inhibit the reaction and allows the starting material to be dissolved therein to a certain extent. The solvent is preferably diethyl ether or tetrahydrofuran, for example. The reduction reaction temperature in the reduction method using a metal hydride must be a temperature that can complete the reaction without promoting formation of an undesirable by-product, and is preferably ice-cold temperature to 100°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

In the method iii), a reductive amination reaction known to a person skilled in the art may be used (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., February 1978, p.1380-1384, for example). The desired amine compound (1a) is preferably obtained by a method of reacting the corresponding carbonyl compound (3) with an amine compound using dehydration reaction by an acid catalyst (such as preferably an inorganic acid such as hydrochloric acid or sulfuric acid; an organic acid such as acetic acid, methanesulfonic acid, p-toluenesulfonic acid or camphorsulfonic acid; an organic acid salt such as pyridinium p-toluenesulfonate; or a Lewis acid such as titanium (IV) isopropoxide); and reducing the resulting imine compound by a metal hydride or the like such as lithium aluminum hydride, sodium borohydride, sodium triacetoxyborohydride or sodium cyanoborohydride, or hydrogenating the imine compound in the presence of a metal catalyst such as a palladium catalyst, for example, Pd-C, or platinum oxide.
Preferably 0.5 to 50.0 equivalents, for example, and more preferably 1.0 to 10.0 equivalents, for example, of the amine compound may be used with respect to the carbonyl compound (3). Preferably 0.01 to 10.0 equivalents, for example, and more preferably 0.1 to 5.0 equivalents of the acid catalyst may be used with respect to the carbonyl compound (3). The imine compound as an intermediate can be obtained by reacting the amine compound with the carbonyl compound (3) in a solvent such as preferably an aromatic hydrocarbon such as toluene, xylene or benzene; an ether such as diethyl ether; or a halogenated hydrocarbon such as chloroform or dichloromethane, and more preferably benzene or toluene preferably at -78°C to 150°C, for example, and more preferably at room temperature to 100°C, for example, preferably for 1 to 24 hours, for example, and more preferably for 1 to 10 hours, for example.
The resulting imine compound may be reduced directly without purification or after once evaporating the solvent and replacing it with a solvent suitable for a reducing agent.
When the metal hydride is used as a reducing agent, preferably 1.0 to 10.0 equivalents of the metal hydride may be used with respect to the carbonyl compound (3), for example. When the metal catalyst is used, the catalyst may be used in a weight ratio to the carbonyl compound (3) of preferably 1 to 100%, for example, and more preferably 1 to 10%, for example.
The solvent used in this reaction varies according to the starting material, and is not particularly limited insofar as it does not inhibit the reaction and allows the starting material to be dissolved therein to a certain extent. When the metal hydride is used as a reducing agent, it is possible to use a solvent such as preferably diethyl ether, tetrahydrofuran, methanol or ethanol, and more preferably an ether solvent such as tetrahydrofuran. When hydrogenation is performed using the metal catalyst, it is possible to use a solvent such as preferably diethyl ether, tetrahydrofuran, methanol or ethanol, and more preferably an alcohol solvent such as methanol or ethanol.
When the metal hydride is used as a reducing agent in the reduction reaction, the amine compound (1a) can be obtained by reacting preferably at -20 to 100°C, for example, and more preferably at 0 to 60°C, for example, preferably for 1 to 24 hours, for example, and more preferably for 1 to 10 hours, for example.
When hydrogenation is performed using the metal catalyst in the reduction reaction, the amine compound (1a) can be obtained by reacting preferably at room temperature to 100°C, for example, and more preferably at room temperature to 50°C, for example, preferably for 1 to 24 hours, for example, and more preferably for 1 to 10 hours, for example.
The progress of the reductive amination reaction in the method iii) can be monitored by a known chromatography technique. An undesirable by-product can be removed by a technique known to a person skilled in the art such as a conventional chromatography technique or/and crystallization.

[Preparation of compound (3)]

[Formula 9]

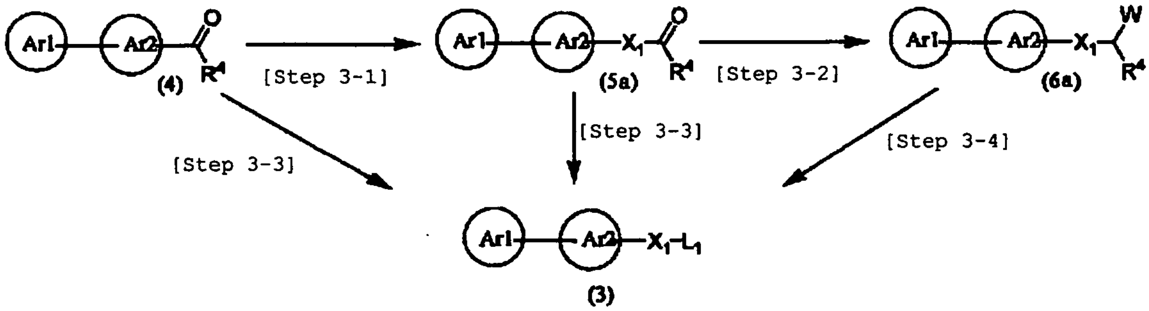


above; $L_1$ represents a hydroxyl group, a halogen atom, a nitro group, a nitrile group, an oxime group, an azide group, an amide group or a carbonyl group; $R^4$ represents a group selected from Substituent Group A3 shown above; and W represents a hydroxyl group, a sulfonate group such as a methanesulfonate group or a p-toluenesulfonate group, or a halogen atom such as a chlorine atom, a bromine atom or an iodine atom.

The compound (3) can be prepared as shown above, for example; however, the method for preparing the compound (3) is not limited thereto and varies according to the definition of $X_1$ or $L_1$. For example, the compound (3), wherein $X_1$ represents a C1 alkylene group which may be substituted with 1 to 2 substituents from Substituent Group A3 shown above; and $L_1$ represents a hydroxyl group or an oxime group, i) can be prepared from a carbonyl compound (4) according to Step 3-3. Alternatively, the compound (3), wherein $L_1$ represents a hydroxyl group or an oxime group, can be prepared from a compound (5a) according to the above Step 3-3. Alternatively, the compound (3), wherein $L_1$ represents a halogen atom, an azido group or a nitrile group, ii) can be prepared from a compound (6a) according to the above Step 3-4.

In the method i), Step 3-3 represents reduction reaction or oximation reaction. The reduction reaction in Step 3-3 varies according to the starting material and is not particularly limited insofar as the conditions are similar to those in this reaction. A known method described in many documents may be used for the reaction (see Shin Jikken Kagaku Koza (New Courses in Experimental Chemistry), vol.14, Yuki Gosei (Organic Synthesis) [II], edited by The Chemical Society of Japan, Maruzen Co., Ltd., August 2005, p.1-49, for example).
The oximation reaction in Step 3-3 varies according to the starting material and is not particularly limited insofar as the conditions are similar to those in this reaction. A known method described in many documents may be used for the reaction (see Shin Jikken Kagaku Koza (New Courses in Experimental Chemistry), vol.14, Yuki Gosei (Organic Synthesis) [II], edited by The Chemical Society of Japan, Maruzen Co., Ltd., August 2005, p.417-419, for example). Preferably 1.0 to 100 equivalents of hydroxylamine (or its monohydrochloride) with respect to the carbonyl compound (4) or (5a), for example, is stirred in a solvent in the presence of preferably 1.0 to 100 equivalents of a base or an acid with respect to the compound, for example. The base used varies according to the starting material and is not particularly limited. Preferable examples of the base include inorganic bases such as sodium acetate, sodium hydroxide, potassium hydroxide, potassium carbonate, sodium carbonate and sodium bicarbonate; and organic bases such as triethylamine, pyridine and aqueous ammonia. The acid used varies according to the starting material and is not particularly limited. The acid is preferably acetic acid, for example. The solvent used varies according to the starting material, and is not particularly limited insofar as it does not inhibit the reaction and allows the starting material to be dissolved therein to a certain extent. Preferable examples of the solvent include alcohol solvents such as methanol, ethanol and ethylene glycol; halogenated hydrocarbons such as dichloromethane and chloroform; ketones such as acetone and methyl ethyl ketone; water and mixed solvents thereof. The reaction temperature must be a temperature that can complete the reaction without promoting formation of an undesirable by-product, and is preferably -20°C to 100°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

In the method ii), Step 3-4 represents halogenation reaction or azidation reaction. The halogenation reaction in Step 3-4 varies according to the starting material and is not particularly limited insofar as the conditions are similar to those in this reaction. A known method described in many documents may be used for the reaction (see Shin Jikken Kagaku Koza (New Courses in Experimental Chemistry), vol.19, Yuki Gosei (Organic Synthesis) [I], edited by The Chemical Society of Japan, Maruzen Co., Ltd., June 1992, p.438-446, for example). The azidation reaction in Step 3-4

varies according to the starting material and is not particularly limited insofar as the conditions are similar to those in this reaction. A known method described in many documents may be used for the reaction (see Shin Jikken Kagaku Koza (New Courses in Experimental Chemistry), vol.14, Yuki Gosei (Organic Synthesis) [II], edited by The Chemical Society of Japan, Maruzen Co., Ltd., August 2005, p.480-487, for example).

[Synthesis of compound (6a)]

The compound (6a) can be prepared by reduction reaction of a compound (5a) or subsequent conversion into a leaving group according to Step 3-2, for example. Specifically, Step 3-2 varies according to the starting material and is not particularly limited insofar as the conditions are similar to those in this reaction. A known method described in many documents may be used for the reaction (see Shin Jikken Kagaku Koza (New Courses in Experimental Chemistry), vol.14, Yuki Gosei (Organic Synthesis) [II], edited by The Chemical Society of Japan, Maruzen Co., Ltd., August 2005, p.1-49; and Bailey, W., Org. Synth., vol.81, 204, p.121, for example).

[Preparation of carbonyl compound (5a)]

The carbonyl compound (5a) can be prepared from a carbonyl compound (4) according to Step 3-1. The preparation method in Step 3-1 varies according to the definition of $X_1$, and the carbonyl compound (5a) can be prepared from the carbonyl compound (4) by a method known to a person skilled in the art.

[Preparation of carbonyl compound (4) ($Ar_1$ = imidazolyl group)]

[Formula 10]

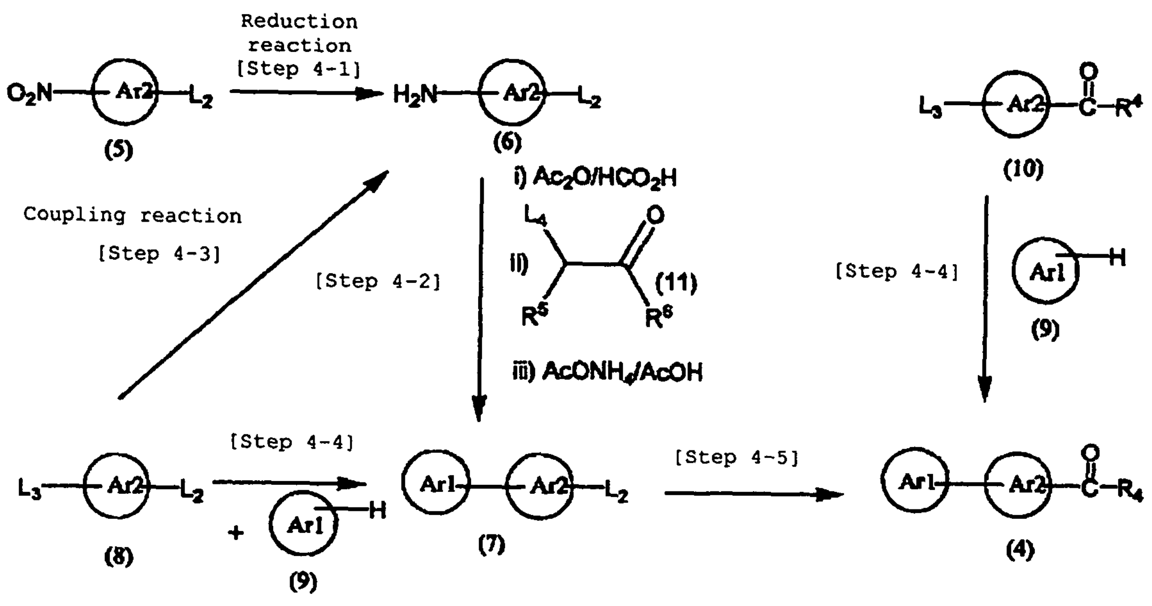


In the formula, $Ar_2$ is as defined above; $Ar_1$ represents an imidazolyl group; $L_2$ represents a carbonyl group such as an aldehyde group or an acetyl group, an alkoxycarbonyl group such as a methyl ester group, a halogen atom such as a fluorine atom, a chlorine atom, a bromine atom or an iodine atom, a sulfonate group such as a trifluoromethanesulfonate group, a trialkyltin group, a boronic acid or boronate group or a nitro group; $L_3$ represents a hydrogen atom or a leaving group such as a halogen atom such as a fluorine atom, a chlorine atom, a bromine atom or an iodine atom, a sulfonate group such as a trifluoromethanesulfonate group, a trialkyltin group, or a boronic acid or boronate group; $L_4$ represents a halogen atom such as a fluorine atom, a chlorine atom, a bromine atom or an iodine atom, or a sulfonate group such

as a trifluoromethanesulfonate group; $R^4$ represents a group selected from Substituent Group A3 shown above; and $R^5$ and $R^6$ each represent a group selected from Substituent Group A1 shown above.

The carbonyl compound (4) can be prepared from a compound (10) as a starting material according to Step 4-4, for example. Specifically, Step 4-4 varies according to the starting material and is not particularly limited insofar as the conditions are similar to those in this reaction. A method known to a person skilled in the art may be used for the reaction. For example, the compound (10) and 1.0 to 5.0 equivalents of a compound (9) with respect to the compound (10) are stirred in a solvent in the presence or absence of 1.0 to 5.0 equivalents of a base with respect to the compound (10). (see D.D. Davey et al., "J. Med. Chem.", 1991, vol.39, p.2671-2677, for example). Examples of the base used include, for example, sodium hydride, sodium hydroxide, potassium hydroxide, potassium carbonate, sodium carbonate, cesium carbonate and barium carbonate. The solvent used in this reaction varies according to the starting material, and is not particularly limited insofar as it does not inhibit the reaction and allows the starting material to be dissolved therein to a certain extent. Preferable examples of the solvent include acetonitrile, tetrahydrofuran, dimethyl sulfoxide, N,N-dimethylformamide and N-methylpyrrolidine. The reaction temperature must be a temperature that can complete the reaction without promoting formation of an undesirable by-product, and is preferably, for example, room temperature to 150°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

The carbonyl compound (4) can also be prepared from a compound (7) as a starting material according to Step 4-5, for example. Specifically, Step 4-5 varies according to the starting material and is not particularly limited insofar as the conditions are similar to those in this reaction. A method known to a person skilled in the art may be used for the reaction. For example, it is possible to use a two-stage method of converting the compound (7), wherein $L_2$ represents a chlorine atom, a bromine atom, an iodine atom or a sulfonate group such as a trifluoromethanesulfonate group, into a vinyl compound by Stille coupling reaction with a vinyltin compound; and then oxidizing the resulting compound by ozone oxidation reaction (see S.S. Chandran et al., "Bioorg. Med. Chem. Lett.", 2001, vol.11, p.1493-1496, for example). It is also possible to employ carbon monoxide insertion reaction using a transition metal catalyst (see T. Okano et al., "Bull. Chem. Soc. Jpn.", 1994, vol.67, p.2329-2332, for example).

[Preparation of compound (10)]

The compound (10) used in this step is commercially available or can be obtained by a technique known to a person skilled in the art. If not commercially available, the preferable compound (10), wherein $L_3$ represents a fluorine atom, a chlorine atom or a bromine atom, can be obtained by oxidizing a corresponding alcohol compound by an oxidation reaction known to a person skilled in the art (see Shin Jikken Kagaku Koza (New Courses in Experimental Chemistry), vol.21, Yuki Gosei (Organic Synthesis) [III], edited by The Chemical Society of Japan, Maruzen Co., Ltd., February 1991, p.2-23 and 196-240, for example); or the carbonyl compound can be obtained by reducing an ester compound by a known reduction reaction (see Shin Jikken Kagaku Koza (New Courses in Experimental Chemistry), vol.21, Yuki Gosei (Organic Synthesis) [III], edited by The Chemical Society of Japan, Maruzen Co., Ltd., February 1991, p.83-85 and 289-298, for example).

[Preparation of compound (7) ($Ar_1$ = imidazolyl group)]

The compound (7) can be obtained by a technique known to a person skilled in the art. Preferably, the compound (7) can be prepared i) from a compound (8) as a starting material according to Step 4-4 or ii) from an amine compound (6) as a starting material according to Step 4-2, for example.

In the method i), the compound (7) can be obtained according to the above Step 4-4.
In the method ii), the amine compound (6) can be efficiently converted into the compound (7) in Step 4-2 by treating the amine compound (6) with a mixed solvent of acetic anhydride and formic acid in the first stage; condensing the compound with a compound (11) under basic conditions in the second stage; and heating the condensate with ammonium acetate and acetic acid in the third stage, for example.
In the first stage, the compound (6) is preferably treated in a mixed solvent of preferably 2.0 to 10.0 equivalents of acetic anhydride and 10.0 to 20.0 equivalents of formic acid with respect to the compound (6), for example, preferably at ice-cold temperature to 50°C, for example. In the second stage, 1.0 to 5.0 equivalents of a base is preferably used with respect to the compound (6), for example. Preferable examples of the base include sodium hydride, sodium hydroxide, potassium hydroxide, lithium hydroxide, n-butyl lithium, lithium diisopropylamide, lithium bis(trimethylsilyl)amide and sodium bis(trimethylsilyl)amide. The solvent used in the present reaction varies according to the starting material, and is not particularly limited insofar as it does not inhibit the reaction and allows the starting material to be dissolved therein to a certain extent. Preferable examples of the solvent include diethyl ether, tetrahydrofuran, dimethyl sulfoxide and N, N-dimethylformamide. Preferably, potassium iodide or sodium iodide may be added, for example, in order to make the

reaction efficiently proceed. Preferably, 0.1 to 10.0 equivalents of such an additive is used with respect to the compound (6), for example. The reaction temperature must be a temperature that can complete the reaction without promoting formation of an undesirable by-product, and is preferably room temperature to 100°C, for example.
In the third stage, the condensate is preferably treated in a mixture of preferably 5.0 to 10.0 equivalents of ammonium acetate and 10.0 to 20.0 equivalents of acetic acid with respect to the compound (6), for example, preferably at 50 to 100°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique or/and crystallization.

The compound (11) used in the second stage of this step is commercially available or can be obtained by a technique known to a person skilled in the art. If not commercially available, the preferable compound (11) can be prepared from a corresponding carbonyl compound by a halogenation reaction known to a person skilled in the art (see Shin Jikken Kagaku Koza (New Courses in Experimental Chemistry), vol.19, Yuki Gosei (Organic Synthesis) [I], edited by The Chemical Society of Japan, Maruzen Co., Ltd., June 1992, p.363-482, for example).

[Preparation of amine compound (6)]

The amine compound (6) is commercially available or can be obtained by a technique known to a person skilled in the art. Preferably, the compound can be prepared from a nitro compound (5), wherein $L_2$ represents an alkoxycarbonyl group, as a starting material according to Step 4-1, for example. Specifically, the reduction reaction in Step 4-1 is performed by the same method as the method ii) in the above Step 2-1.

The preferable amine compound (6) can also be prepared from a compound (8) as a starting material which is commercially available or can be obtained by a technique known to a person skilled in the art, according to coupling reaction in Step 4-3. Specifically, the coupling reaction in Step 4-3 varies according to the starting material and is not particularly limited insofar as the conditions are similar to those in this reaction. A method known to a person skilled in the art may be used for the reaction. Preferably, for example, it is possible to use a two-stage method of performing coupling reaction of benzophenone imine using a transition metal catalyst and then performing a known benzophenone removal reaction treatment (see S.L. Buchwald et al., "Tetrahedron Lett.", 1997, vol.38, p.6367-6370; or J.F. Hartwig et al., "J. Am. Chem. Soc.", 1998, vol.120, p.827-828, for example).
In the coupling reaction of benzophenone imine, the compound (8) and 1.0 to 10.0 equivalents of benzophenone imine with respect to the compound (8) are stirred in a solvent in the presence of preferably 0.01 to 0.2 equivalent of a catalyst, for example. Preferable examples of the catalyst that can be used include known palladium complexes such as palladium (II) acetate, dichlorobis(triphenylphosphine)palladium (II), tetrakis(triphenylphosphine)palladium (0) and tris(dibenzylideneacetone)dipalladium (0); and known nickel catalysts such as (1,5-cyclooctadiene)nickel (0). Preferably, a phosphorus ligand such as triphenylphosphine, tri-o-tolylphosphine, tri-tert-butylphosphine, 2-(di-tert-butylphosphino)biphenyl, 2,2'-bis(diphenylphosphino)-1,1'-binaphthyl, 1,2-bis(diphenylphosphino)ethane or 1,1'-bis(diphenylphosphino)ferrocene may be appropriately added in order to make the reaction efficiently proceed, for example. A preferable result may be achieved in the presence of a base. The base used is not particularly limited insofar as it is used in a coupling reaction similar to this reaction. Preferable examples of the base include sodium hydroxide, barium hydroxide, potassium fluoride, cesium fluoride, sodium carbonate, potassium carbonate, cesium carbonate, potassium phosphate and sodium tert-butoxide. The solvent used varies according to the starting material and the transition metal catalyst used, and is not particularly limited insofar as it does not inhibit the reaction and allows the starting material to be dissolved therein to a certain extent. Preferable examples of the solvent include acetonitrile, tetrahydrofuran, 1,4-dioxane, 1,2-dimethoxyethane, benzene, toluene, xylene, 1-methyl-2-pyrrolidone and N,N-dimethylformamide. The reaction temperature must be a temperature that can complete the coupling reaction, and is preferably room temperature to 100°C, for example. This reaction is performed preferably in an inert gas atmosphere, and more preferably in a nitrogen or argon atmosphere, for example. A method known to a person skilled in the art may be used for the treatment after the second stage (see T.W. Green, "Protective Groups in Organic Synthesis", John Wiley & Sons, Inc., 1981). An undesirable by-product can be removed by a technique known to a person skilled in the art such as a conventional chromatography technique or/and crystallization.

In the preferable amine compound (6), $L_2$ can be modified by a method known to a person skilled in the art, and a hydrogen atom in $L_2$ can be preferably converted into a halogen substituent, for example (see Shin Jikken Kagaku Koza (New Courses in Experimental Chemistry), vol.14, Yuki Kagobutsu No Gosei To Hannou (Synthesis and Reaction of Organic Compounds) [I], edited by The Chemical Society of Japan, Maruzen Co., Ltd., November 1977, p.354-360, for example).

[Preparation of nitro compound (5)]

The nitro compound (5) is commercially available or can be obtained by a technique known to a person skilled in the art. If not commercially available, the preferable compound (5), wherein $L_2$ represents a fluorine atom, a chlorine atom, a bromine atom or an iodine atom, can be efficiently obtained from a corresponding precursor by a nitration reaction known to a person skilled in the art (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., February 1978, p.1261-1300, for example).

[Preparation of carbonyl compound (4) ($Ar_1$ = triazolyl group)]

[Formula 11]

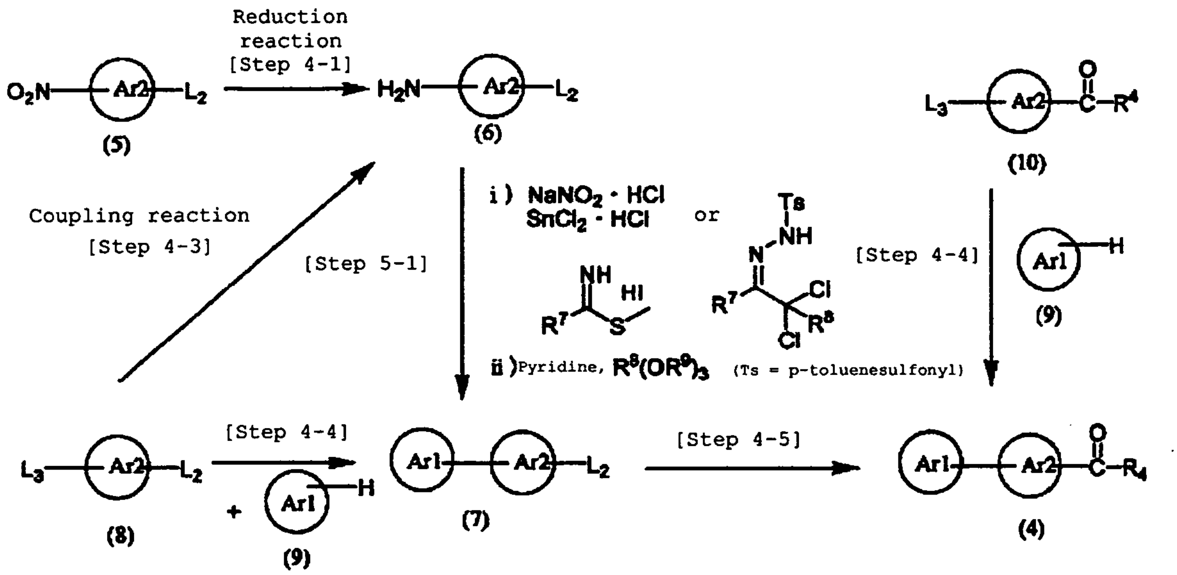


In the formula, $Ar_2$ is as defined above; $Ar_1$ represents a triazolyl group; $L_2$ represents a carbonyl group such as an aldehyde group or an acetyl group, an alkoxycarbonyl group such as a methyl ester group, a halogen atom such as a fluorine atom, a chlorine atom, a bromine atom or an iodine atom, a sulfonate group such as a trifluoromethanesulfonate group, a trialkyltin group, a boronic acid or boronate group or a nitro group; $L_3$ represents a hydrogen atom or a leaving group such as a halogen atom such as a fluorine atom, a chlorine atom, a bromine atom or an iodine atom, a sulfonate group such as a trifluoromethanesulfonate group, a trialkyltin group, or a boronic acid or boronate group; $R^4$ represents a group selected from Substituent Group A3 shown above; $R^7$ and $R^8$ each represent a group selected from Substituent Group A1 shown above; and $R^9$ represents a C1-6 alkyl group.

The carbonyl compound (4) can be prepared according to the above Step 4-4 and Step 4-5.

[Preparation of compound (7) ($Ar_1$ = triazolyl group)]

The compound (7) can be obtained by a technique known to a person skilled in the art. Preferably, the compound (7) can be prepared i) from a compound (8) as a starting material according to Step 4-4 or ii) from an amine compound (6) as a starting material according to Step 5-1, for example.

In the method i), the compound (7) can be obtained according to the above Step 4-4.

In the method ii), the compound (7) can be obtained in Step 5-1 by a technique known to a person skilled in the art, for example. Preferably, the compound can be prepared from the amine compound (6) as a starting material according to Step 5-1, for example. Specifically, in Step 5-1, i) when $Ar_1$ is [1,2,4]triazole, the amine compound (6) can be efficiently converted into the compound (7) by generating a diazonium salt using sodium nitrite and treating the diazonium salt

with stannic chloride to prepare hydrazine in the first stage; condensing the hydrazine with a thioimidate in the second stage; and cyclizing the condensate with an ortho ester in the presence of a base in the third stage. Preferably, in the first stage, the compound (6) is reacted with preferably 1.0 to 1.1 equivalents of sodium nitrite with respect to the compound (6), for example, preferably in a hydrochloric acid solvent, for example, preferably at -20°C to 0°C, for example, to prepare a diazonium salt, and then the diazonium salt is treated with preferably 3.5 to 4.0 equivalents of tin chloride with respect to the compound (6), for example, at the same temperature. The thioimidate used in the second stage can be easily obtained by reacting a corresponding thioamide compound with preferably 1.0 to 10.0 equivalents of methyl iodide with respect to the compound (6), for example, in an ether solvent at room temperature. 1.0 to 1.1 equivalents of the thioimidate is preferably used with respect to the compound (6), for example. The reaction solvent is preferably an alcohol solvent such as methanol or ethanol. The reaction temperature is preferably ice-cold temperature to room temperature. In the third stage, preferably 5 to 15 equivalents of the ortho ester with respect to the compound (6), for example, is preferably reacted in the presence of preferably 1.0 to 3.0 equivalents of a base with respect to the compound (6), for example.
The base used is preferably potassium carbonate, triethylamine or pyridine, for example, and more preferably pyridine. The solvent used in the present reaction varies according to the starting material, and is not particularly limited insofar as it does not inhibit the reaction and allows the starting material to be dissolved therein to a certain extent. The solvent is preferably toluene, tetrahydrofuran or dioxane, for example. The reaction temperature must be a temperature that can complete the reaction without promoting formation of an undesirable by-product, and is preferably room temperature to solvent reflux temperature, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique or/and crystallization. ii) When $Ar_1$ is [1,2,3]triazole, the compound (7) can be obtained by treating tosylhydrazone obtained from p-toluenesulfonylhydrazine and $\alpha,\alpha$-dichloroketone with the compound (6) in an alcohol solvent by a known method (see K. Sakai et al., "Bull. Chem. Soc. Jpn.", 1986, vol.59, p.179-183, for example).

[Preparation of isocyanate (2)]

The isocyanate (2) is commercially available or can be prepared by a method known to a person skilled in the art (see Shin Jikken Kagaku Koza (New Courses in Experimental Chemistry), vol.14, Yuki Gosei (Organic Synthesis) [II], edited by The Chemical Society of Japan, Maruzen Co., Ltd., August 2005, p.537-542, for example). Examples of the method include i) a method of converting a corresponding carboxylic acid compound or acid chloride by a known method and ii) a method of converting a corresponding amine compound by a known method.

In the method i), the conversion can be performed by a method described in many known documents. For example, the method is preferably Curtius rearrangement in which the isocyanate compound is obtained by reacting the corresponding carboxylic acid compound or acid chloride or a mixed acid anhydride obtained from the acid and a C1-6 alkyl halogenoformate with an azidating agent in the presence or absence of a base, and then heating the resulting acid azide compound in a solvent (see K. Ninomiya et al., Tetrahedron, vol.30, 1974, p.2151, for example). The azidating agent used varies according to the starting material and is not particularly limited. Preferable examples of the azidating agent include di-$C_1$-$C_4$ alkylphosphoryl azides such as dimethylphosphoryl azide, diethylphosphoryl azide and dibutylphosphoryl azide; di-$C_6$-$C_{10}$ arylphosphoryl azides such as diphenylphosphoryl azide and ditolylphosphoryl azide; hydrogen azide; alkali metal azides such as sodium azide and potassium azide; and tri-$C_1$-$C_4$ alkylsilyl azides such as trimethylsilyl azide, triethylsilyl azide and tri-tert-butylsilyl azide. Di-$C_6$-$C_{10}$ arylphosphoryl azides or alkali metal azides are more preferable, and diphenylphosphoryl azide or sodium azide is particularly preferable.

Di-$C_1$-$C_4$ alkylphosphoryl azides or di-$C_6$-$C_{10}$ arylphosphoryl azides can be reacted with a carboxylic acid compound in the presence of a base. Hydrogen azide can be reacted with a mixed acid anhydride obtained from an acid chloride (such as an acid chloride, an acid bromide or an acid iodide, and preferably an acid chloride) or a carboxylic acid compound and a $C_1$-$C_6$ alkyl halogenoformate (such as methyl chloroformate, ethyl chloroformate, ethyl bromoformate, propyl chloroformate, butyl chloroformate, isobutyl chloroformate, isobutyl bromoformate or hexyl chloroformate, and preferably ethyl chloroformate or isobutyl chloroformate) in the presence of a base. Alkali metal azides or tri-$C_1$-$C_4$ alkylsilyl azides can be reacted with a mixed acid anhydride obtained from an acid chloride or a carboxylic acid compound and a $C_1$-$C_6$ alkyl halogenoformate.

Preferable examples of the base used include alkali metal hydroxides such as lithium hydroxide, sodium hydroxide and potassium hydroxide; alkali metal carbonates such as lithium carbonate, sodium carbonate and potassium carbonate; alkali metal bicarbonates such as sodium bicarbonate and potassium bicarbonate; alkali metal alkoxides such as a lithium methoxide, sodium methoxide, sodium ethoxide and potassium tert-butoxide; and organic amines such as triethylamine, diisopropylethylamine, N-methylmorpholine, pyridine, 4-dimethylaminopyridine, 1,5-diazabicyclo[4.3.0]-5-nonene and 1,8-diazabicyclo[5.4.0]-7-undecene. When a phosphoryl azide is reacted, the base is preferably an organic amine, for example. When hydrogen azide is reacted, the base is preferably an alkali metal hydroxide or an

alkali metal carbonate, for example, and more preferably sodium hydroxide or potassium hydroxide. The solvent used varies according to the starting material and the base used, and is not particularly limited insofar as it does not inhibit the reaction and allows the starting material to be dissolved therein to a certain extent. Preferable examples of the solvent include hydrocarbons such as hexane, cyclohexane, benzene, toluene and xylene; halogenated hydrocarbons such as dichloromethane, chloroform, carbon tetrachloride and 1,2-dichloroethane; ethers such as ethyl ether, tetrahydrofuran and dioxane; ketones such as acetone and 2-butanone; nitriles such as acetonitrile; amides such as N,N-dimethylformamide, N,N-dimethylacetamide and N-methyl-2-pyrrolidinone; sulfoxides such as dimethyl sulfoxide; and mixed solvents thereof. When a phosphoryl azide is reacted, the solvent is preferably a hydrocarbon, a halogenated hydrocarbon or an ether, for example. When hydrogen azide or an alkali metal azide is reacted, the solvent is preferably a halogenated hydrocarbon, an ether or an amide, for example. When a trialkylsilyl azide is reacted, the solvent is preferably a hydrocarbon, a halogenated hydrocarbon or an ether, and more preferably a hydrocarbon or an ether. The reaction temperature must be a temperature that can complete the reaction without promoting formation of an undesirable by-product, and is preferably -10°C to 100°C, for example. Under preferable reaction conditions, the reaction is completed in 30 minutes to 24 hours, for example.

The reaction of heating the resulting acid azide compound is preferably performed in an inert solvent. The inert solvent used is the same as the above solvent, and is more preferably an aromatic hydrocarbon, an amide or an ether, and still more preferably an aromatic hydrocarbon or an ether. The reaction temperature varies according to the type of the solvent and the like and is preferably room temperature to 150°C, for example. The reaction time varies according to the reaction temperature and the like, and the reaction is preferably completed in 30 minutes to 24 hours, for example. The progress of the reaction can be monitored by a known chromatography technique. An undesirable by-product can be removed by a technique known to a person skilled in the art such as a conventional chromatography technique, extraction or/and crystallization.

In the method ii), the conversion can be performed by a method described in many known documents. For example, the method is preferably a method of converting the corresponding amine compound into the isocyanate compound by condensation with triphosgene, trichloromethyl chloroformate, di-tert-butyl dicarbonate or the like in the presence or absence of a base (see HANESSIAN, S. et al., Tetrahedron Letters, vol.41, 2000, p.4999-5003; K. Kurita et al., Org. Synth., VI, 1988, p.715; and Knoelker, H.-J. et al., Synlett, vol.8, 1997, p.925-928, for example).

In the reaction, an inert solvent is preferably used. The inert solvent varies according to the starting material and the base used, and is not particularly limited insofar as it does not inhibit the reaction and allows the starting material to be dissolved therein to a certain extent. Preferable examples of the inert solvent include hydrocarbon such as petroleum ether; amides such as formamide, N,N-dimethylformamide, N,N-dimethylacetamide, N-methyl-2-pyrrolidone, N-methylpyrrolidinone and hexamethylphosphoric acid triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutyronitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. Preferable examples of the base used include alkali metal carbonates such as sodium carbonate, potassium carbonate and lithium carbonate; alkali metal bicarbonates such as sodium bicarbonate, potassium bicarbonate and lithium bicarbonate; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU). The base is more preferably an organic base, for example. The reaction temperature varies according to the starting material, the inert solvent used, and the like; however, the reaction temperature must be a temperature that can complete the reaction without promoting formation of an undesirable by-product, and is preferably -20 to 150°C, for example. The reaction time varies according to the starting material, the inert solvent used, the reaction temperature and the like, and the reaction is preferably completed in 0.5 to 24 hours, for example. The progress of the reaction can be monitored by a known chromatography technique. An undesirable by-product can be removed by a technique known to a person skilled in the art such as a conventional chromatography technique, extraction or/and crystallization.

[General Preparation Method 2]

Typically used General Preparation Method 2 for the compound of the general formula (I) of the present invention will be described below.

[Formula 12]

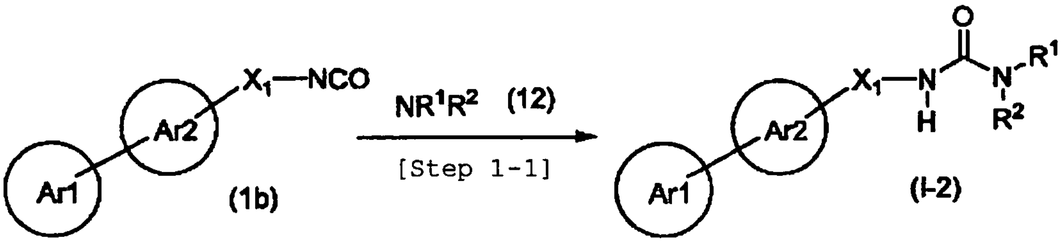


In the formula, $Ar_1$, $Ar_2$, $R^1$, $R^2$ and $X_1$ are as defined above.
The above General Preparation Method 2 is an example of a method for preparing the compound of the general formula (I-2) comprising reacting an isocyanate compound (1b) with an amine compound (12) by addition reaction in the above Step 1-1.

[Preparation of compound of general formula (I-2)]

The compound of the general formula (I-2) can be prepared by reacting an isocyanate compound (1b) with an amine compound (12) according to the above Step 1-1.

[Preparation of amine compound (12)]

The amine compound (12) is commercially available or can be obtained by a technique known to a person skilled in the art. If not commercially available, the amine compound (12) can be obtained from a corresponding alcohol compound, alkyl halide compound, nitro compound, nitrile compound, oxime compound, azide compound or amide compound by a method known to a person skilled in the art according to the above Step 2-1, for example.

[Preparation of isocyanate compound (1b)]

[Formula 13]

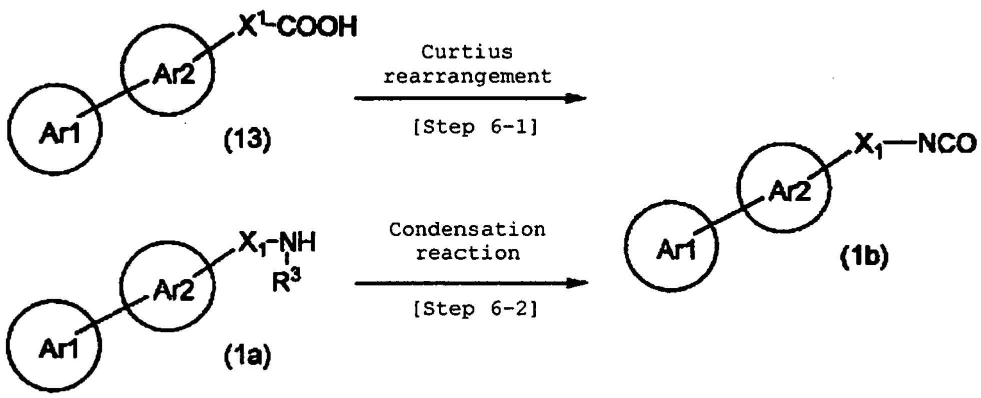


In the formula, $Ar_1$, $Ar_2$, $R^1$ and $X_1$ are as defined above; and $R^3$ represents a hydrogen atom.
The isocyanate compound (1b) can be obtained by the method described in the above [Preparation of isocyanate compound (2)] using a method known to many persons skilled in the art, for example. The reaction shown above is an example of such a method. Preferably, the isocyanate compound (1b) can be prepared from a carboxylic acid compound (13) according to Step 6-1, for example. Specifically, Step 6-1 varies according to the starting material and is not

particularly limited insofar as the conditions are similar to those in this reaction. A known method described in many documents may be used for the reaction (see K. Ninomiya et al., Tetrahedron, vol.30, 1974, p.2151, for example). Preferably, the isocyanate compound (1b) can be obtained by reacting the carboxylic acid compound (13) with 1.0 to 100.0 equivalents of an azidating agent with respect to the carboxylic acid compound (13) in the presence or absence of 1.0 to 100.0 equivalents of a base with respect to the carboxylic acid compound (13); and then heating the resulting acid azide compound in a solvent, for example. The base, the solvent, the reaction temperature, the reaction time and the purification method used in this reaction are the same as in the method i) of the above [Preparation of isocyanate (2)].

The isocyanate compound (1b) can also be prepared from an amine compound (1a) according to Step 6-2. Specifically, Step 6-2 varies according to the starting material and is not particularly limited insofar as the conditions are similar to those in this reaction. A known method described in many documents may be used for the reaction. For example, the method is the same as the method ii) of the above [Preparation of isocyanate (2)]. Preferably, the isocyanate compound (1b) can be prepared by condensing the amine compound (1a) with triphosgene, for example (see HANESSIAN, S. et al., Tetrahedron Letters, vol.41, 2000, p.4999-5003, for example). Preferably, the isocyanate compound (1b) can be obtained by stirring 1.0 to 100.0 equivalents of triphosgene with respect to the amine compound (1a) in a solvent in the presence or absence of 1.0 to 100.0 equivalents of a base with respect to the amine compound (1a), for example. The base, the solvent, the reaction temperature, the reaction time and the purification method used in this reaction are the same as in the method ii) of the above [Preparation of isocyanate (2)].

[Preparation of carboxylic acid compound (13)]

[Formula 14]

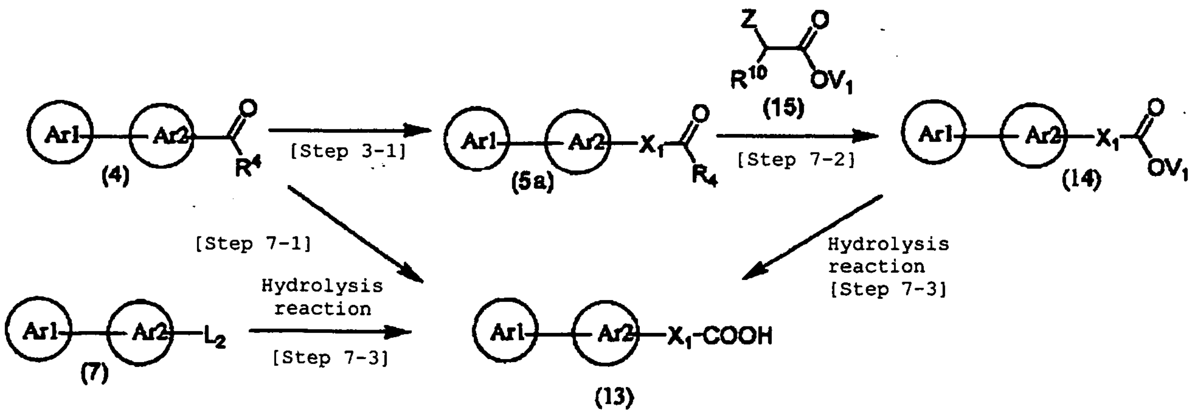


In the formula, $Ar_1$, $Ar_2$ and $X_1$ are as defined above; $L_2$ represents an alkoxycarbonyl group such as a methyl ester group; $R^4$ and $R^{10}$ each represent a group selected from Substituent Group A3 shown above; $V_1$ represents a protecting group for a carboxyl group such as a methyl group, an ethyl group, a benzyl group, an allyl group, a triphenylmethyl group, a tert-butyl group or a tert-butyldimethylsilyl group; and Z represents a phosphate group such as a diethylphosphonyl group, a diphenylphosphonyl group or a bis(2,2,2-trifluoroethyl)phosphonyl group, a phosphonium salt such as triphenylphosphonium bromide, or a silyl group such as a trimethylsilyl group.

The carboxylic acid compound (13) is prepared by hydrolysis of an ester compound (14) according to Step 7-3. Specifically, Step 7-3 varies according to the starting material and is not particularly limited insofar as the conditions are similar to those in this reaction. A known method described in many documents may be used for the reaction (see Shin Jikken Kagaku Koza (New Courses in Experimental Chemistry), vol.22, Yuki Gosei (Organic Synthesis) [IV], edited by The Chemical Society of Japan, Maruzen Co., Ltd., November 1992, p.6-11, for example). Preferably, the ester compound (14) is stirred in a solvent in the presence of 1.0 to 100.0 equivalents of a base or an acid with respect to the ester compound (14), for example. The base used varies according to the starting material and is not particularly limited. Preferable examples of the base include sodium hydride, sodium hydroxide, potassium hydroxide, potassium carbonate, sodium carbonate, cesium carbonate and barium carbonate. The acid used varies according to the starting material and is not particularly limited. Preferable examples of the acid include inorganic acids such as hydrochloric acid and sulfuric acid; organic acids such as trifluoroacetic acid and p-toluenesulfonic acid; and Lewis acids such as boron trichloride.

The solvent used varies according to the starting material, and is not particularly limited insofar as it does not inhibit the reaction and allows the starting material to be dissolved therein to a certain extent. Preferable examples of the solvent include alcohol solvents such as methanol, ethanol and ethylene glycol; and ether solvents such as tetrahydrofuran. In the case of acid hydrolysis, an organic acid such as acetic acid or formic acid may be used as a solvent. The reaction temperature must be a temperature that can complete the reaction without promoting formation of an undesirable by-product, and is preferably room temperature to 100°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

When $X_1$ is a single bond, the carboxylic acid compound (13) can be prepared from a carbonyl compound (4), wherein $R^4$ represents a hydrogen atom, by oxidation reaction according to Step 7-1. Specifically, Step 7-1 varies according to the starting material and is not particularly limited insofar as the conditions are similar to those in this reaction. A known method described in many documents may be used for the reaction (see Shin Jikken Kagaku Koza (New Courses in Experimental Chemistry), vol.22, Yuki Gosei (Organic Synthesis) [IV], edited by The Chemical Society of Japan, Maruzen Co., Ltd., November 1992, p.1-5, for example). The carboxylic acid compound (13) can also be prepared from a compound (7), wherein $L_2$ represents an ester group such as a methyl ester group, by ester hydrolysis according to the above Step 7-3.

[Preparation of ester compound (14)]

The ester compound (14) can be prepared from a carbonyl compound (5a) and a compound (15) according to Step 7-2. Specifically, the coupling reaction in Step 7-2 varies according to the starting material and is not particularly limited insofar as the conditions are similar to those in this reaction. A method known to a person skilled in the art may be used for the reaction. Preferable examples of the method include Wittig reaction, Horner-Emmons reaction and Peterson reaction (see Shin Jikken Kagaku Koza (Courses in Experimental Chemistry), vol.19, Yuki Gosei (Organic Synthesis) [I], edited by The Chemical Society of Japan, Maruzen Co., Ltd., June 1992, p.57-85, for example).

In Wittig reaction, preferably, the compound (15), wherein Z represents a phosphonium salt, and 0.5 to 2.0 equivalents of the carbonyl compound (5a) with respect to the compound (15) are stirred in a solvent in the presence of 1.0 to 5.0 equivalents of a base with respect to the compound (15), for example. This reaction may be a method of first treating the compound (15) and a base to form a phosphorus ylide and then adding the carbonyl compound (5a) to the ylide; or a method of adding a base in the presence of the compound (15) and the carbonyl compound (5a). This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent used include polar solvents such as nitromethane, acetonitrile, 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; halogenated solvents such as chloroform and methylene chloride; and water and a mixed solvent thereof. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; and alkali metal hydrides such as sodium hydride. The reaction temperature must be a temperature that can complete the reaction without promoting formation of an undesirable by-product, and is preferably, for example, -78 to 150°C. Under preferable reaction conditions, the reaction is completed preferably, for example, in 1 to 24 hours, and the progress of the reaction can be monitored by a known chromatography technique. An undesirable by-product can be removed by a technique known to a person skilled in the art such as a conventional chromatography technique, extraction or/and crystallization.

In Horner-Emmons reaction, preferably, for example, the compound (15), wherein W is a phosphite group, is stirred with 0.5 to 2.0 equivalents to of the carbonyl compound (5a) with respect to the compound (15) in a solvent in the presence of 1.0 to 5.0 equivalents of a base with respect to the compound (15). This reaction may be a method of first treating the compound (15) and a base to form a carbanion and then adding the carbonyl compound (5a) to the carbanion; or a method of adding a base in the presence of the compound (15) and the carbonyl compound (5a). This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent include polar solvents such as 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; and water and a mixed solvent thereof.

The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; alkali metal hydrides such as sodium hydride; and alkali metal ammonia salts such as sodium amide. The reaction temperature must be a temperature that can complete the reaction without promoting formation of an undesirable by-product, and is preferably, for example, -78 to 150°C. Under preferable reaction conditions, the reaction is preferably, for example, completed in 1 to 24 hours, and the progress of the reaction can be monitored by a known chromatography technique. An undesirable by-product can be removed by a technique known to a person skilled in the art such as a conventional chromatography technique, extraction or/and crystallization.

In Peterson reaction, for example, the compound (15), wherein W is a silyl group, is stirred with preferably, for example, 0.5 to 2.0 equivalents of the carbonyl compound (5a) with respect to the compound (15) in a solvent in the presence of preferably, for example, 1.0 to 5.0 equivalents of a base with respect to the compound (15). This reaction may be a method of first treating the compound (15) and a base to form a carbanion and then adding the carbonyl compound (5a) to the carbanion; or a method of adding a base in the presence of the compound (15) and the carbonyl compound (5a). This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent include polar solvents such as 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; and water and a mixed solvent thereof. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; alkali metal hydrides such as sodium hydride; and alkali metal ammonia salts such as sodium amide. The reaction temperature must be a temperature that can complete the reaction without promoting formation of an undesirable by-product, and is preferably, for example, -78 to 150°C. Under preferable reaction conditions, the reaction is preferably, for example, completed in 1 to 24 hours, and the progress of the reaction can be monitored by a known chromatography technique. An undesirable by-product can be removed by a technique known to a person skilled in the art such as a conventional chromatography technique, extraction or/and crystallization.

[General Preparation Method 3]

Typically used General Preparation Method 3 for the compound of the general formula (I) of the present invention will be described below.

[Formula 15]

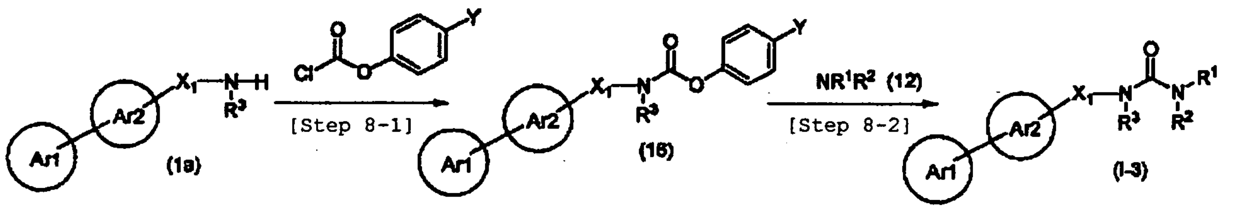


In the formula, $Ar_1$, $Ar_2$, $R^1$, $R^2$ and $X_1$ are as defined above; $R^3$ represents a group selected from Substituent Group A4 shown above; and Y represents a hydrogen atom or a nitro group.

The above General Preparation Method 3 is an example of a method for preparing the compound of the general formula (I-3) comprising converting an amine compound (1a) and phenyl chloroformate or p-nitrophenyl chloroformate into a carbamate compound (16) according to Step 8-1; and then reacting the carbamate compound (16) with an amine compound (12) by nucleophilic substitution reaction according to Step 8-2.

[Preparation of compound of general formula (I-3)]

The compound of the general formula (I-3) can be prepared by reacting a carbamate compound (16) with an amine compound (12) according to Step 8-2. Specifically, Step 8-2 varies according to the starting material and is not particularly limited insofar as the conditions are similar to those in this reaction. A known method described in many documents may be used for the reaction (see Thavonekham, B. et al., Synthesis, vol.10, 1997, p.1189-1194; and Raju, B. et al., Bioorg. Med. Chem. Lett., vol.8, p.3043-3048, for example). In this reaction, the carbamate compound (16) is preferably reacted with 1.0 to 10.0 equivalents of the amine compound (12) with respect to the carbamate compound (16) in an inert solvent in the presence or absence of 1.0 to 100.0 equivalents of a base with respect to the carbamate compound (16). The inert solvent used is not particularly limited insofar as it does not inhibit the reaction and allows the starting material to be dissolved therein to a certain extent. Preferable examples of the inert solvent include hydrocarbons such as pentane, hexane, octane, petroleum ether and ligroin; amides such as formamide, N,N-dimethylformamide, N, N-dimethylacetamide, N-methyl-2-pyrrolidone, N-methylpyrrolidinone and hexamethylphosphoric acid triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutylonitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof. The inert solvent is more preferably N,N-dimethylformamide or tetrahydrofuran, for example. Preferable examples of the base used include alkali metal carbonates such as sodium carbonate, potassium carbonate and lithium carbonate; alkali metal bicarbonates such as sodium bicarbonate, potassium bicarbonate and lithium bicarbonate; alkali metal hydrides such as lithium hydride, sodium hydride and potassium hydride; alkali metal hydroxides such as sodium hydroxide, potassium hydroxide, barium hydroxide and lithium hydroxide; alkali metal alkoxide such as sodium methoxide, sodium ethoxide, sodium t-butoxide, potassium methoxide, potassium ethoxide, potassium t-butoxide and lithium methoxide; alkali metal trialkylsiloxides such as sodium trimethylsiloxide, potassium trimethylsiloxide and lithium trimethylsiloxide; alkali metal mercaptans such as sodium methyl mercaptan and sodium ethyl mercaptan; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU); and organometallic bases such as lithium diisopropylamide and lithium bis(trimethylsilyl)amide. The base is more preferably triethylamine, for example. The reaction temperature is a temperature that can complete the reaction without promoting formation of an undesirable by-product and varies according to the starting material, the inert solvent used, and the like. The reaction temperature is preferably 0°C to 100°C, for example. Under preferable reaction conditions, the reaction time is preferably 0.5 to 24 hours, for example, and the progress of the reaction can be monitored by a known chromatography technique. An undesirable by-product can be removed by a technique known to a person skilled in the art such as a conventional chromatography technique, extraction or/and crystallization.

[Preparation of carbamate compound (16)]

The carbamate compound (16) can be prepared by reacting an amine compound (1a) with phenyl chloroformate or p-nitrophenyl chloroformate according to Step 8-1. Specifically, Step 8-1 varies according to the starting material and is not particularly limited insofar as the conditions are similar to those in this reaction. A known method described in many documents may be used for the reaction (see Atwal, K.S. et al., J. Med. Chem., vol.39, 1996, p.304-313, for example). In this reaction, the amine compound (1a) is preferably reacted with preferably 1.0 to 10.0 equivalents of phenyl chloroformate or p-nitrophenyl chloroformate with respect to the amine compound (1a), for example, in an inert solvent in the presence of preferably 1.0 to 100.0 equivalents of a base with respect to the amine compound (1a), for example. The inert solvent used is not particularly limited insofar as it does not inhibit the reaction and allows the starting material to be dissolved therein to a certain extent. Preferable examples of the inert solvent include hydrocarbons such as pentane, hexane, octane, petroleum ether and ligroin; amides such as formamide, N,N-dimethylformamide, N,N-dimethylacetamide, N-methyl-2-pyrrolidone, N-methylpyrrolidinone and hexamethylphosphoric acid triamide; ethers such as diethyl ether, diisopropyl ether, tetrahydrofuran, dioxane, dimethoxyethane and diethylene glycol dimethyl ether; sulfoxides such as dimethyl sulfoxide and sulfolane; nitriles such as acetonitrile and isobutylonitrile; esters such as ethyl formate, ethyl acetate, propyl acetate, butyl acetate and diethyl carbonate; ketones such as acetone, methyl ethyl ketone, 4-methyl-2-pentanone, methyl isobutyl ketone, isophorone and cyclohexanone; nitro compounds such as nitroethane and nitrobenzene; halogenated hydrocarbons such as dichloromethane, 1,2-dichloroethane, dichlorobenzene, chloroform and carbon tetrachloride; aromatic hydrocarbons such as benzene, toluene and xylene; and mixed solvents thereof.

The inert solvent is more preferably N,N-dimethylacetamide or tetrahydrofuran, for example. Preferable examples of the base used include alkali metal carbonates such as sodium carbonate, potassium carbonate and lithium carbonate; alkali metal bicarbonates such as sodium bicarbonate, potassium bicarbonate and lithium bicarbonate; alkali metal hydrides such as lithium hydride, sodium hydride and potassium hydride; alkali metal hydroxides such as sodium hydroxide, potassium hydroxide, barium hydroxide and lithium hydroxide; alkali metal alkoxide such as sodium methoxide, sodium ethoxide, sodium t-butoxide, potassium methoxide, potassium ethoxide, potassium t-butoxide and lithium methoxide; alkali metal trialkylsiloxides such as sodium trimethylsiloxide, potassium trimethylsiloxide and lithium trimethylsiloxide; alkali metal mercaptans such as sodium methyl mercaptan and sodium ethyl mercaptan; organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU); and organometallic bases such as lithium diisopropylamide and lithium bis(trimethylsilyl)amide. The base is more preferably triethylamine, for example. The reaction temperature is a temperature that can complete the reaction without promoting formation of an undesirable by-product and varies according to the starting material, the inert solvent used, and the like. The reaction temperature is preferably -20°C to 100°C, for example. Under preferable reaction conditions, the reaction time is 0.5 to 24 hours, for example, and the progress of the reaction can be monitored by a known chromatography technique. An undesirable by-product can be removed by a technique known to a person skilled in the art such as a conventional chromatography technique, extraction or/and crystallization.

[General Preparation Method 4]

Typically used General Preparation Method 4 for the compound of the general formula (I) of the present invention will be described below.

[Formula 16]

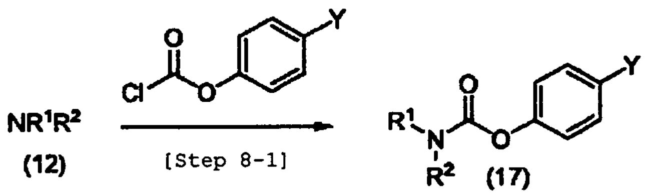


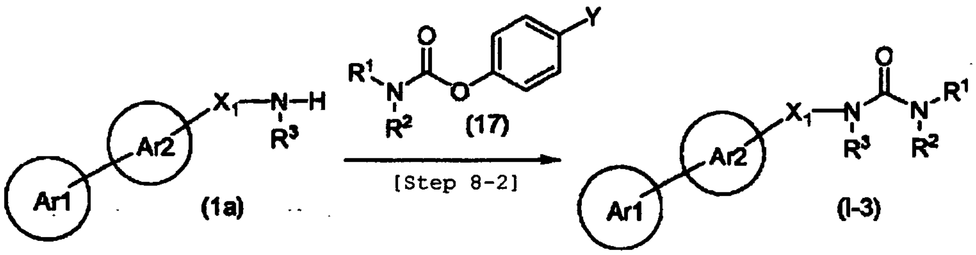


In the formula, $Ar_1$, $Ar_2$, $R^1$, $R^2$ and $X_1$ are as defined above; $R^3$ represents a group selected from Substituent Group A4 shown above; and Y represents a hydrogen atom or a nitro group.

The above General Preparation Method 4 is an example of a method for preparing the compound of the general formula (I-3) comprising converting an amine compound (12) and phenyl chloroformate or p-nitrophenyl chloroformate into a carbamate compound (17) according to the above Step 8-1; and then reacting the carbamate compound (17) with an amine compound (12) by nucleophilic substitution reaction according to the above Step 8-2.

[Preparation of compound (I-3)]

The compound (I-3) can be prepared from an amine compound (1a) and a carbamate compound (17) according

to the above Step 8-2 by a method known to a person skilled in the art.

[Preparation of carbamate compound (17)]

The carbamate compound (17) can be prepared from an amine compound (12) according to the above Step 8-1 by a method known to a person skilled in the art.

[General Preparation Method 5]

Typically used General Preparation Method 5 for the compound of the general formula (I) of the present invention will be described below.

[Formula 17]

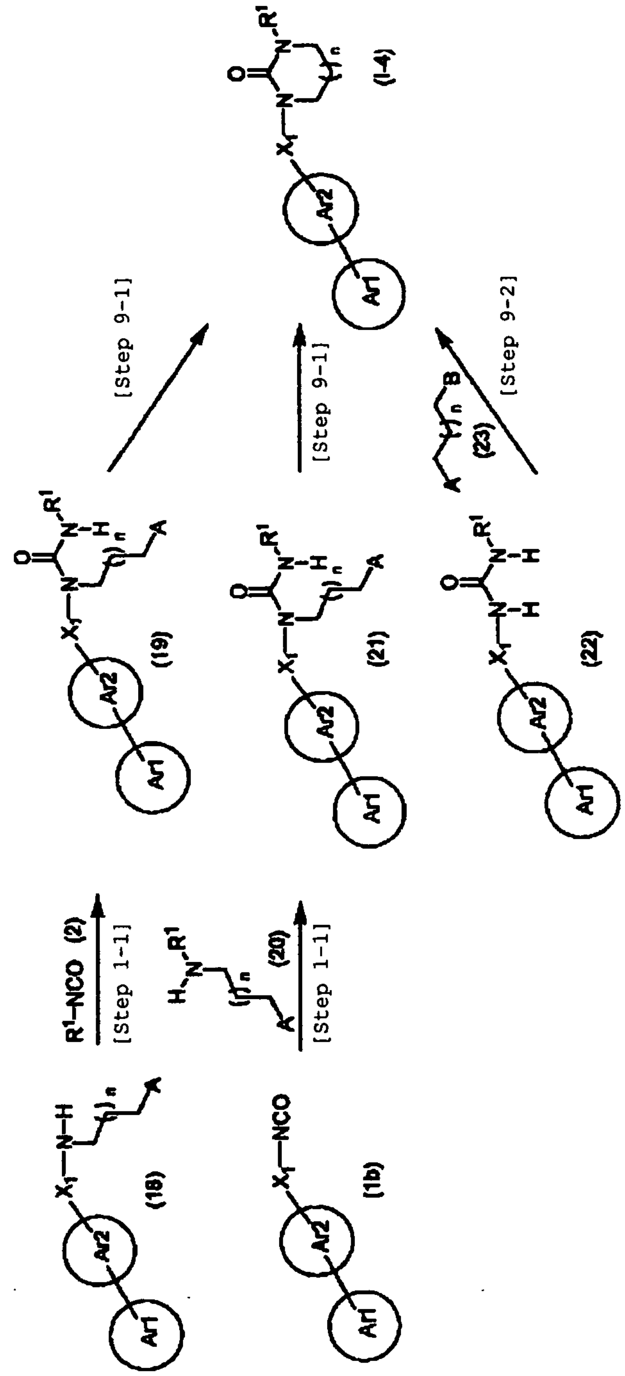


In the formula, $Ar_1$, $Ar_2$, $R^1$ and $X_1$ are as defined above; A and B each represent a halogen atom such as a chlorine atom, a bromine atom or an iodine atom, or a sulfonate group such as a methanesulfonate group, a p-toluenesulfonate group or a trifluoromethanesulfonate group; and n represents an integer of 0 to 3.

The above General Preparation Method 5 is an example of a method for preparing the compound of the general formula (I-4) comprising converting an amine compound (18) and an isocyanate compound (2) into a urea compound (19) or converting an isocyanate compound (1b) and an amine compound (20) into a urea compound (21) according to the above Step 1-1, respectively; and then subjecting the urea compound (19) or (21) to intramolecular cyclization reaction according to Step 9-1, or reacting a urea compound (22) with a compound (23) according to Step 9-2.

[Preparation of compound of general formula (I-4)]

The compound of the general formula (I-4) can be prepared from a urea compound (19) or (21) by intermolecular cyclization reaction according to Step 9-1. Specifically, Step 9-1 varies according to the starting material and is not particularly limited insofar as the conditions are similar to those in this reaction. A known method described in many documents may be used for the reaction (see Santilli, A., J. Org. Chem., vol.31, 1966, p.4268). The method is preferably a method of stirring the urea compound (19) or (21) in a solvent in the presence of 1.0 to 10.0 equivalents of a base with respect to the urea compound (19) or (21), for example. The base used in this reaction varies according to the starting material and is not particularly limited. Preferable examples of the base used include alkali metal hydrides such as sodium hydride and lithium hydride; alkali metal salts such as potassium carbonate, sodium carbonate and cesium carbonate; and metal alkoxides such as sodium methoxide and tert-butyl potassium. The solvent used in this reaction varies according to the starting material, and is not particularly limited insofar as it does not inhibit the reaction and allows the starting material to be dissolved therein to a certain extent. Preferable examples of the solvent include ether solvents such as tetrahydrofuran, 1,4-dioxane and diethyl ether; halogenated solvents such as methylene chloride, 1,2-dichloroethane and chloroform; polar solvents such as dimethylformamide and N-methylpyrrolidone; non-polar solvents such as toluene and benzene; and mixtures thereof. The reaction temperature must be a temperature that can complete the reaction without promoting formation of an undesirable by-product, and is preferably 0°C to 200°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique, extraction or/and crystallization.

The compound of the general formula (I-4) can also be prepared by reacting a urea compound (22) with a compound (23) according to Step 9-2. Specifically, Step 9-2 varies according to the starting material and is not particularly limited insofar as the conditions are similar to those in this reaction. A known method described in many documents may be used for the reaction (see Santilli, A., J. Org. Chem., vol.31, 1966, p.4268). The method is preferably a method of stirring 1.0 to 10.0 equivalents of the compound (23) with respect to the urea compound (22) in a solvent in the presence of 1.0 to 10.0 equivalents of a base with respect to the urea compound (22), for example. The base used in this reaction varies according to the starting material and is not particularly limited. Preferable examples of the base used include alkali metal hydrides such as sodium hydride and lithium hydride; alkali metal salts such as potassium carbonate, sodium carbonate and cesium carbonate; and metal alkoxides such as sodium methoxide and tert-butyl potassium. The solvent used in this reaction varies according to the starting material, and is not particularly limited insofar as it does not inhibit the reaction and allows the starting material to be dissolved therein to a certain extent. Preferable examples of the solvent include ether solvents such as tetrahydrofuran, 1,4-dioxane and diethyl ether; halogenated solvents such as methylene chloride, 1,2-dichloroethane and chloroform; polar solvents such as dimethylformamide and N-methylpyrrolidone; non-polar solvents such as toluene and benzene; and mixtures thereof. The reaction temperature must be a temperature that can complete the reaction without promoting formation of an undesirable by-product, and is preferably 0°C to 200°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique, extraction or/and crystallization.

[Preparation of amine compound (18)]

The amine compound (18) can be prepared from a compound (3) according to the above Step 2-1 by a method known to a person skilled in the art.

[Preparation of amine compound (20)]

The amine compound (20) is commercially available or can be prepared from a corresponding starting material by the same method as in the above Step 2-1 using a method known to a person skilled in the art.

The present inventors performed the following tests in order to exhibit utility of the compounds of the general formulas (I) of the present invention.

Test Example 1 [Quantification of Aβ peptide in neuronal culture from rat fetus brain]

(1) Rat primary neuronal culture

Primary neuronal cultures were prepared from the cerebral cortex of embryonic day 18 Wistar rats (Charles

River Japan, Yokohama, Japan). Specifically, the embryos were aseptically removed from pregnant rats under ether anesthesia. The brain was isolated from the embryo and immersed in an ice-cold L-15 medium (such as Invitrogen Corp. Cat #11415-064, Carlsbad, CA, USA, or SIGMA L1518). The cerebral cortex was collected from the isolated brain under a stereoscopic microscope. The cerebral cortex fragments collected were enzymatically treated in an enzyme solution containing 0.25% trypsin (Invitrogen Corp. Cat #15050-065, Carlsbad, CA, USA) and 0.01% DNase (Sigma D5025, St. Louis, MO, USA) at 37°C for 30 minutes to disperse the cells. Here, the enzymatic reaction was stopped by adding inactivated horse serum to the solution. The enzymatically treated solution was centrifuged at 1,500 rpm for five minutes to remove the supernatant. 5 to 10 ml of a medium was added to the resulting cell mass. Neurobasal medium (Invitrogen Corp. Cat #21103-049, Carlsbad, CA, USA) supplemented with 2% B27 supplement (Invitrogen Corp. Cat #17504-044, Carlsbad, CA, USA), 25 μM 2-mercaptoethanol (2-ME, WAKO Cat #139-06861, Osaka, Japan), 0.5 mM L-glutamine (Invitrogen Corp. Cat #25030-081, Carlsbad, CA, USA), and Antibiotics-Antimycotics (Invitrogen Corp. Cat #15240-062, Carlsbad, CA, USA) was used as the medium (Neurobasal/B27/2-ME). However, the above Neurobasal medium not supplemented with 2-ME (Neurobasal/B27) was used for the assay. The cells were redispersed by mild pipetting of the cell mass to which the medium was added. The cell dispersion was filtered through a 40-μm nylon mesh (Cell Strainer, Cat #35-2340, Becton Dickinson Labware, Franklin Lakes, NJ, USA) to remove the remaining cell mass, and thus a neuronal cell suspension was obtained. The neuronal cell suspension was diluted with the medium and then plated in a volume of 100 μl/well at an initial cell density of $5 \times 10^5$ cells/cm$^2$ in a 96-well polystyrene culture plate precoated with poly-L or D-lysine (Falcon Cat #35-3075, Becton Dickinson Labware, Franklin Lakes, NJ, USA coated with poly-L-lysine using the method shown below, or BIOCOAT™ cell environments Poly-D-lysine cell ware 96-well plate, Cat #35-6461, Becton Dickinson Labware, Franklin Lakes, NJ, USA). Poly-L-lysine coating was carried out as follows. 100 μg/ml of a poly-L-lysine (SIGMA P2636, St. Louis, MO, USA) solution was aseptically prepared with a 0.15 M borate buffer (pH 8.5). 100 μg/well of the solution was added to the 96-well polystyrene culture plate and incubated at room temperature for one or more hours or at 4°C overnight or longer. The coated 96-well polystyrene culture plate was washed with sterile water four or more times, and then dried or rinsed with, for example, sterile PBS or medium, and used for cell plating. The plated cells were cultured in the culture plate at 37°C in 5% $CO_2$-95% air for one day. Then, the total amount of the medium was replaced with a fresh Neurobasal™/B27/2-ME medium, and then the cells were cultured for further three days.

Addition of compounds

The drug was added to the culture plate on Day 4 of culture as follows. The total amount of the medium was removed from the wells, and 180 μl/well of Neurobasal medium not containing 2-ME and containing 2% B-27 (Neurobasal/B27) was added thereto. A solution of the test compound in dimethyl sulfoxide (hereinafter abbreviated as DMSO) was diluted with Neurobasal/B27 to a concentration 10-fold higher than the final concentration. 20 μl/well of the dilution was added to and sufficiently mixed with the medium. The final DMSO concentration was 1% or less. Only DMSO was added to the control group.

Sampling

The cells were cultured for three days after addition of the compound, and the total amount of the medium was collected. The resulting medium was used as an ELISA sample. The sample was not diluted for ELISA measurement of Aβx-42 and diluted to 5-fold with a diluent supplied with an ELISA kit for ELISA measurement of Aβx-40.

Evaluation of cell survival

Cell survival was evaluated by an MTT assay according to the following procedure. After collecting the medium, 100 μl/well of a pre-warmed medium was added to the wells. Further, 8 μl/well of a solution of 8 mg/ml of MTT (SIGMA M2128, St. Louis, MO, USA) in D-PBS(-) (Dulbecco's phosphate buffered Saline, SIGMA D8537, St. Louis, MO, USA) was added to the wells. The 96-well polystyrene culture plate was incubated in an incubator at 37°C in 5% $CO_2$-95% air for 20 minutes. 100 μl/well of an MTT lysis buffer was added thereto, and MTT formazan crystals were sufficiently dissolved in the buffer in the incubator at 37°C in 5% $CO_2$-95% air. Then, the absorbance at 550 nm in each well was measured. The MTT lysis buffer was prepared as follows. 100 g of SDS (sodium dodecyl sulfate (sodium lauryl sulfate), WAKO 191-07145, Osaka, Japan) was dissolved in a mixed solution of 250 mL of N,N'-dimethylformamide (WAKO 045-02916, Osaka, Japan) and 250 mL of distilled water. 350 μl each of concentrated hydrochloric acid and acetic acid were further added to the solution to allow the solution to have a final pH of about 4.7.
Upon measurement, wells having no cells plated and containing only the medium and MTT solution were set as background (bkg). The measured values were respectively applied to the following formula including subtracting bkg values from them. Thus, the proportion against the control group (group not treated with the drug, CTRL) (% of CTRL) was calculated to compare and evaluate cell survival activities.

$$\% \text{ of CTRL} = (A550_sample-A550_bkg)/(A550_CTRL-bkg) \times 100$$

(A550_sample: absorbance at 550 nm of sample well,

A550_bkg: absorbance at 550 nm of background well,
A550_CTRL: absorbance at 550 nm of control group well)

Aβ ELISA

Aβ ELISA employed Human/Rat β Amyloid (42) ELISA Kit Wako (#290-62601) and Human/Rat β Amyloid (40) ELISA Kit Wako (#294-62501) from Wako Pure Chemical Industries, Ltd., or Human Amyloid beta (1-42) Assay Kit (#27711) and Human Amyloid beta (1-40) Assay Kit (#27713) from Immuno-Biological Laboratories, Co., Ltd. (IBL Co., Ltd.). Aβ ELISA was carried out according to the protocols recommended by the manufacturers (methods described in the attached documents). However, the Aβ calibration curve was created using beta-amyloid peptide 1-42, rat and beta-amyloid peptide 1-40, rat (Calbiochem, #171596 [$A\beta_{42}$], #171593 [$A\beta_{40}$]). The results are shown in Table 1 as percentage to the Aβ concentration in the medium of the control group (% of CTRL).

(2) Accordingly, the compound of the present invention was proved to have an Aβ42 production reducing effect.

Consequently, since the compound of the general formula (I) or a pharmaceutically acceptable salt thereof have an Aβ42 production reducing effect, the present invention can particularly provide a prophylactic or therapeutic agent for a neurodegenerative disease caused by Aβ such as Alzheimer's disease and Down's syndrome.
[Table 1]

| Test compound | Aβ42 production reducing effect IC50 (μM) |
|---|---|
| Example 6 | 5.2 |
| Example 8 | 1.71 |
| Example 10 | 1.22 |
| Example 11 | 0.97 |
| Example 14 | 0.86 |
| Example 15 | 0.16 |
| Example 16 | 1.42 |
| Example 17 | 0.56 |
| Example 18 | 0.86 |
| Example 19 | 2.07 |
| Example 20 | 1.61 |
| Example 21 | 1.66 |
| Example 23 | 1.05 |
| Example 24 | 0.22 |
| Example 25 | 0.19 |
| Example 26 | 0.23 |
| Example 27 | 0.54 |
| Example 29 | 3.84 |

The "salt" refers to a pharmaceutically acceptable salt, and is not particularly limited insofar as it forms a pharmaceutically acceptable salt with the compound of the general formula (I) as a prophylactic or therapeutic agent for a disease caused by Aβ. Preferable specific examples of the salt include hydrohalides (such as hydrofluorides, hydrochlorides, hydrobromides and hydroiodides), inorganic acid salts (such as sulfates, nitrates, perchlorates, phosphates, carbonates and bicarbonates), organic carboxylates (such as acetates, oxalates, maleates, tartrates, fumarates and citrates), organic sulfonates (such as methanesulfonates, trifluoromethanesulfonates, ethanesulfonates, benzenesulfonates, toluenesulfonates and camphorsulfonates), amino acid salts (such as aspartates and glutamates), quaternary amine salts, alkali metal salts (such as sodium salts and potassium salts) and alkali earth metal salts (such as magnesium salts and calcium salts).

The therapeutic agent for a disease caused by Aβ according to the present invention can be prepared by a conventional method. Preferable examples of the dosage form include tablets, powders, fine granules, granules, coated tablets, capsules, syrups, troches, inhalants, suppositories, injections, ointments, ophthalmic solutions, ophthalmic ointments, nasal drops, ear drops, cataplasms and lotions. The prophylactic or therapeutic agent can be prepared by using ingredients typically used such as an excipient, a binder, a lubricant, a colorant and a corrective, and ingredients used where necessary such as a stabilizer, an emulsifier, an absorbefacient, a surfactant, a pH adjuster, a preservative and an antioxidant, and can be prepared by blending ingredients generally used as materials for a pharmaceutical preparation. Examples of such ingredients include animal and vegetable oils such as soybean oil, beef tallow and synthetic glyceride; hydrocarbons such as liquid paraffin, squalane and solid paraffin; ester oils such as octyldodecyl myristate and isopropyl myristate; higher alcohols such as cetostearyl alcohol and behenyl alcohol; a silicone resin; silicone oil; surfactants such as polyoxyethylene fatty acid ester, sorbitan fatty acid ester, glycerin fatty acid ester, polyoxyethylene sorbitan fatty acid ester, polyoxyethylene hydrogenated castor oil and a polyoxyethylene-polyoxypropylene block copolymer; water-soluble polymers such as hydroxyethylcellulose, polyacrytic acid, a carboxyvinyl polymer, polyethylene glycol, polyvinylpyrrolidone and methylcellulose; lower alcohols such as ethanol and isopropanol; polyhydric alcohols such as glycerin, propylene glycol, dipropylene glycol and sorbitol; sugars such as glucose and sucrose; inorganic powders such as silicic anhydride, magnesium aluminum silicate and aluminum silicate; and purified water. Examples of the excipient used include lactose, corn starch, saccharose, glucose, mannitol, sorbitol, crystalline cellulose and silicon dioxide. Examples of the binder used include polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, a polypropylene glycolpolyoxyethylene block copolymer and meglumine. Examples of the disintegrator used include starch, agar, gelatin powder, crystalline cellulose, calcium carbonate, sodium bicarbonate, calcium citrate, dextrin, pectin and carboxymethylcellulose calcium. Examples of the lubricant used include magnesium stearate, talc, polyethylene glycol, silica and hydrogenated vegetable oil. Examples of the colorant used include those permitted to be added to pharmaceuticals. Examples of the corrective used include cocoa powder, menthol, empasm, mentha oil, borneol and cinnamon powder.

For example, an oral preparation is prepared by adding an active ingredient compound or a salt thereof or a hydrate of the compound or salt, an excipient, and, where necessary, a binder, a disintegrant, a lubricant, a colorant and a corrective, for example, and then forming the mixture into powder, fine granules, granules, tablets, coated tablets or capsules, for example, by a conventional method. It is obvious that tablets or granules may be appropriately coated, for example, sugar coated, where necessary. A syrup or an injection preparation is prepared by adding a pH adjuster, a solubilizer and an isotonizing agent, for example, and a solubilizing agent, a stabilizer and the like where necessary by a conventional method. An external preparation may be prepared by any conventional method without specific limitations. As a base material, any of various materials usually used for a pharmaceutical, a quasi drug, a cosmetic or the like may be used. Examples of the base material include materials such as animal and vegetable oils, mineral oils, ester oils, waxes, higher alcohols, fatty acids, silicone oils, surfactants, phospholipids, alcohols, polyhydric alcohols, water-soluble polymers, clay minerals and purified water. A pH adjuster, an antioxidant, a chelator, a preservative and fungicide, a colorant, a flavor or the like may be added where necessary. Further, an ingredient having a differentiation inducing effect such as a blood flow enhancer, a bactericide, an antiphlogistic, a cell activator, vitamin, amino acid, a humectant or a keratolytic agent may be blended where necessary. The dose of the therapeutic or prophylactic agent of the present invention varies according to the degree of symptoms, age, sex, body weight, mode of administration, type of salt and specific type of disease, for example. Typically, the therapeutic or prophylactic agent is orally administered to an adult at about 30 μg to 10 g, preferably 100 μg to 5 g, and more preferably 100 μg to 100 mg per day, or is administered to an adult by injection at about 30 μg to 1 g, preferably 100 μg to 500 mg, and more preferably 100 μg to 30 mg per day, in one or several doses, respectively.

BEST MODE FOR CARRYING OUT THE INVENTION

The present invention will now be described in detail with reference to examples; however, the examples are provided only for illustration purposes. The prophylactic or therapeutic agent for a disease caused by Aβ according to the present invention is not limited to the following specific examples in any cases. A person skilled in the art can fully

implement the present invention by making various modifications to not only the following reference examples and examples but also the claims of the present specification, and such modifications are within the scope of the claims of the present specification.

The following abbreviations are used in the following examples.

DMF: Dimethylformamide
THF: Tetrahydrofuran
IPEA: Diisopropylethylamine
DPPA: Diphenyl phosphorazidate

Example 1

Synthesis of 1-benzo[1,3]dioxol-5-yl-3-[3-fluoro-4-(1H-imidazol-1-yl)phenyl]urea

[Formula 18]

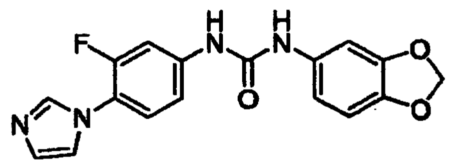

Synthesis of 1-(2-fluoro-4-nitrophenyl)-1H-imidazole

Imidazole (613 mg) and potassium carbonate (1.90 g) were added to a solution of 3,4-difluoronitrobenzene (1.00 mL) in DMF (15 mL), and the reaction solution was stirred at 80°C for five hours. Water and ethyl acetate were added to the reaction solution, and the organic layer was separated. The resulting organic layer was washed with brine, dried over anhydrous magnesium sulfate and then concentrated under reduced pressure to obtain 1.80 g of a crude imidazole compound. The property values of the compound are as follows.
$^1$H-NMR ($CDCl_3$) δ (ppm): 7.29 (s, 1H), 7.36-7.38 (m, 1H), 7.61-7.66 (m, 1H), 8.01 (d, J = 9.6 Hz, 2H), 8.21-8.24 (m, 1H).

Synthesis of 3-fluoro-4-(1H-imidazol-1-yl)phenylamine

Sodium borohydride (255 mg) was slowly added to a solution of 1-(2-fluoro-4-nitrophenyl)-1H-imidazole (698 mg) and nickel chloride hexahydrate (40.0 mg) in dichloromethane-methanol (1:1, 10 mL) under ice-cooling, and the reaction solution was stirred for 2.5 hours. The reaction solution was concentrated under reduced pressure. The resulting residue was purified by silica gel column chromatography (carrier: Chromatorex NH; elution solvent: heptane-ethyl acetate system -> ethyl acetate) to obtain 377 mg of the title compound. The property values of the compound are as follows.
$^1$H-NMR ($CDCl_3$) δ (ppm): 3.95 (brs, 2H), 6.48-6.54 (m, 2H), 7.10-7.17 (m, 3H), 7.68 (s, 1H).

Synthesis of 1-benzo[1,3]dioxol-5-yl-3-[3-fluoro-4-(1H-imidazol-1-yl)phenyl]urea

3,4-Methylenedioxyphenyl isocyanate (57.0 mg) was added to a solution of 3-fluoro-4-(1H-4-imidazol-1-yl) phenylamine (62.0 mg) in toluene (7.0 mL), and the reaction solution was heated under reflux for four hours. The reaction solution was left to cool to room temperature and then the precipitated solid was collected by filtration to obtain 107 mg of the title compound. The property values of the compound are as follows.
$^1$H-NMR (DMSO-d6) δ (ppm): 5.98 (s, 2H), 6.79 (dd, J = 2.0, 8.4 Hz, 1H), 6.85 (d, J = 8.4 Hz, 1H), 7.10-7.11 (m, 1H), 7.19-7.20 (m, 1H), 7.25 (dd, J = 2.0, 8.8 Hz, 1H), 7.49 (t, J = 1.2 Hz, 1H), 7.52 (d, J = 8.8 Hz, 1H), 7.70 (dd, J = 2.0, 14 Hz, 1H), 7.95 (d, J = 1.2 Hz, 1H), 8.73 (s, 1H), 9.05 (s, 1H).

Example 2

Synthesis of 1-benzyl-3-13-fluoro-4-(1H-imidazol-1-yl)phenyl] urea

[Formula 19]

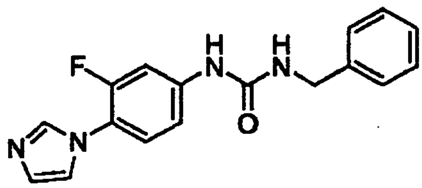

93.0 mg of the title compound was obtained from 3-fluoro-4-(1H-imidazol-1-yl)phenylamine (62.0 mg) and benzyl isocyanate (48.0 μL) by the same method as in Example 1. The property values of the compound are as follows. $^1$H-NMR ($CDCl_3$) δ (ppm): 4.40 (d, J = 4.8 Hz, 2H), 6.08 (brs, 1H), 7.09-7.10 (m, 1H), 7.17-7.18 (m, 1H), 7.20-7.31 (m, 7H), 7.53 (dd, J = 2.0, 14 Hz, 1H), 7.80 (s, 1H), 8.30 (brs, 1H).

Example 3

Synthesis of 1-(9H-fluoren-9-yl)-3-[4-(1H-imidazol-1-yl)-3-methoxyphenyl]urea

[Formula 20]

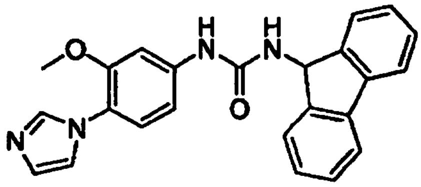

Synthesis of 4-(1H-imidazol-1-yl)-3-methoxybenzaldehyde

1H-imidazole (42.0 mg) and potassium carbonate (180 mg) were added to a solution of 4-fluoro-3-methoxybenzaldehyde (100 mg) in DMF (2.0 mL), and the reaction solution was stirred at 80°C overnight. Water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 36.2 mg of the title compound. The property values of the compound are as follows.
$^1$H-NMR ($CDCl_3$) δ (ppm): 3.98 (s, 3H), 7.21 (s, 1H), 7.30 (s, 1H), 7.48 (d, J = 7.6 Hz, 1H), 7.56-7.60 (m, 2H), 7.92 (s, 1H), 10.0 (s, 1H).

Synthesis of 4-(1H-imidazol-1-yl)-3-methoxybenzoic acid

2-Methyl-2-butene (5.0 mL), sodium dihydrogenphosphate dihydrate (1.55 g) and sodium chlorite (3.14 g) were added to a solution containing 4-(1H-imidazol-1-yl)-3-methoxybenzaldehyde (2 g) in water (10 mL) and tert-butanol (30

mL), and the reaction solution was stirred for two hours. The generated solid was collected by filtration to obtain the title compound (745 mg). The property values of the compound are as follows.
$^1$H-NMR (DMSO-d6) δ (ppm): 3.89 (s, 3H), 7.09 (s, 1H), 7.54 (m, 2H), 7.63 (dd, J = 8.0, 1.6 Hz, 1H), 7.69 (d, J = 1.6 Hz, 1H), 8.05 (s, 1H).

Synthesis of 1-(9H-fluoren-9-yl)-3-[4-(1H-imidazol-1-yl)-3-methoxyphenyl]urea

Triethylamine (34 µL) and diphenylphosphoryl azide (50 µL) were added to a suspension containing 4-(1H-imidazol-1-yl)-3-methoxybenzoic acid (51 mg) in THF (2 mL), and the reaction solution was heated under reflux until it was converted into a solution. The reaction solution was further heated under reflux for 30 minutes, and 9-aminofluorene hydrochloride (51 mg) was added to the reaction solution with heating under reflux. The reaction solution was further heated under reflux for 20 hours. The reaction solution was returned to room temperature. Water and ethyl acetate were added and the organic layer was separated. The organic layer was dried over magnesium sulfate, and the solvent was evaporated under reduced pressure. The residue was purified by LC-MS and then washed with a saturated sodium bicarbonate solution to obtain the title compound (30 mg). The property values of the compound are as follows.
$^1$H-NMR ($CD_3OD$) δ (ppm): 3.88 (s, 3H), 6.00 (s, 1H), 6.99 (dd, J = 8.4, 2.0 Hz, 1H), 7.07 (s, 1H), 7.43-7.25 (m, 6H), 7.64-7.62 (m, 3H), 7.77 (m, 2H), 7.82 (s, 1H). ESI-MS; m/z 397 [$M^+$ + H].

Example 4

Synthesis of 1-benzo[1,3]dioxol-5-yl-3-[4-(1H-imidazol-1-yl)-3-methoxyphenyl]urea

[Formula 21]

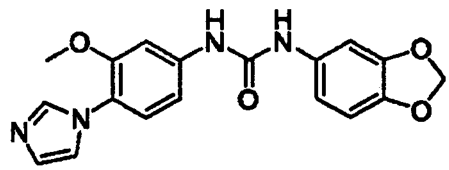

The title compound (20.0 mg) was obtained from 4-(1H-imidazol-1-yl)-3-methoxybenzoic acid (51. mg) and 3,4-methylenedioxyaniline (28.0 mg) in the same manner as in Example 3. The property values of the compound are as follows.
$^1$H-NMR ($CD_3OD$) δ (ppm): 3.86 (s, 3H), 5.92 (s, 2H), 6.75 (m, 2H), 6.99-6.95 (m, 1H), 7.06 (dd, J = 1.2, 1.2 Hz, 1H), 7.12 (dd, J = 1.6, 1.2 Hz, 1H), 7.28-7.25 (m, 2H), 7.55 (d, J = 2.4 Hz, 1H), 7.81 (dd, J = 0.8, 0.8 Hz, 1H).
ESI-MS; m/z 353 [$M^+$ + H].

Example 5

Synthesis of 1-(1,2-diphenylethyl)-3-[4-(1H-imidazol-1-yl)-3-methoxyphenyl]urea

[Formula 22]

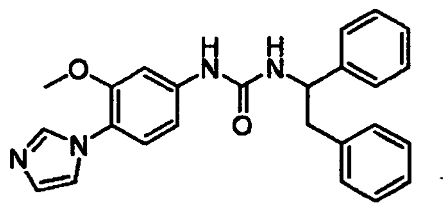

The title compound (13.0 mg) was obtained from 4-(1H-imidazol-1-yl)-3-methoxybenzoic acid (38.0 mg) and 1,2-diphenylethylamine (40.0 μL) in the same manner as in Example 3. The property values of the compound are as follows.
$^1H$-NMR ($CD_3OD$) δ (ppm): 3.08 (m, 2H), 3.78 (s, 3H), 5.08 (m, 1H), 6.80 (m, 1H), 7.03 (dd, J = 1.2, 1.2 Hz, 1H), 7.25-7.13 (m, 8H), 7.31-7.28 (m, 4H), 7.42 (d, J = 2.0 Hz, 1H), 7.77 (dd, J = 0.8, 0.8 Hz, 1H).

Example 6

Synthesis of 1-(9H-fluoren-9-ylmethyl)-3-[4-(1H-imidazol-1-yl)-3-methoxyphenyl]urea

[Formula 23]

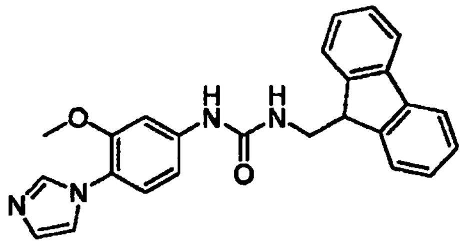

Synthesis of tert-butyl [4-(1H-imidazol-1-yl)-3-methoxyphenyl]carbamate

Triethylamine (0.31 mL) and diphenylphosphoryl azide (0.66 mL) were added to a solution containing 4-(1H-imidazol-1-yl)-3-methoxybenzoic acid (633 mg) in toluene (15 mL) and tert-butyl alcohol (15 mL), and the reaction solution was heated under reflux for 20 hours. The reaction solution was returned to room temperature. A saturated sodium bicarbonate solution and ethyl acetate were added to the reaction solution, and the organic layer was separated. The organic layer was dried over magnesium sulfate, and the solvent was evaporated under reduced pressure. The residue was purified by silica gel column chromatography (elution solvent: heptane-ethyl acetate system) to obtain the title compound (136 mg). The property values of the compound are as follows.
$^1H$-NMR ($CDCl_3$) δ (ppm): 1.54 (s, 9H), 3.85 (s, 3H), 6.60 (s, 1H), 6.77 (m, 1H), 7.17-7.14 (m, 3H), 7.44 (s, 1H), 7.74 (s, 1H).

Synthesis of 4-(1H-imidazol-1-yl)-3-methoxy-phenylamine

tert-Butyl [4-(1H-imidazol-1-yl)-3-methoxyphenyl]carbamate (135 mg) was dissolved in dichloromethane (5.0 mL). Trifluoroacetic acid (5.0 mL) was added to the reaction solution, and the reaction solution was stirred for four hours. The solvent was evaporated under reduced pressure. A 1 N sodium hydroxide solution and dichloromethane were added to the residue, and the organic layer was separated. The organic layer was dried over magnesium sulfate, and the solvent was evaporated under reduced pressure. The residue was purified by silica gel column chromatography (carrier: Chromatorex NH; elution solvent: hexane-ethyl acetate system) to obtain the title compound (82.0 mg). The property values of the compound are as follows.
$^1H$-NMR ($CD_3OD$) δ (ppm): 3.76 (s, 3H), 6.32 (dd, J = 8.0, 2.0 Hz, 1H), 6.49 (d, J = 2.0 Hz, 1H), 7.02-7.00 (m, 2H), 7.16 (dd, J = 1.2, 1.2 Hz, 1H), 7.68 (dd, J = 1.2, 1.2 Hz, 1H).

Synthesis of 1-(9H-fluoren-9-ylmethyl)-3-[4-(1H-imidazol-1-yl)-3-methoxyphenyl]urea

The title compound (10.0 mg) was obtained from 9-fluoreneacetic acid (40.0 mg) and 4-(1H-imidazol-1-yl)-3-methoxy-phenylamine (30.0 mg) in the same manner as in Example 3. The property values of the compound are as follows.
$^1H$-NMR ($CD_3OD$) δ (ppm): 3.77 (m, 2H), 3.79 (s, 3H), 4.17 (t, J = 5.6 Hz, 1H), 6.08 (m, 1H), 7.04 (d, J = 0.8 Hz, 1H), 7.16 (dd, J = 8.0, 1.6 Hz, 1H), 7.23 (d, J = 1.2 Hz, 1H), 7.42-7.30 (m, 5H), 7.65-7.59 (m, 2H), 7.81-7.76 (m, 3H).

Example 7

Synthesis of 1-benzyl-3-{(E)-2-[3-methoxy-4-(4-methyl-1H-imidazol-1-yl)phenyl]vinyl}urea

[Formula 24]

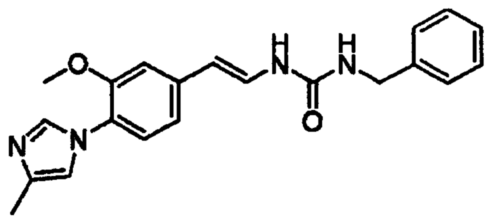

Synthesis of methyl 3-methoxy-4-nitrobenzoate

Methyl iodide (463 g) was added dropwise to a mixture of 3-hydroxy-4-nitrobenzoic acid (199 g) and potassium carbonate (450 g) in DMF (1 L) at room temperature. The reaction solution was stirred at room temperature overnight and then methyl iodide (230 g) was added to the reaction solution. The reaction solution was further stirred at room temperature for six hours. The reaction solution was added to ice water and the precipitated solid was collected by filtration. The resulting solid was dried at 50°C overnight to obtain 178 g of the title compound. The property values corresponded to the reported values (CAS #5081-37-8).

Synthesis of methyl 4-amino-3-methoxybenzoate

10% palladium-carbon (50% wet, 15 g) was added to a solution of methyl 3-methoxy-4-nitrobenzoate (150 g) in methanol (600 mL) and THF (300 mL), and the reaction solution was stirred at a hydrogen pressure of 0.9 MPa at 50°C to 64°C for 6.5 hours. The reaction solution was allowed to cool to room temperature and then filtered through celite. The resulting filtrate was concentrated under reduced pressure to obtain 134 g of the title compound. The property values corresponded to the reported values (CAS #41608-64-4).

Synthesis of methyl 4-formylamino-3-methoxybenzoate

Acetic anhydride (268 mL) was added dropwise to formic acid (401 mL) at room temperature and the reaction solution was stirred at room temperature for 40 minutes. A solution of methyl 4-amino-3-methoxybenzoate (134 g) in THF (600 mL) was added dropwise to the reaction solution at room temperature and the reaction solution was stirred for one hour. To the reaction solution was added 3.8 L of ice water, and the precipitated solid was filtered and further washed with water (2 L). The resulting solid was dried at 50°C overnight to obtain 111 g of the title compound. The property values corresponded to the reported values (CAS #700834-18-0).

Synthesis of methyl 4-[formyl-(2-oxopropyl)amino]-3-methoxybenzoate

Chloroacetone (84.5 mL) was added dropwise to a mixture of methyl 4-formylamino-3-methoxybenzoate (111 g), cesium carbonate (346 g) and potassium iodide (8.78 g) in DMF (497 mL) at room temperature and the reaction solution was stirred for three hours. Cesium carbonate (173 g) and chloroacetone (42 mL) were added to the reaction solution, and the reaction solution was stirred at room temperature for two hours. Ice water and ethyl acetate were added to the reaction solution, and the organic layer was separated. Ethyl acetate was added to the aqueous layer, and the organic layer was separated. The organic layers were combined and washed with water and brine in this order. The resulting organic layer was dried over anhydrous magnesium sulfate and then concentrated under reduced pressure. The residue was diluted with toluene and the solution was concentrated under reduced pressure. tert-Butyl methyl ether and heptane were added to the resulting residue. The precipitated solid was collected by filtration and washed with a solution of 50% tert-butyl methyl ether in heptane. The resulting solid was air-dried overnight to obtain 118 g of the title

compound. $^1$H-NM$_R$ ($CDCl_3$) δ (ppm): 2.19 (s, 3H), 3.91 (s, 3H), 3.94 (s, 3H), 4.49 (s, 2H), 7.31 (d, J = 8.0 Hz, 1H), 7.63 (d, J = 2.0 Hz, 1H), 7.69 (dd, J = 8.0, 2.0 Hz, 1H), 8.33 (s, 1H).

Synthesis of methyl 3-methoxy-4-(4-methyl-1H-imidazol-1-yl)benzoate

A solution of methyl 4-[formyl-(2-oxopropyl)amino]-3-methoxybenzoate (118 g) and ammonium acetate (172 g) in acetic acid (255 mL) was heated and stirred at 140°C for one hour. After completion of the reaction, the reaction solution was neutralized with aqueous ammonia under ice-cooling. Ethyl acetate was added to the reaction solution, and the organic layer was separated. The resulting organic layer was dried over anhydrous magnesium sulfate and then filtered through a silica gel pad. The filtrate was concentrated under reduced pressure. tert-Butyl methyl ether and heptane were added to the residue. The precipitated solid was collected by filtration and washed with a solution of 50% tert-butyl methyl ether in heptane. The resulting solid was air-dried overnight to obtain 68.4 g of the title compound. Further, the crystallization mother liquor was concentrated under reduced pressure. The residue was purified by silica gel column chromatography (elution solvent: heptane-ethyl acetate system) to obtain 22.3 g of the title compound.
$^1$H-NMR ($CDCl_3$,) δ (ppm): 2.30 (s, 3H), 3.94 (s, 3H), 3.96 (s, 3H), 6.98 (brs, 1H), 7.32 (d, J = 8.4 Hz, 1H), 7.71-7.73 (m, 2H), 7.79 (brs, 1H).

Synthesis of 3-methoxy-4-(4-methyl-1H-imidazol-1-yl)benzaldehyde

A solution of pyrrolidine (18 mL) in THF (45 mL) was added dropwise to a solution of sodium bis(2-methoxyethoxy)aluminum hydride (65% solution in toluene, 56 mL) in THF (60 mL) at -5°C or less over 15 minutes. The reaction solution was stirred at room temperature for one hour. Then, a suspension of potassium tert-butoxide (2.10 g) in THF (15 mL) was added dropwise to the reaction solution at room temperature and the reaction solution was stirred for 15 minutes. The above reaction solution was added dropwise to a solution of methyl 3-methoxy-4-(4-methyl-1H-imidazol-1-yl)benzoate (20.0 g) in THF (50 mL) under ice-cooling over 30 minutes. The reaction solution was stirred at room temperature for two hours and then a 5 N sodium hydroxide solution (150 mL) was added dropwise to the reaction solution. Ethyl acetate was added to the reaction solution, and the organic layer was separated. The organic layer was washed with a saturated ammonium chloride solution and brine in this order. The organic layer was dried over anhydrous magnesium sulfate and filtered on a silica gel pad. Then, the filtrate was concentrated under reduced pressure. The residue was diluted with ethyl acetate, and the precipitated solid was collected by filtration. The resulting solid was air-dried overnight to obtain 7.10 g of the title compound. Further, the crystallization mother liquor was concentrated under reduced pressure. The residue was purified by silica gel column chromatography (elution solvent: heptane-ethyl acetate-2-propanol system) to obtain 2.65 g of the title compound.
$^1$H-NMR ($CDCl_3$) δ (ppm): 2.31 (s, 3H), 3.97 (s, 3H), 7.02 (brs, 1H), 7.44 (d, J = 8.0 Hz, 1H), 7.55 (dd, J = 1.6 Hz, 8.0 Hz, 1H), 7.58 (d, J = 1.6 Hz, 1H), 7.84 (brs, 1H), 10.00 (s, 1H).

Synthesis of (E)-3-[3-methoxy-4-(4-methyl-1H-imidazol-1-yl)phenyl]acrylic acid

Ethyl diethylphosphonoacetate (5.70 g) and lithium hydroxide monohydrate (1.30 g) were sequentially added to a mixed solution of 3-methoxy-4-(4-methyl-1H-imidazol-1-yl)benzaldehyde (5.00 g) in THF (20 mL) and ethanol (5.0 mL), and the reaction solution was stirred at room temperature for nine hours. A 2 N sodium hydroxide solution (20 mL) was added to the reaction solution, and the reaction solution was stirred at room temperature for 12 hours. The reaction solution was cooled to 0°C, and 2 N hydrochloric acid (20 mL) was added to the reaction solution. The resulting precipitate was collected by filtration. The resulting precipitate was washed with water and ethyl acetate to obtain 5.10 g of the title compound. The property values of the compound are as follows.
$^1$H-NMR (DMSO-$d_6$) δ (ppm): 7.81 (s, 1H), 7.60 (d, J = 16 Hz, 1H), 7.56 (s, 1H), 7.39 (d, J = 8.0 Hz, 1H), 7.35 (d, J = 8.0 Hz, 1H), 7.16 (s, 1H), 6.66 (d, J = 16 Hz, 1H), 3.88 (s, 3H), 2.15 (s, 3H).

Synthesis of 1-benzyl-3-{(E)-2-[3-methoxy-4-(4-methyl-1H-imidazol-1-yl)phenyl]vinyllurea

DPPA (0.57 mL) was added to a solution of (E)-3-[3-methoxy-4-(4-methyl-1H-imidazol-1-yl)phenyl]acrylic acid (450 mg) and IPEA (0.61 mL) in THF (5.0 mL). The reaction solution was stirred at room temperature for three hours and heated under reflux for four hours. The reaction solution was left to cool to room temperature and then concentrated under reduced pressure. Benzylamine (0.13 mL) was added to a solution of the residue in THF (2.0 mL), and the reaction solution was stirred at room temperature for 17 hours. Ethyl acetate and saturated sodium bicarbonate water were added to the reaction solution, and the organic layer was separated. The resulting organic layer was dried over anhydrous magnesium sulfate and then concentrated under reduced pressure. The residue was purified by silica gel column chromatography (carrier: Chromatorex NH; elution solvent: ethyl acetate -> ethyl acetate:methanol = 10:1) to obtain 14.0

mg of the title compound. The property values of the compound are as follows.
ESI-MS; m/z 363 [$M^+$ + H].$^1$H-NMR ($CDCl_3$) δ (ppm): 2.27 (s, 3H), 3.83 (s, 3H), 4.46 (d, J = 5.6 Hz, 2H), 5.21 (d, J = 5.6 Hz, 1H), 5.83 (d, J = 14.4 Hz, 1H), 6.85 (dd, J = 8.0, 1.6 Hz, 1H), 6.88 (s, 1H), 6.92 (d, J = 1.6 Hz, 1H), 7.11 (d, J = 8.0 Hz, 1H), 7.28-7.38 (m, 6H), 7.51 (dd, J = 14.4, 10.8 Hz, 1H), 7.63 (d, J = 0.8 Hz, 1H).

Example 8

Synthesis of 1-(3,4-dichlorobenzyl)-3-[3-methoxy-4-(4-methyl-1H-imidazol-1-yl)phenyl]urea

[Formula 25]

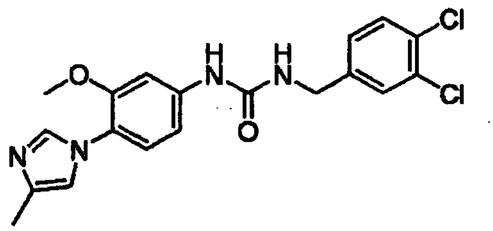

Synthesis of 1-(2-methoxy-4-nitrophenyl)-4-methyl-1H-imidazole

Sodium hydride (743 mg) was added to a solution of 2-bromo-5-nitroanisole (3.00 g) and 4-methyl-1H-imidazole (1.27 g) in DMF (20 mL) under ice-cooling, and the reaction solution was stirred at 70°C for three hours. The reaction solution was left to cool to room temperature. Then, water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 477.0 mg of the title compound. The property values of the compound are as follows.
$^1$H-NMR ($CDCl_3$) δ (ppm): 2.31 (s, 3H), 4.00, (s, 3H), 6.99-7.00 (m, 1H), 7.42 (d, J = 8.4 Hz, 1H), 7.84 (d, J = 1.2 Hz, 1H), 7.93-7.98 (m, 2H).

Synthesis of 3-methoxy-4-(4-methyl-1H-imidazol-1-yl)phenylamine

A solution of 1-(2-methoxy-4-nitrophenyl)-4-methyl-1H-imidazole (477 mg), iron (458 mg) and ammonium chloride (877 mg) in ethanol-water (2:1, 30 mL) was heated under reflux for 1.5 hours. Water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (carrier: Chromatorex NH; elution solvent: heptane-ethyl acetate system -> ethyl acetate-methanol system) to obtain 397 mg of the title compound. The property values of the compound are as follows.
$^1$H-NMR ($CDCl_3$) δ (ppm): 2.29 (s, 3H), 3.77 (s, 3H), 6.28 (dd, J = 2.4, 8.4 Hz, 1H), 6.32 (d, J = 2.4 Hz, 1H), 6.80 (s, 1H), 7.00 (d, J = 8.4 Hz, 1H), 7.57 (s, 1H).

Synthesis of 1-(3,4-dichlorobenzyl)-3-[3-methoxy-4-(4-methyl-1H-imidazol-1-yl)phenyl]urea

46.1 mg of the title compound was obtained from 3-methoxy-4-(4-methyl-1H-imidazol-1-yl)phenylamine (39.0 mg) and 3,4-dichlorobenzyl isocyanate (33.8 μL) by the same method as in Example 1. The property values of the compound are as follows.
$^1$H-NMR ($CDCl_3$) δ (ppm): 2.27 (s, 3H), 3.79 (s, 3H), 4.41 (d, J = 6.0 Hz, 2H), 6.02 (t, J = 6.0 Hz, 1H), 6.65 (dd, J = 2.0, 8.4 Hz, 1H), 6.84 (s, 1H), 7.04 (d, J = 8.0 Hz, 1H), 7.15 (dd, 2.0, 8.4 Hz, 1H), 7.37 (d, J = 8.0 Hz, 1H), 7.40 (d, J = 2.0 Hz, 1H), 7.61 (d, J = 1.2 Hz, 1H), 7.94 (brs, 1H).

Example 9

Synthesis of 1-(3,4-dichlorobenzyl)-3-[3-methoxy-4-(4-methyl-1H-imidazol-1-yl)phenyl]tetrahydropyrimidin-2-one

[Formula 26]

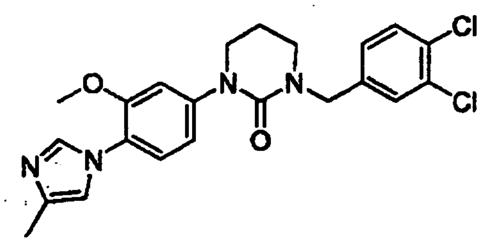

1,3-Dibromopropane (11.2 μL) was added to a solution of 1-(3,4-dichlorobenzyl)-3-[3-methoxy-4-(4-methyl-1H-imidazol-1-yl)phenyl]urea described in Example 8 (37.0 mg) in THF (3.0 mL) under ice-cooling, and the reaction solution was heated under reflux for 1.5 hours. To eliminate the raw material, 1,3-dibromopropane (0.5 mL) was further added and the reaction solution was heated under reflux for one hour. Water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (carrier: Chromatorex NH; elution solvent: heptane-ethyl acetate system -> ethyl acetate) to obtain 5.6 mg of the title compound. The property values of the compound are as follows.
$^1$H-NMR ($CDCl_3$) δ (ppm): 2.12-2.18 (m, 2H), 2.30 (s, 3H), 3.35-3.38 (m, 2H), 3.74-3.78 (m, 2H), 3.85 (s, 3H), 4.57 (s, 2H), 6.86-6.92 (m, 2H), 7.09 (d, J = 1.6 Hz, 1H), 7.18-7.24 (m, 2H), 7.39-7.43 (m, 2H), 7.67 (s, 1H).

Example 10

Synthesis of 1-[(S)-1-(4-fluorophenyl)ethyl]-3-[3-methoxy-4-(4-methyl-1H-imidazol-1-yl)phenyl]urea

[Formula 27]

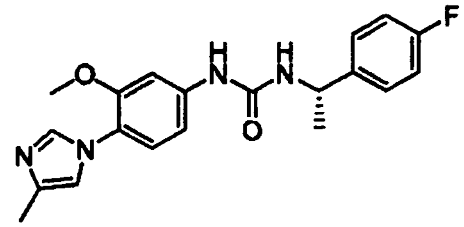

Synthesis of phenyl N-[3-methoxy-4-(4-methyl-1H-imidazol-1-yl)phenyl]-N-(phenoxycarbonyl)carbamate

Triethylamine (602 μL) and phenyl chloroformate (406 μL) were added to a solution of 3-methoxy-4-(4-methyl-1H-imidazol-1-yl)phenylamine described in Example 8 (220 mg) in THF-DMF (100:1, 20.1 mL) under ice-cooling. The reaction solution was stirred at room temperature overnight. Then, water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 86.0

mg of the title compound. The property values of the compound are as follows.
$^1$H-NMR ($CDCl_3$) δ (ppm): 2.30 (s, 3H), 3.91 (s, 3H), 6.94 (s, 1H), 7.12-7.18 (m, 6H), 7.23-7.28 (m, 2H), 7.34-7.41 (m, 5H), 7.73 (s, 1H).

Synthesis of 1-[(S)-1-(4-fluorophenyl)ethyl]-3-[3-methoxy-4-(4-methyl-1H-imidazol-1-yl)phenyl]urea

Triethylamine (0.3 mL) was added to a solution of (S)-1-(4-fluorophenyl)ethylamine (31.5 mg) and phenyl N-[3-methoxy-4-(4-methyl-1H-imidazol-1-yl)phenyl]-N-(phenoxycarbonyl)carbamate (43.0 mg) in DMF (1.0 mL), and the reaction solution was stirred at room temperature for 5.5 hours. Water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 24.9 mg of the title compound. The property values of the compound are as follows.
$^1$H-NMR ($CDCl_3$) δ (ppm): 1.44-1.45 (m, 3H), 2.26 (s, 3H), 3.74 (s, 3H), 4.95-5.02 (m, 1H), 6.07 (brs, 1H), 6.57-6.60 (m, 1H), 6.85 (s, 1H), 6.87-7.04 (m, 3H), 7.26-7.29 (m, 2H), 7.52-7.58 (m, 2H), 8.11 (brs, 1H).

Example 11

Synthesis of 1-[2-(3,4-dichlorophenyl)ethyl]-3-[3-methoxy-4-(4-methyl-1H-imidazol-1-yl)phenyl]urea

[Formula 28]

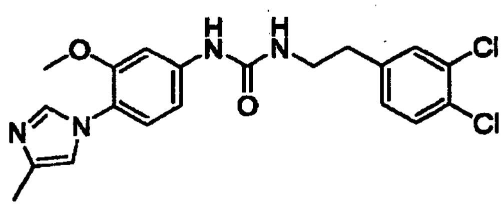

29.8 mg of the title compound was obtained from 3,4-dichlorophenethylamine (36.9 mg) and phenyl N-[3-methoxy-4-(4-methyl-1H-imidazol-1-yl)phenyl]-N-(phenoxycarbonyl)carbamate (43.0 mg) by the same method as in Example 10. The property values of the compound are as follows.
$^1$H-NMR ($CDCl_3$) δ (ppm): 2.23 (s, 3H), 2.79-2.86 (m, 2H), 3.46-3.56 (m, 2H), 3.79 (d, J = 1.2 Hz, 3H), 5.80 (brs, 1H), 6.62-6.64 (m, 1H), 6.86 (s, 1H), 7.02 (d, J = 8.4 Hz, 2H), 7.26-7.34 (m, 2H), 7.53-7.55 (m, 2H), 8.24 (brs, 1H).

Example 12

Synthesis of 1-[(S)-1-(4-fluorophenyl)ethyl]-3-[3-methoxy-4-(4-methyl-1H-imidazol-1-yl)benzyl]urea

[Formula 29]

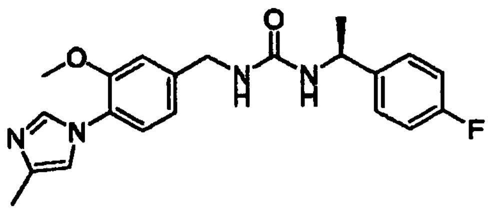

Synthesis of 3-methoxy-4-(4-methyl-1H-imidazol-1-yl)benzylamine

A solution of 3-methoxy-4-(4-methyl-1H-imidazol-1-yl)benzaldehyde described in Example 7 (300 mg) and hydroxylamine monohydrochloride (145 mg) in ethanol (5.0 mL) was heated under reflux for two hours. Water and ethyl acetate were added to the reaction solution, and the organic layer was separated. The resulting organic layer was washed with brine, dried over anhydrous magnesium sulfate and then concentrated under reduced pressure to obtain a crude oxime compound. 10% palladium-carbon (50% wet, 100 mg) was added to a solution of the resulting crude oxime compound in methanol (20 mL), and the reaction solution was stirred at a hydrogen pressure of 0.4 MPa at room temperature for 4.5 hours. The reaction solution was filtered through celite, and the resulting filtrate was concentrated under reduced pressure. The residue was purified by silica gel column chromatography (support: Chromatorex NH, elution solvent: heptane-ethyl acetate system -> ethyl acetate-methanol system) to obtain 110 mg of the title compound. The property values of the compound are as follows.
$^1$H-NMR ($CDCl_3$) $\delta$ (ppm): 2.30 (s, 3H), 3.86 (s, 3H), 3.93 (s, 2H), 6.90 (s, 1H), 6.94-6.98 (m, 1H), 7.04-7.08 (m, 2H), 7.66 (d, J = 1.2 Hz, 1H).

Synthesis of phenyl [(S)-1-(4-fluorophenyl)ethyl]carbamate

A solution of (S)-1-(4-fluorophenyl)ethylamine (100 mg) in THF-DMF (100:1, 10.1 mL) was cooled to 0°C, and triethylamine (401 μL) and phenyl chloroformate (271 μL) were added to the reaction solution. The reaction solution was stirred at room temperature overnight. Then, water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 225 mg of the title compound. The property values of the compound are as follows.
$^1$H-NMR ($CDCl_3$) $\delta$ (ppm): 1.55 (d, J = 7.2 Hz, 3H), 4.90 (qd, J = 7.2, 14 Hz, 1H), 5.31 (brs, 1H), 7.01-7.08 (m, 2H), 7.08-7.14 (m, 2H), 7.15-7.20 (m, 1H), 7.30-7.37 (m, 4H).

Synthesis of 1-[(S)-1-(4-fluorophenyl)ethyl]-3-[3-methoxy-4-(4-methyl-1H-imidazol-1-yl)benzyl]urea

Triethylamine (0.5 mL) was added to a solution of 3-methoxy-4-(4-methyl-1H-imidazol-1-yl)benzylamine (90.0 mg) and phenyl [(S)-1-(4-fluorophenyl)ethyl]carbamate (158 mg) in DMF (2.0 mL), and the reaction solution was stirred at room temperature overnight. Water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 89.0 mg of the title compound. The property values of the compound are as follows.
$^1$H-NMR ($CDCl_3$) $\delta$ (ppm): 1.44 (d, J = 6.8 Hz, 3H), 2.26 (s, 3H), 3.75 (d, J = 3.6 Hz, 3H), 4.28-4.42 (m, 2H), 4.82-4.92 (m, 1H), 5.10-5.36 (m, 2H), 6.81 (d, J = 8.0 Hz, 1H), 6.87 (d, J = 18 Hz, 2H), 6.97-7.02 (m, 2H), 7.05-7.10 (m, 1H), 7.25-7.32 (m, 2H), 7.38-7.44 (m, 1H).

Example 13

Synthesis of 1-[2-(4-fluorophenyl)-1,1-dimethyl-ethyl]-3-[3-methoxy-4-(4-methyl-1H-imidazol-1-yl)phenyl]urea monotrifluoroacetate

[Formula 30]

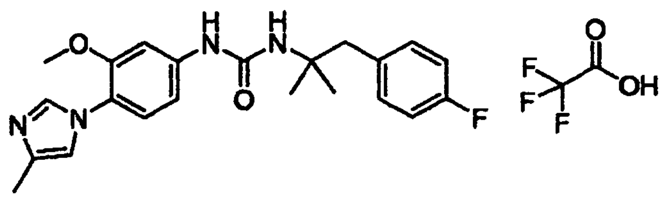

Synthesis of phenyl [3-methoxy-4-(4-methyl-1H-imidazol-1-yl)phenyl]carbamate

Triethylamine (544 μL) and phenyl chloroformate (245 μL) were added to a solution of 3-methoxy-4-(4-methyl-1H-imidazol-1-yl)phenylamine described in Example 8 (397 mg) in THF-DMF (100:1, 20.11 mL) under ice-cooling. The reaction solution was stirred at room temperature overnight. Then, water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 531 mg of the title compound. The property values of the compound are as follows.
$^1$H-NMR ($CDCl_3$) δ (ppm): 2.31 (s, 3H), 3.84 (s, 3H) 6.86-6.88 (m, 2H), 7.16-7.24 (m, 4H), 7.26-7.30 (m, 1H), 7.38-7.44 (m, 2H), 7.58 (brs, 1H), 7.69 (s, 1H).

Synthesis of 1-[2-(4-fluorophenyl)-1,1-dimethyl-ethyl]-3-[3-methoxy-4-(4-methyl-1H-imidazol-1-yl)phenyl]urea monotrifluoroacetate

Triethylamine (0.2 mL) was added to a solution of 2-(4-fluorophenyl)-1,1-dimethyl-ethylamine (7.8 mg) and phenyl [3-methoxy-4-(4-methyl-1H-imidazol-1-yl)phenyl]carbamate (10.0 mg) in THF (0.5 mL), and the reaction solution was stirred at room temperature for five hours. To eliminate the starting material, DMF (0.2 mL) was added to the reaction solution, and the reaction solution was stirred at 50°C for 2.5 hours. The reaction solution was separated by LC-MS to obtain 4.7 mg of the title compound. The property values of the compound are as follows.
$^1$H-NMR ($CDCl_3$) δ (ppm): 1.32 (s, 6H), 2.42 (s, 3H), 3.06 (s, 2H), 3.83 (s, 3H), 6.72-6.78 (m, 1H), 6.86-6.94 (m, 3H), 6.98 (s, 1H), 7.12-7.16 (m, 2H), 7.73 (s, 1H), 8.25 (brs, 1H), 8.38 (brs, 1H).

Example 14

Synthesis) of 1-[3-methoxy-4-(4-methyl-1H-imidazol-1-yl)phenyl]-3-(1,2,3,4-tetrahydronaphthalen-2-ylmethyl)urea monotrifluoroacetate

[Formula 31]

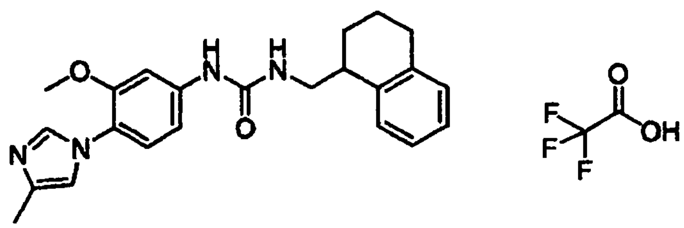

10.8 mg of the title compound was obtained from 1,2,3,4-tetrahydro-1-naphthalene-methaneamine (7.5 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 1.70-1.90 (m, 3H), 2.42 (s, 3H), 2.71-2.80 (m, 3H), 2.98-3.06 (m, 1H), 3.32-3.37 (m, 1H), 3.58-3.62 (m, 1H), 3.79 (s, 3H), 6.74-6.76 (m, 1H), 6.87 (d, J = 8.8 Hz, 1H), 6.98 (s, 1H), 7.04-7.16 (m, 4H), 7.73 (s, 1H), 8.25 (s, 1H), 8.70 (brs, 1H).

Example 15

Synthesis of 1-[3-methoxy-4-(4-methyl-1H-imidazol-1-yl)phenyl]-3-(2-phenyl-indan-2-ylmethyl)urea monotrifluoroacetate

[Formula 32]

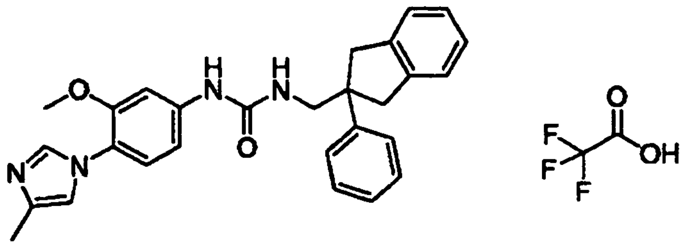

11.3 mg of the title compound was obtained from C-(2-phenyl-indan-2-yl)-methylamine (10.0 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 2.38 (s, 3H), 3.32 (s, 4H), 3.45 (s, 2H), 3.71 (s, 3H), 5.47 (brs, 1H), 6.34 (brd, J = 9.6 Hz, 1H), 6.85 (brd, J = 7.6 Hz, 1H), 6.92 (s, 1H), 7.12-7.18 (m, 2H), 7.20-7.29 (m, 3H), 7.30-7.40 (m, 4H), 7.48 (s, 1H), 8.10 (brs, 1H), 8.17 (s, 1H).

Example 16

Synthesis of 2-[2-(2-fluorophenyl)ethyl]piperidine-1-carboxylic acid [3-methoxy-4-(4-methyl-1H-imidazol-1-yl)phenyl] amide monotrifluoroacetate

[Formula 33]

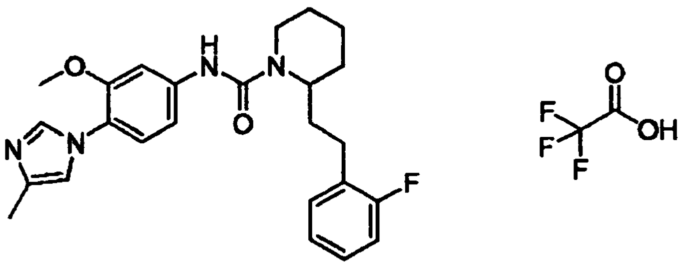

11.1 mg of the title compound was obtained from 2-[2-(2-fluorophenyl)ethyl]piperidine (9.6 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 1.42-1.60 (m, 1H), 1.62-1.80 (m, 5H), 1.88-2.00 (m, 1H), 2.04-2.16 (m, 1H), 2.44 (s, 3H), 2.60-2.76 (m, 2H), 2.98-3.08 (m, 1H), 3.86 (s, 3H), 4.01 (brd, J = 12.8 Hz, 1H), 4.24 (brs, 1H), 6.71 (brd, J = 7.6 Hz, 1H), 6.98 (s, 1H), 7.00-7.12 (m, 4H), 7.16-7.26 (m, 2H), 7.59 (d, J = 2.0 Hz, 1H), 8.49 (brs, 1H).

Example 17

Synthesis of 1-(1,2-diphenyl-ethyl)-[3-methoxy-4-(4-methyl-1H-imidazol-1-yl)phenyl]urea monotrifluoroacetate

[Formula 34]

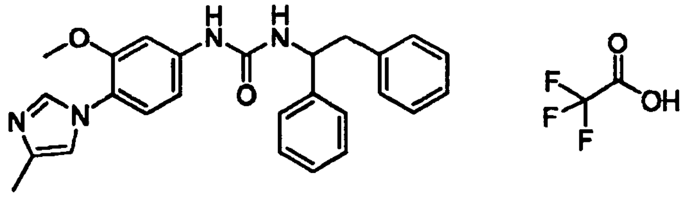

8.90 mg of the title compound was obtained from 1,2-diphenyl-ethylamine (9.2 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 2.33 (s, 3H), 3.03-3.05 (m, 2H), 3.51 (s, 3H), 5.05-5.09 (m, 1H), 6.49-6.53 (m, 1H), 6.63 (d, J = 8.4 Hz, 1H), 6.87 (s, 1H), 7.11-7.24 (m, 8H), 7.24-7.32 (m, 2H), 7.53 (s, 1H), 8.17 (brs, J = 1H), 8.74 (brs, 1H).

Example 18

Synthesis of 1-[3-methoxy-4-(4-methyl-1H-imidazol-1-yl)phenyl]-3-(3-phenylpropyl)urea monotrifluoroacetate

[Formula 35]

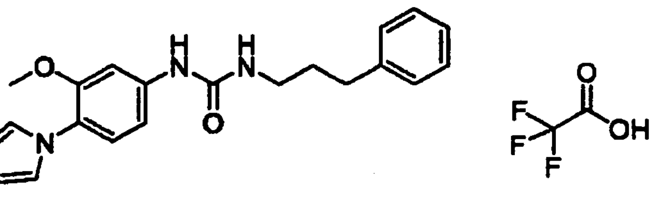

8.9 mg of the title compound was obtained from 3-phenyl-propylamine (6.0 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 1.82-1.90 (m, 2H), 2.40 (s, 3H), 2.65-2.69 (m, 2H), 3.26-3.29 (m, 2H), 3.77 (s, 3H), 6.74 (brd, J = 8.0 Hz, 1H), 6.86 (brd, J = 8.4 Hz, 1H), 6.96 (s, 1H), 7.14-7.20 (m, 3H), 7.22-7.30 (m, 2H), 7.74 (brs, 1H), 8.22 (brs, 1H), 8.71 (brs, 1H).

Example 19

Synthesis of 1-[3-methoxy-4-(4-methyl-1H-imidazol-1-yl)phenyl]-3-(3,4,5-trifluorobenzyl)urea monotrifluoroacetate

[Formula 36]

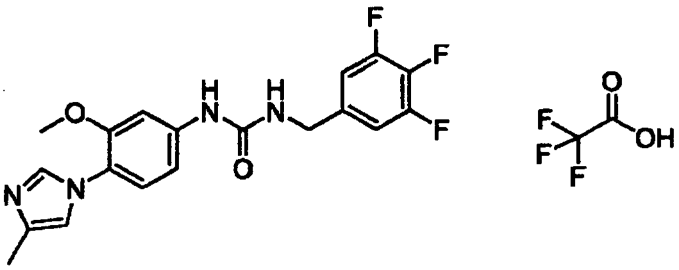

9.0 mg of the title compound was obtained from 3,4,5-trifluorobenzylamine (7.5 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 2.43 (s, 3H), 3.78 (s, 3H), 4.36 (s, 2H), 6.77-6.80 (m, 1H), 6.87-6.89 (m, 1H), 6.93-6.96 (m, 2H), 7.02 (s, 1H), 7.75 (d, J = 2.0 Hz, 1H), 8.19 (s, 1H), 9.04 (s, 1H).

Example 20

Synthesis of 4-phenyl-piperidine-1-carboxylic acid [3-methoxy-4-(4-methyl-1H-imidazol-1-yl)phenyl]amide monotrifluoroacetate

[Formula 37]

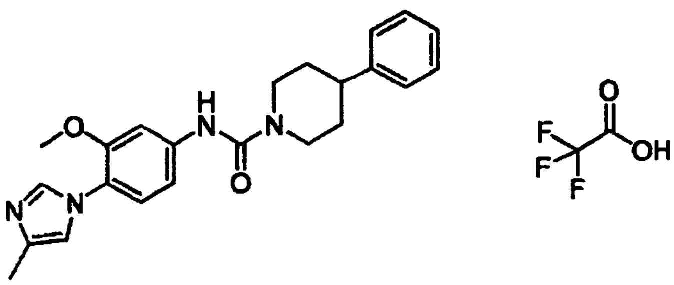

8.0 mg of the title compound was obtained from 4-phenyl-piperidine (7.5 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 1.71-1.80 (m, 2H), 1.96-2.00 (m, 2H), 2.45 (s, 3H), 2.74-2.80 (m, 1H), 3.02-3.09 (m, 2H), 3.88 (s, 3H), 4.27 (brd, J = 12.8 Hz, 2H), 6.90 (brd, J = 7.6 Hz, 1H), 6.99-7.04 (m, 2H), 7.14-7.16 (m, 1H), 7.20-7.28 (m, 3H), 7.30-7.36 (m, 2H), 7.67 (brs, 1H), 8.47 (brs, 1H).

Example 21

Synthesis of 3-phenyl-piperidine-1-carboxylic acid [3-methoxy-4-(4-methyl-1H-imidazol-1-yl)phenyl]amide monotrifluoroacetate

[Formula 38]

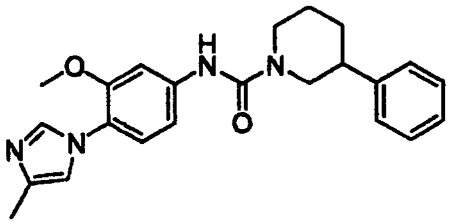 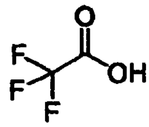

8.5 mg of the title compound was obtained from 3-phenyl-piperidine (7.5 mg) and phenyl [3-methoxy-4-(4-methyl-1H-imidazol-1-yl)phenyl]carbamate (10.0 mg) by the same method as in Example 13. The property values of the compound are as follows.

$^1$H-NMR ($CDCl_3$) δ (ppm): 1.68-1.80 (m, 1H), 1.86-1.96 (m, 1H), 2.06-2.16 (m, 1H), 2.44 (s, 3H), 2.76-2.86 (m, 1H), 2.74-3.03 (m, 2H), 3.86 (s, 3H), 4.18 (brd, J = 13.6 Hz, 2H), 6.87 (brd, J = 8.4 Hz, 1H), 6.93-7.02 (m, 2H), 7.14 (d, J = 8.4 Hz, 1H), 7.24-7.30 (m, 3H), 7.32-7.38 (m, 2H), 7.60 (d, J = 2.0 Hz, 1H), 8.50 (brs, 1H).

Example 22

Synthesis of 1-((S)-1-hydroxymethyl-2-phenylethyl)-3-[3-methoxy-4-(4-methyl-1H-imidazol-1-yl)phenyl]urea

[Formula 39]

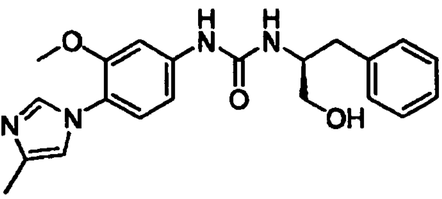

Triethylamine (0.5 mL) was added to a solution of L-phenylalaninol (14.0 mg) and phenyl [3-methoxy-4-(4-methyl-1H-imidazol-1-yl)phenyl]carbamate (20.0 mg) in DMF (2.0 mL), and the reaction solution was stirred at room temperature for 40 minutes. Water and ethyl acetate were added to the reaction solution, and the organic layer was separated. The resulting organic layer was washed with brine, dried over anhydrous magnesium sulfate and then concentrated under reduced pressure. The resulting residue was purified by silica gel column chromatography (elution solvent: heptane-ethyl acetate system -> ethyl acetate-methanol system) to obtain 11.0 mg of the title compound. The property values of the compound are as follows.

$^1$H-NMR ($CDCl_3$) δ (ppm): 2.19 (brs, 1H), 2.23-2.27 (m, 3H), 2.87-2.88 (m, 2H), 3.59 (dd, J = 5.6, 13.2 Hz, 1H), 3.68 (s, 3H), 3.71-3.78 (m, 1H), 4.08-4.16 (m, 1H), 5.91 (brd, J = 7.6 Hz, 1H), 6.61 (dd, J = 2.0, 8.4 Hz, 1H), 6.79 (brs, 1H), 6.90 (d, J = 8.4 Hz, 1H), 7.14-7.28 (m, 5H), 7.36 (d, J = 2.0 Hz, 1H), 7.51 (brs, 1H), 8.17 (s, 1H).

Example 23

Synthesis of 1-((R)-1-hydroxymethyl-2-phenylethyl)-3-[3-methoxy-4-(4-methyl-1H-imidazol-1-yl)phenyl]urea

[Formula 40]

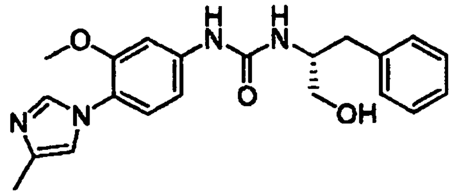

13.0 mg of the title compound was obtained from D-phenylalaninol (14.0 mg) and phenyl [3-methoxy-4-(4-methyl-1H-imidazol-1-yl)phenyl]carbamate (20.0 mg) by the same method as in Example 22. The property values of the compound are as follows.

$^1$H-NMR ($CDCl_3$) δ (ppm): 2.19 (brs, 1H), 2.23-2.27 (m, 3H), 2.87-2.88 (m, 2H), 3.59 (dd, J = 5.6, 13.2 Hz, 1H), 3.68 (s, 3H), 3.71-3.78 (m, 1H), 4.08-4.16 (m, 1H), 5.91 (brd, J = 7.6 Hz, 1H), 6.61 (dd, J = 2.0, 8.4 Hz, 1H), 6.79 (brs, 1H), 6.90 (d, J = 8.4 Hz, 1H), 7.14-7.28 (m, 5H), 7.36 (d, J = 2.0 Hz, 1H), 7.51 (brs, 1H), 8.17 (s, 1H).

Example 24

Synthesis of 1-(3,3-diphenylpropyl)-3-[3-methoxy-4-(4-methyl-1H-imidazol-1-yl)phenyl]urea monotrifluoroacetate

[Formula 41]

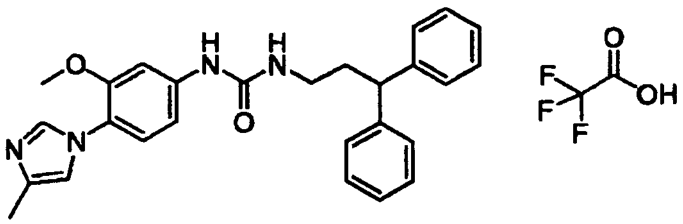

8.1 mg of the title compound was obtained from 3,3-diphenyl-propylamine (9.8 mg) and phenyl [3-methoxy-4-(4-methyl-1H-imidazol-1-yl)phenyl]carbamate (10.0 mg) by the same method as in Example 13. The property values of the compound are as follows.

$^1$H-NMR ($CDCl_3$) δ (ppm): 2.27-2.33 (m, 2H), 2.39 (s, 3H), 3.17-3.20 (m, 2H), 3.75 (s, 3H), 3.98-4.03 (m, 1H), 6.68 (brd, J = 7.2 Hz, 1H), 6.81 (brd, J = 6.4, 1H), 6.93 (s, 1H), 7.14-7.17 (m, 2H), 7.20-7.30 (m, 8H), 7.72 (s, 1H), 8.17 (brs, 1H), 8.66 (brs, 1H).

Example 25

Synthesis of 1-(2,2-diphenylethyl)-3-[3-methoxy-4-(4-methyl-1H-imidazol-1-yl)phenyl]urea monotrifluoroacetate

[Formula 42]

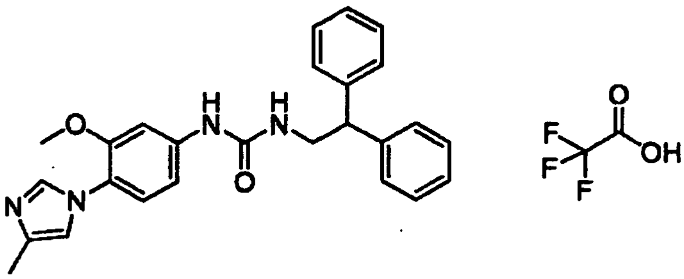

7.2 mg of the title compound was obtained from 2,2-diphenyl-ethylamine (9.2 mg) and phenyl [3-methoxy-4-(4-methyl-1H-imidazol-1-yl)-phenyl]carbamate (10.0 mg) by the same method as in Example 13. The property values of the compound are as follows.
$^1$H-NMR ($CDCl_3$) δ (ppm): 2.36 (s, 3H), 3.74 (s, 3H), 3.88 (d, J = 7.6 Hz, 2H), 4.25 (t, J = 7.6 Hz, 1H), 6.61 (brd, J = 7.6 Hz, 1H), 6.79 (brd, J = 7.6 Hz, 1H), 6.92 (s, 1H), 7.17-7.24 (m, 2H), 7.24-7.32 (m, 8H), 7.64 (s, 1H), 8.18 (brs, 1H), 8.35 (brs, 1H).

Example 26

Synthesis of 1-[3-(4-cyclohexyl-phenyl)propyl]-3-[3-methoxy-4-(4-methyl-1H-imidazol-1-yl)phenyl]urea monotrifluoroacetate

[Formula 43]

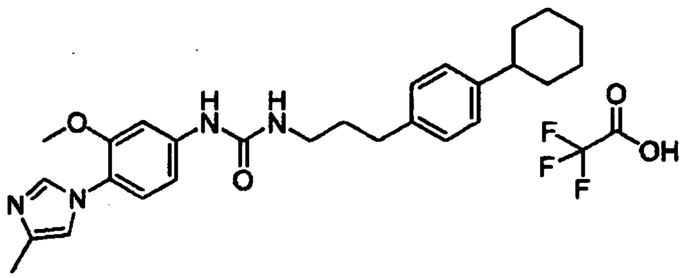

5.0 mg of the title compound was obtained from 2-(4-cyclohexyl-phenyl)propylamine monotrifluoroacetate (15.0 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 1.20-1.45 (m, 4H), 1.70-1.90 (m, 6H), 2.41 (s, 3H), 2.41-2.50 (m, 1H), 2.62-2.66 (m, 2H), 3.26-3.29 (m, 2H), 3.78 (s, 3H), 6.70 (brd, J = 7.6 Hz, 1H), 6.84 (brd, J = 7.6 Hz, 1H), 6.95 (s, 1H), 7.08-7.15 (m, 4H), 7.77 (s, 1H), 8.21 (brs, 1H), 8.60 (brs, 1H).

Example 27

Synthesis of 1-[3-methoxy-4-(4-methyl-1H-imidazol-1-yl)phenyl]-3-(2-methyl-2-phenylpropyl)urea monotrifluoroacetate

[Formula 44]

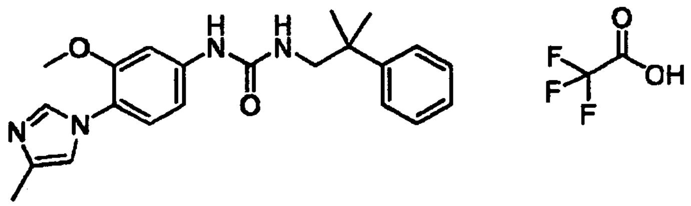

5.2 mg of the title compound was obtained from 2-methyl-2-phenyl-propylamine (6.9 mg) and phenyl [3-methoxy-4-(4-methyl-1H-imidazol-1-yl)phenyl]carbamate (10.0 mg) by the same method as in Example 13. The property values of the compound are as follows.
$^1$H-NMR ($CDCl_3$) δ (ppm): 1.37 (s, 6H), 2.39 (s, 3H), 3.46 (s, 2H), 3.76 (s, 3H), 5.59 (brs, 3H), 6.66 (brd, J = 7.6 Hz, 1H), 6.84 (brd, J = 7.6 Hz, 1H), 6.93 (s, 1H), 7.18-7.23 (m, 1H), 7.30-7.35 (m, 2H), 7.38-7.42 (m, 2H), 7.67 (s, 1H), 8.18 (brs, 1H), 8.37 (brs, 1H).

Example 28

Synthesis of 1-(S)-2-hydroxy-2-phenylethyl-3-[3-methoxy-4-(4-methyl-1H-imidazol-1-yl)phenyl]urea

[Formula 45]

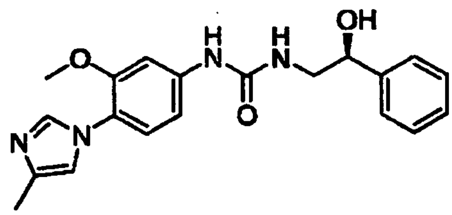

3.2 mg of the title compound was obtained from (S)-2-amino-1-phenyl-ethanol (6.4 mg) and phenyl [3-methoxy-4-(4-methyl-1H-imidazol-1-yl)phenyl]carbamate (10.0 mg) by the same method as in Example 22. The property values of the compound are as follows.
$^1$H-NMR ($CDCl_3$) δ (ppm): 2.30 (s, 3H), 3.34-3.42 (m, 1H), 3.60-3.68 (m, 1H), 3.77 (s, 3H), 4.89 (dd, J = 2.8, 8.8 Hz, 1H), 6.01 (brs, 1H), 6.80 (dd, J = 2.0, 8.4 Hz, 1H), 6.88 (s, 1H), 7.02 (d, J = 8.4 Hz, 1H), 7.30-7.42 (m, 5H), 7.48 (d, J = 2.0 Hz, 1H), 7.85 (brs, 1H), 8.03 (brs, 1H).

Example 29

Synthesis of 1-(R)-2-hydroxy-2-phenylethyl-3-[3-methoxy-4-(4-methyl-1H-imidazol-1-yl)phenyl]urea

[Formula 46]

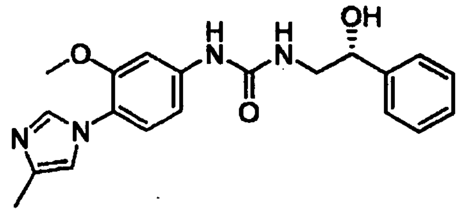

1.8 mg of the title compound was obtained from (R)-2-amino-1-phenyl-ethanol (6.4 mg) and phenyl [3-methoxy-4-(4-methyl-1H-imidazol-1-yl)phenyl]carbamate (10.0 mg) by the same method as in Example 22. The property values of the compound are as follows.
$^1$H-NMR ($CDCl_3$) δ (ppm): 2.30 (s, 3H), 3.34-3.42 (m, 1H), 3.60-3.68 (m, 1H), 3.77 (s, 3H), 4.89 (dd, J = 2.8, 8.8 Hz, 1H), 6.01 (brs, 1H), 6.80 (dd, J = 2.0, 8.4 Hz, 1H), 6.88 (s, 1H), 7.02 (d, J = 8.4 Hz, 1H), 7.30-7.42 (m, 5H), 7.48 (d, J = 2.0 Hz, 1H), 7.85 (brs, 1H), 8.03 (brs, 1H).

INDUSTRIAL APPLICABILITY

The compound of the general formula (I) or pharmaceutically acceptable salt thereof according to the present invention have an Aβ42 production reducing effect. Thus, the present invention can particularly provide a prophylactic or therapeutic agent for a neurodegenerative disease caused by Aβ such as Alzheimer's disease or Down's syndrome.

**Claims**

**1.** A compound represented by the formula (I):

[Formula 1]

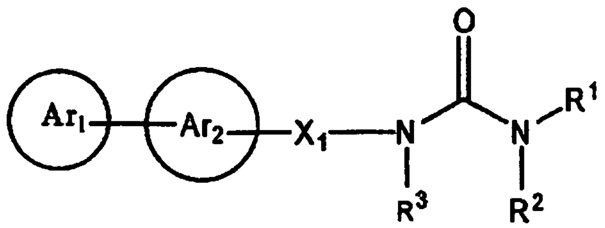

(I)

or a pharmacologically acceptable salt thereof,
wherein $Ar_1$ represents an imidazolyl group or a triazolyl group which may be substituted with 1 to 3 substituents selected from Substituent Group A1 shown below;
$Ar_2$ represents a pyridinyl group or a phenyl group which may be substituted with 1 to 3 substituents selected from Substituent Group A2 shown below;
$X_1$ represents a single bond, a C1-6 alkylene group which may be substituted with 1 to 3 substituents selected from Substituent Group A3 shown below or a C2-6 alkenylene group which may be substituted with 1 to 3 substituents selected from Substituent Group A3 shown below;

(1) $R^1$ and $R^2$ are the same or different and each represent a group selected from Substituent Group A4 shown below; or
(2) $R^1$ and $R^2$, together with a nitrogen atom to which they are bonded, form a 5- to 11-membered heterocyclic group which may be substituted with 1 to 4 substituents selected from Substituent Group A4 and is represented

by the formula (II):

[Formula 2]

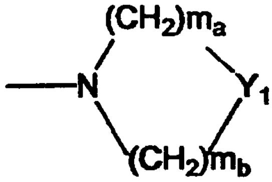

(II)

wherein $Y_1$ represents (1) -NH-, (2) -O-, (3) -S-, (4) -SO-, (5) $-SO_2-$ (6) $-CH_2-$, (7) -CO-, (8) -CONH-, (9) -NHCO-, (10) $-CR^5=CR^6-$ (wherein $R^5$ and $R^6$ are the same or different and each represent a substituent selected from Substituent Group A4 shown below), (11) a single bond or (12) $>C=CR^{13}R^{14}$ (wherein $R^{13}$ and $R^{14}$ are the same or different and each represent a substituent selected from Substituent Group A4 shown below); and $m_a$ and $m_b$ each represent an integer of 0 to 4; and $R^3$ represents a group selected from Substituent Group A4 shown below, or
represents, together with $-N-CO-N-R^2$, a 5- to 8-membered ring group.
Substituent Group Al: (1) a hydrogen atom, (2) a halogen atom and (3) a C1-6 alkyl group (wherein the C1-6 alkyl group may be substituted with 1 to 3 substituents selected from the group consisting of a halogen atom, a hydroxyl group, a cyano group, a C1-6 alkoxy group, a C3-8 cycloalkyl group and a C1-6 alkylcarbonyl group).
Substituent Group A2: (1) a hydrogen atom, (2) a halogen atom, (3) a cyano group and (4) a C1-6 alkoxy group (wherein the C1-6 alkoxy group may be substituted with 1 to 3 substituents selected from the group consisting of a halogen atom, a cyano group, a C1-6 alkoxy group, a C2-6 alkenyl group, a C2-6 alkynyl group and a C3-8 cycloalkyl group).
Substituent Group A3: (1) a hydrogen atom and (2) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4)).
Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from

Substituent Group A4), (31) -CO-A (wherein A is as defined above) and (32) =CH-A (wherein A is as defined above).

**2.** The compound or pharmacologically acceptable salt thereof according to claim 1, wherein $Ar_1$ is an imidazolyl group or a triazolyl group which may be substituted with 1 or 2 substituents selected from the group consisting of (1) a hydrogen atom and (2) a C1-6 alkyl group (wherein the C1-6 alkyl group may be substituted with 1 to 3 halogen atoms).

**3.** The compound or pharmacologically acceptable salt thereof according to claim 2, wherein $Ar_1$ is an imidazolyl group which may be substituted with a C1-6 alkyl group.

**4.** The compound or pharmacologically acceptable salt thereof according to claim 3, wherein $Ar_1$ is an imidazolyl group which may be substituted with a methyl group.

**5.** The compound or pharmacologically acceptable salt thereof according to claim 1, wherein $Ar_2$ is a phenyl group which may be substituted with 1 to 3 substituents selected from the group consisting of (1) a hydrogen atom, (2) a halogen atom, (3) a cyano group, (4) a C1-6 alkoxy group (wherein the C1-6 alkoxy group may be substituted with 1 to 3 substituents selected from a C2-6 alkenyl group, a C2-6 alkynyl group and a C3-8 cycloalkyl group), (5) a C2-6 alkenyloxy group and (6) a C2-6 alkynyloxy group.

**6.** The compound or pharmacologically acceptable salt thereof according to claim 5, wherein $Ar_2$ is a phenyl group which may be substituted with 1 to 3 substituents selected from the group consisting of (1) a hydrogen atom, (2) a halogen atom, (3) a cyano group and (4) a C1-6 alkoxy group.

**7.** The compound or pharmacologically acceptable salt thereof according to claim 6, wherein $Ar_2$ is a phenyl group which may be substituted with a C1-6 alkoxy group.

**8.** The compound or pharmacologically acceptable salt thereof according to claim 7, wherein $Ar_2$ is a phenyl group which may be substituted with a methoxy group.

**9.** The compound or pharmacologically acceptable salt thereof according to claim 1, wherein $X_1$ is a C1-6 alkylene group.

**10.** The compound or pharmacologically acceptable salt thereof according to claim 1, wherein $X_1$ is a C2-6 alkenylene group.

**11.** The compound or pharmacologically acceptable salt thereof according to claim 1, wherein $X_1$ is a single bond.

**12.** The compound or pharmacologically acceptable salt thereof according to claim 1, wherein $R^1$ and $R^2$ are the same or different and each represent a group selected from Substituent Group A4 shown below. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**13.** The compound or pharmacologically acceptable salt thereof according to claim 12, wherein $R^1$ and $R^2$ are the same

or different and each represent a group selected from Substituent Group A5 shown below. Substituent Group A5: (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A6, a 5- to 14-membered aromatic heterocyclic group which may be substituted with 1 to 3 substituents selected from Substituent Group A6, a 5-to 14-membered non-aromatic heterocyclic group which may be substituted with 1 to 3 substituents selected from Substituent Group A6 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A6 or a 5- to 14-membered aromatic heterocyclic group which may be substituted with 1 to 3 substituents selected from Substituent Group A6)), (3) a 6- to 14-membered aromatic hydrocarbon ring group which may be substituted with 1 to 3 substituents selected from Substituent Group A6, (4) a 5- to 14-membered aromatic heterocyclic group which may be substituted with 1 to 3 substituents selected from Substituent Group A6 and (5) -X-$A^2$ (wherein X and $A^2$ are as defined above). Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above).

**14.** The compound or pharmacologically acceptable salt thereof according to claim 13, wherein $R^1$ and $R^2$ are the same or different and each represent (1) a hydrogen atom or (2) a C1-6 alkyl group (wherein the C1-6 alkyl group is a hydrogen atom, a C3-8 cycloalkyl group, a C3-8 cycloalkoxy group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7 or -O-$A^4$ (wherein $A^4$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)).
Substituent Group A7: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A7, (9) -CO-$A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic heterocyclic group which may be substituted with 1 to 3 substituents selected from Substituent Group A7.

**15.** The compound or pharmacologically acceptable salt thereof according to claim 1, wherein $R^1$ and $R^2$, together with a nitrogen atom to which they are bonded, are a 5- to 11-membered heterocyclic group which may be substituted with 1 to 4 substituents selected from Substituent Group A4 shown below and is represented by the formula (II):

[Formula 3]

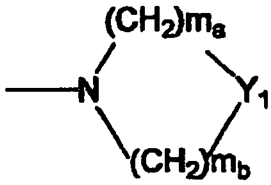

(II)

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ each represent a substituent selected from Substituent Group A4 shown below), (11) a single bond or (12) $>C=CR^{13}R^{14}$ (wherein $R^{13}$ and $R^{14}$ are the same or different and each represent a substituent selected from Substituent Group A4 shown below); and $m_a$ and $m_b$ are the same and different and each represent an integer of 0 to 4.

Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**16.** The compound or pharmacologically acceptable salt thereof according to claim 15, wherein the 5- to 11-membered heterocyclic group is a piperidinyl group, a pyrrolidinyl group, an azepinyl group, an azocanyl group, a piperazinyl group, a 1,4-diazepanyl group, a morpholinyl group or a thiomorpholinyl group.

**17.** The compound or pharmacologically acceptable salt thereof according to claim 16, wherein $R^1$ and $R^2$, together with a nitrogen atom to which are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 3 substituents selected from the group consisting of (1) a_hydrogen atom, (2) a halogen atom, (3) a hydroxyl group, (4) a formyl group, (5) a hydroxyimino group, (6) a C1-6 alkoxyimino group, (7) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A6 shown below and a 5- to 14-membered aromatic heterocyclic group which may be substituted with 1 to 3 substituents selected from Substituent Group A6 shown below), (8) a 6- to 14-membered aromatic hydrocarbon ring group which may be substituted with 1 to 3 substituents selected from Substituent Group A6 shown below, (9) a 5- to 14-membered aromatic heterocyclic group which may be substituted with 1 to 3 substituents selected from Substituent Group A6 shown below, (10) $-O-A^2$ (wherein $A^2$ represents a 6- to 14-membered aromatic hydrocarbon ring group which may be substituted with 1 to 3 substituents selected from Substituent Group A6 shown below or a 5- to 14-membered aromatic heterocyclic group which may be substituted with 1 to 3 substituents selected from Substituent Group A6 shown below), (11) $-CO-A^2$ (wherein $A^2$

is as defined above) and (12) =CH-$A^2$ (wherein $A^2$ is as defined above).

Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group
(wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above).

**18.** The compound or pharmacologically acceptable salt thereof according to claim 17, wherein $R^1$ and $R^2$, together with a nitrogen atom to which they are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 4 substituents selected from the group consisting of (1) a hydrogen atom, (2) a halogen atom, (3) a hydroxyl group, (4) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A8 shown below), (5) a 6- to 14-membered aromatic hydrocarbon ring group which may be substituted with 1 to 3 substituents selected from Substituent Group A8 shown below, (6) a 5- to 14-membered aromatic heterocyclic group which may be substituted with 1 to 3 substituents selected from Substituent Group A8 shown below, (7) -O-$A^6$ (wherein $A^6$ represents a 6- to 14-membered aromatic hydrocarbon ring group which may be substituted with 1 to 3 substituents selected from Substituent Group A8 shown below) and (8) =CH-$A^6$ (wherein $A^6$ is as defined above). Substituent Group A8: (1) a hydrogen atom, (2) a halogen atom, (3) a C1-6 alkyl group (wherein the C1-6 alkyl group may be substituted with 1 to 5 halogen atoms), (4) a C1-6 alkoxy group and (5) a 6- to 14-membered aromatic hydrocarbon ring group.

**19.** The compound or pharmacologically acceptable salt thereof according to claim 12, wherein $R^1$ is $-X_{21}-X_{22}-Ar_3$ (wherein $X_{21}$ represents 1) a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group) and a 5- to 14-membered non-aromatic heterocyclic group which may be substituted with 1 to 3 substituents selected from Substituent Group A6) or 2) a single bond; $X_{22}$ represents a single bond, an imino group which may be substituted with a substituent selected from Substituent Group A6, -O- or -S-; and $Ar_3$ represents a 6- to 14-membered aromatic hydrocarbon which may be substituted with 1 to 3 substituents selected from Substituent Group A6 or a 5- to 14-membered aromatic heterocyclic group which may be substituted with 1 to 3 substituents selected from Substituent Group A6).
Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with

1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above).

**20.** The compound or pharmacologically acceptable salt thereof according to claim 19, wherein $R^1$ is -$X_{21a}$-$X_{22a}$-$Ar_{3a}$ (wherein $X_{21a}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms) and a 5- to 14-membered non-aromatic heterocyclic group which may be substituted with 1 to 3 substituents selected from Substituent Group A7); $X_{22a}$ represents a single bond or an oxygen atom; and $Ar_{3a}$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7).
Substituent Group A7: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A7, (9) -CO-$A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic heterocyclic group which may be substituted with 1 to 3 substituents selected from Substituent Group A7.

**21.** The compound or pharmacologically acceptable salt thereof according to claim 20, wherein $Ar_{3a}$ is a 6-to 14-membered aromatic hydrocarbon ring group selected from the group consisting of a phenyl group, a naphthyl group and a fluorenyl group or a 5- to 14-membered aromatic heterocyclic group selected from the group consisting of a thienyl group, a pyridinyl group, a quinolinyl group, an isoquinolinyl group, an indolyl group, a benzothiazolyl group, a benzoxazolyl group and a furyl group, which may be substituted with 1 to 3 substituents selected from Substituent Group A7. Substituent Group A7: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A7, (9) -CO-$A^3$ (wherein $A^3$ represents a 6- to 14-membered aromatic hydrocarbon ring group which may be substituted with 1 to 3 substituents selected from Substituent Group A7), (10) a 6- to 14-membered aromatic hydrocarbon ring group which may be substituted with 1 to 3 substituents selected from Substituent Group A7 and (11) a 5- to 14-membered aromatic heterocyclic group which may be substituted with 1 to 3 substituents selected from Substituent Group A7.

**22.** The compound or pharmacologically acceptable salt thereof according to claim 1, wherein $R^1$ is a 6- to 14-membered non-aromatic hydrocarbon ring group or a 5-to 14-membered non-aromatic heterocyclic group represented by the formula (III):

[Formula 4]

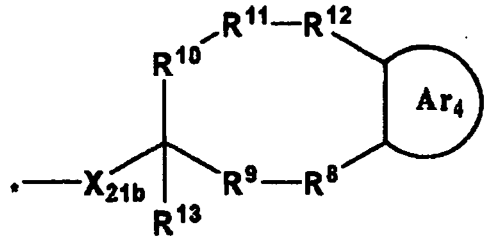

(III)

wherein $R^8$ to $R^{12}$ are the same or different and each represent 1) a single bond, 2) -CO-, 3) a methylene group which may be substituted with 1 or 2 substituents selected from Substituent Group A4, 4) -O-, 5) an imino group which may have a substituent selected from Substituent Group A4 or 6) -S-; $R^{13}$ represents a substituent selected from Substituent Group A9 shown below; $Ar_4$ represents a 6- to 14-membered aromatic hydrocarbon ring group which may be substituted with 1 to 3 substituents selected from Substituent Group A4 shown below or a 5- to 14-membered aromatic heterocyclic group which may be substituted with 1 to 3 substituents selected from Substituent Group A4 shown below; and $X_{21b}$ represents a C1-6 alkylene group. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

Substituent Group A9: (1) a hydrogen atom, (2) a halogen atom, (3) a hydroxyl group, (4) a cyano group, (5) a C3-8 cycloalkyl group, (6) a C2-6 alkenyl group, (7) a C2-6 alkynyl group, (8) a C2-6 alkenyloxy group, (9) a C2-6 alkynyloxy group, (10) a C3-8 cycloalkoxy group, (11) a C3-8 cycloalkylthio group, (12) a C1-6 alkylcarbonyl group, (13) a C1-6 alkylthio group, (14) a C1-6 alkylsulfinyl group, (15) a C3-8 cycloalkylsulfinyl group, (16) a C1-6 alkylsulfonyl group, (17) a C3-8 cycloalkylsulfonyl group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4 and (25) a 5- to 14-membered non-aromatic heterocyclic group which may be substituted with 1 to 3 substituents selected from Substituent Group A4.

**23.** The compound or pharmacologically acceptable salt thereof according to claim 22, wherein $Ar_4$ is a phenyl group or a 5- to 14-membered aromatic heterocyclic group selected from the group consisting of a pyridinyl group, a pyrimidinyl group, a pyrazinyl group, a thienyl group, an oxazolyl group, a pyrrolyl group, a thiazolyl group and a furyl group, which may be substituted with 1 to 3 substituents selected from the group consisting of a halogen atom, a C1-6 alkyl group (wherein the C1-6 alkyl group may be substituted with 1 to 3 substituents selected from the group

consisting of a halogen atom and a C1-6 alkyl group), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A6, a 5- to 14-membered aromatic heterocyclic group which may be substituted with 1 to 3 substituents selected from Substituent Group A6, a 5-to 14-membered non-aromatic heterocyclic group which may be substituted with 1 to 3 substituents selected from Substituent Group A6 and -CO-$A^2$ (wherein $A^2$ represents a 6- to 14-membered aromatic hydrocarbon ring group which may be substituted with 1 to 3 substituents selected from Substituent Group A6 shown below or a 5- to 14-membered aromatic heterocyclic group which may be substituted with 1 to 3 substituents selected from Substituent Group A6 shown below). Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above).

**24.** The compound or pharmacologically acceptable salt thereof according to claim 23, wherein $R^{13}$ is a phenyl group or a pyridinyl group which may be substituted with 1 to 3 substituents selected from Substituent Group A6.
Substituent Group A6: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group which may be substituted with 1 to 3 substituents selected from Substituent Group A6)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A6, (15) a 5- to 14-membered aromatic heterocyclic group which may be substituted with 1 to 3 substituents selected from Substituent Group A6, (16) a 5- to 14-membered non-aromatic heterocyclic group which may be substituted with 1 to 3 substituents selected from Substituent Group A6 and (17) -CO-$A^3$ (wherein $A^3$ is as defined above).

**25.** The compound or pharmacologically acceptable salt thereof according to claim 24, wherein $R^1$ is an indanyl group, an azaindanyl group, a tetrahydronaphthyl group, an azatetrahydronaphthyl group, a chromanyl group, an azachromanyl group, a tetrahydrobenzofuranyl group or a tetrahydrobenzothienyl group, which may be substituted with 1 to 3 substituents selected from the group consisting of (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 halogen atoms or C1-6 alkyl groups), (7) a C1-6 alkoxy group (wherein the C1-6 alkoxy group may be substituted with 1 to 3 halogen atoms), (8) an amino group (wherein the amino group may be substituted with a C1-6 alkyl group optionally having 1 to 5 halogen atoms) and (9) a 5- to 14-membered non-aromatic heterocyclic group.

**26.** The compound or pharmacologically acceptable salt thereof according to claim 1, wherein $R^3$ is a substituent selected from Substituent Group A4.

**27.** The compound or pharmacologically acceptable salt thereof according to claim 26, wherein $R^3$ is (1) a hydrogen atom or (2) C1-6 alkyl (wherein the C1-6 alkyl group may be substituted with a 6- to 14-membered aromatic hydrocarbon ring group which may be substituted with 1 to 3 substituents selected from Substituent Group A4 or a 5- to 14-membered aromatic heterocyclic group which may be substituted with 1 to 3 substituents selected from Substituent Group A4.

**28.** The compound or pharmacologically acceptable salt thereof according to claim 27, wherein $R^3$ is (1) a hydrogen atom or (2) C1-6 alkyl.

**29.** A medicine comprising the compound or pharmacologically acceptable salt thereof according to any one of claims 1 to 28 as an active ingredient.

**30.** A prophylactic or therapeutic agent for a disease caused by amyloid-β, comprising the compound or pharmacologically acceptable salt thereof according to any one of claims 1 to 28 as an active ingredient.

**31.** The prophylactic or therapeutic agent according to claim 30, wherein the disease caused by amyloid-β is Alzheimer's disease, dementia, Down's syndrome or amyloidosis.

**INTERNATIONAL SEARCH REPORT**

International application No.
PCT/JP2007/060187

A. CLASSIFICATION OF SUBJECT MATTER
*C07D233/64*(2006.01)i, *A61K31/4164*(2006.01)i, *A61K31/4178*(2006.01)i, *A61K31/454*(2006.01)i, *A61K31/513*(2006.01)i, *A61P25/28*(2006.01)i, *C07D249/06*
(2006.01)i, *C07D249/08*(2006.01)i, *C07D401/04*(2006.01)i,

B. FIELDS SEARCHED

Minimum documentation searched (classification system followed by classification symbols)
C07D233/64-C07D233/95, C07D249/06-C07D249/14, C07D401/04-C07D421/14, A61K31/4164-A61K31/554

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
REGISTRY/CAplus(STN), WPI

C. DOCUMENTS CONSIDERED TO BE RELEVANT

| Category* | Citation of document, with indication, where appropriate, of the relevant passages | Relevant to claim No. |
|---|---|---|
| X | JP 2001-508767 A (Fujisawa Pharmaceutical Co., Ltd.), 03 July, 2001 (03.07.01), Full text; particularly, Claims 1 to 3, 5 to 7; examples 4, 15, 18, 21, 37, 39 & WO 98/24785 A1 | 1-31 |
| X | JP 2005-526807 A (Glaxo Group Ltd.), 08 September, 2005 (08.09.05), Full text; particularly, Claims 1 to 9, 22, 26; Par. Nos. [0124] to [0126]; example 22 & WO 03/082292 A1 & EP 1487455 A1 | 1-31 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 July, 2007 (24.07.07) | 07 August, 2007 (07.08.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/060187

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2006-502247 A (Sanofi Aventis Deutschland GmbH),<br>19 January, 2006 (19.01.06),<br>Full text; particularly, Claims 1 to 3, 6; examples 1 to 6<br>& WO 2004/007455 A1 & EP 1523475 A1<br>& US 2004/0152743 A1 & US 2007/0021474 A1<br>& US 7138414 B2 & CN 1668593 A | 1-29<br>30,31 |
| X<br>A | JP 10-510512 A (Fujisawa Pharmaceutical Co., Ltd.),<br>13 October, 1998 (13.10.98),<br>Full text; particularly, Claims 1 to 4, 10, 11; examples 22, 23<br>& WO 96/10559 A1 & EP 784612 A1 | 1-29<br>30,31 |
| X<br>A | Varnes, J. G. et al. Discovery of N-propylurea 3-benzylpiperidines as selective CC chemokine receptor-3 (CCR3) antagonists. Bioorg. Med. Chem. Lett. 2004, Vol.14, No.7, pp.1645-1649. | 1-29<br>30,31 |
| X<br>A | Stark, H. et al. Search for novel leads for histamine H3-receptor antagonists: amine derivatives. Pharmazie 1997, Vol.52, No.6, pp.419-423. | 1-29<br>30,31 |
| X<br>A | Kajbaf, M. et al. Rapid and efficient p urification of cimetropium bromide and mifentidine drug metabolite mixtures derived from microsomal incubates for analysis by mass spectrometry. J. Chromatogr: Biomedical Applications, 1992, Vol.575, No.1, pp.75-85. | 1-28<br>29-31 |
| X<br>A | Marcus, S. L. et al. Stimulation of Rauscher leukemia virus DNA polymerase DNA-directed DNA synthesis by cationic trypanocides and polyamines. Cancer Res. 1985, Vol.45, No.1, pp.112-115. | 1-29<br>30,31 |
| X<br>A | Yale, H. L. et al. Substituted s-triazoles and related compounds. J. Med. Chem. 1966, Vol.9, No.1, pp.42-46. | 1-29<br>30,31 |

Form PCT/ISA/210 (continuation of second sheet) (April 2005)

**INTERNATIONAL SEARCH REPORT**

International application No.
PCT/JP2007/060187

Continuation of A. CLASSIFICATION OF SUBJECT MATTER
(International Patent Classification (IPC))

*C07D401/12*(2006.01)i, *C07D403/10*(2006.01)i, *C07D405/12*(2006.01)i

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KLEIN WL.** Alzheimer's disease-affected brain: Presence of oligomeric Aβ ligands (ADDLs) suggests a molecular basis for reversible memory loss. *Proceeding National Academy of Science USA,* 02 September 2003, vol. 100 (18), 10417-10422
- **NITSCH RM.** Antibodies against β-amyloid slow cognitive decline in Alzheimer's disease. *Neuron,* 22 May 2003, vol. 38, 547-554
- **JARRETT JT.** The carboxy terminus of the β amyloid protein is critical for the seeding of amyloid formation: Implications for the pathogenesis of Alzheimers' disease. *Biochemistry,* 1993, vol. 32 (18), 4693-4697
- **GLENNER GG.** Alzheimer's disease: initial report of the purification and characterization of a novel cerebrovascular amyloid protein. *Biochemical and biophysical research communications,* 16 May 1984, vol. 120 (3), 885-890
- **MASTERS CL.** Amyloid plaque core protein in Alzheimer disease and Down syndrome. *Proceding National Academy of Science USA,* June 1985, vol. 82 (12), 4245-4249
- **GOURAS GK.** Intraneuronal Aβ42 accumulation in human brain. *American Journal of Pathology,* January 2000, vol. 156 (1), 15-20
- **SCHEUNER D.** Secreted amyloid β-protein similar to that in the senile plaques of Alzheimer's disease is increased in vivo by the presenilin 1 and 2 and APP mutations linked to familial Alzheimer's disease. *Nature Medicine,* August 1996, vol. 2 (8), 864-870
- **FORMAN MS.** Differential effects of the swedish mutant amyloid precursor protein on β-amyloid accumulation and secretion in neurons and nonneuronal cells. *The Journal of Biological Chemistry,* 19 December 1997, vol. 272 (51), 32247-32253
- **SHEARMAN MS.** L-685,458, an Aspartyl Protease Transition State Mimic, Is a Potent Inhibitor of Amyloid β-Protein Precursor γ-Secretase Activity. *Biochemistry,* 01 August 2000, vol. 39 (30), 8698-8704
- **SHEARMAN MS.** Catalytic Site-Directed γ-Secretase Complex Inhibitors Do Not Discriminate Pharmacologically betweeen Notch S3 and β-APP Clevages. *Biochemistry,* 24 June 2003, vol. 42 (24), 7580-7586
- **LANZ TA.** Studies of Aβ pharmacodynamics in the brain, cerebrospinal fluid, and plasma in young (plaque-free) Tg2576 mice using the γ-secretase inhibitor N2-[(2S)-2-(3,5-difluorophenyl)-2-hydroxyethanoyl]-N1-[(7S)-5-methyl-6-oxo-6,7-dihydro-5H-dibenzo[b,d]azepin-7-yl]-L-alaninamide (LY-411575. *The journal of pharmacology and experimental therapeutics,* April 2004, vol. 309 (1), 49-55
- **WONG GT.** Chronic treatment with the γ-secretase inhibitor LY-411,575 inhibits β-amyloid peptide production and alters lymphopoiesis and intestinal cell differentiation. *The journal of biological chemistry,* 26 March 2004, vol. 279 (13), 12876-12882
- **KLEIN WL.** Alzheimer's disease-affected brain: Presence of oligomeric Aβ ligands (ADDLs) suggests a molecular basis for reversible memory loss. *Proceding National Academy of Science USA,* 02 September 2003, vol. 100 (18), 10417-10422
- **NITSCH RM.** Antibodies against β-amyloid slow cognitive decline in Alzheimer's disease. *Neuron,* 22 May 2003, vol. 38 (4), 547-554
- **JARRETT JT.** The carboxy terminus of the β amyloid protein is critical for the seeding of amyloid formation: Implications for the pathogenesis of Alzheimers' disease. *Biochemistry,* 11 May 1993, vol. 32 (18), 4693-4697
- **GLENNER GG.** Alzheimer's disease; initial report of the purification and characterization of a novel cerebrovascular amyloid protein. *Biochemical,* 16 May 1984, vol. 120 (3), 885-890
- **MASTERS CL.** Amyloid plaque core protein in Alzheimer disease and Down syndrome. *Proceeding National Academy of Science USA,* June 1985, vol. 82 (12), 4245-4249
- **GOURAS GK.** Intraneuronal Aβ42 accumulation in human brain. *American journal of pathology,* January 2000, vol. 156 (1), 15-20
- **FORMAN MS.** Differential effects of the swedish mutant amyloid precursor protein on β-amyloid accumulation and secretion in neurons and nonneuronal cells. *The journal of biological chemistry,* 19 December 1997, vol. 272 (51), 32247-32253
- **BLASS JP.** Brain metabolism and brain disease: Is metabolic deficiency the proximate cause of Alzheimer dementia?. *Journal of Neuroscience Research,* 01 December 2001, vol. 66 (5), 851-856

- **EVIN G.** Alternative transcripts of presenilin-1 associated with frontotemporal dementia. *Neuroreport,* 16 April 2002, vol. 13 (5), 719-723
- **YASUHARA O.** Accumulation of amyloid precursor protein in brain lesions of patients with Pick disease. *Neuroscience Letters,* 25 April 1994, vol. 171 (1-2), 63-66
- **TELLER JK.** Presence of soluble amyloid β-peptide precedes amyloid plaque formation in Down's syndrome. *Nature Medicine,* January 1996, vol. 2 (1), 93-95
- **TOKUDA T.** Plasma levels of amyloid β proteins Aβ1-40 and Aβ1-42(43) are elevated in Down's syndrome. *Annals of Neurology,* February 1997, vol. 41 (2), 271-273
- **HAYASHI Y.** Evidence for presenilin-1 involvement in amyloid angiopathy in the Alzheimer's disease-affected brain. *Brain Research,* 13 April 1998, vol. 789 (2), 307-314
- **BARELLI H.** Characterization of new polyclonal antibodies specific for 40 and 42 amino acid-long amyloid β peptides: their use to examine the cell biology of presenilins and the immunohistochemistry of sporadic Alzheimer's disease and cerebral amyloid angiopathy cases. *Molecular Medicine,* October 1997, vol. 3 (10), 695-707
- **CALHOUN ME.** Neuronal overexpression of mutant amyloid precursor protein results in prominent deposition of cerebrovascular amyloid. *Proceeding National Academy of Science USA,* 23 November 1999, vol. 96 (24), 14088-14093
- **DERMAUT B.** Cerebral amyloid angiopathy is a pathogenic lesion in Alzheimer's Disease due to a novel presenilin-1 mutation. *Brain,* December 2001, vol. 124 (12), 2383-2392
- **CRAS P.** Presenile Alzheimer dementia characterized by amyloid angiopathy and large amyloid core type senile plaques in the APP 692Ala-->Gly mutation. *Acta Neuropathologica(Berl,* September 1998, vol. 96 (3), 253-260
- **HERZIG MC.** Aβ is targeted to the vasculature in a mouse model of hereditary cerebral hemorrhage with amyloidosis. *Nature Neuroscience,* September 2004, vol. 7 (9), 954-960
- **VAN DUINEN SG.** Hereditary cerebral hemorrhage with amyloidosis in patients of Dutch origin is related to Alzheimer disease. *Proceeding National Academy of Science USA,* August 1987, vol. 84 (16), 5991-5994
- **LEVY E.** Mutation of the Alzheimer's disease amyloid gene in hereditary cerebral hemorrhage, Dutch type. *Science,* 01 June 1990, vol. 248 (4959), 1124-1126
- **LAWS SM.** Association between the presenilin-1 mutation Glu318Gly and complaints of memory impairment. *Neurobiology of Aging,* January 2002, vol. 23 (1), 55-58
- **VAUCHER E.** Object recognition memory and cholinergic parameters in mice expressing human presenilin 1 transgenes. *Experimental Neurology,* June 2002, vol. 175 (2), 398-406
- **MORGAN D.** Aβ peptide vaccination prevents memory loss in an animal model of Alzheimer's disease. *Nature,* 21 December 2000, vol. 408 (6815), 982-985
- **MORAN PM.** Age-related learning deficits in transgenic mice expressing the 751-amino acid isoform of human β-amyloid precursor protein. *Proceeding National Academy of Science USA,* 06 June 1995, vol. 92 (12), 5341-5345
- **LAWS SM.** Association between the presenilin-1 mutation Glu318Gly and complaints of memory impairment, Neurobiology of Aging. *Association between the presenilin-1 mutation Glu318Gly and complaints of memory impairment, Neurobiology of Aging,* January 2002, vol. 23 (1), 55-58
- **KOISTINAHO M.** β-amyloid precursor protein transgenic mice that harbor diffuse Aβ deposits but do not form plaques show increased ischemic vulnerability: Role of inflammation. *Proceeding National Academy of Science USA,* 05 February 2002, vol. 99 (3), 1610-1615
- **ZHANG F.** Increased susceptibility to ischemic brain damage in transgenic mice overexpressing the amyloid precursor protein. *The journal of neuroscience,* 15 October 1997, vol. 17 (20), 7655-7661
- **SADOWSKI M.** Links between the pathology of Alzheimer's disease and vascular dementia. *Neurochemical Research,* June 2004, vol. 29 (6), 1257-1266
- **O'RIORDAN S.** Presenilin-1 mutation(E280G), spastic paraparesis, and cranial MRI white-matter abnormalities. *Neurology,* 08 October 2002, vol. 59 (7), 1108-1110
- **GEHRMANN J.** Amyloid precursor protein (APP) expression in multiple sclerosis lesions. *Glia,* October 1995, vol. 15 (2), 141-51
- **REYNOLDS WF.** Myeloperoxidase polymorphism is associated with gender specific risk for Alzheimer's disease. *Experimental Neurology,* January 1999, vol. 155 (1), 31-41
- **SMITH DH.** Protein accumulation in traumatic brain injury. *NeuroMolecular Medicine,* 2003, vol. 4 (1-2), 59-72
- **MATSUBARA-TSUTSUI M.** Molecular evidence of presenilin 1 mutation in familial early onset dementia. *American journal of Medical Genetics,* 08 April 2002, vol. 114 (3), 292-298
- **KIRKITADZE MD.** Paradigm shifts in Alzheimer's disease and other neurodegenerative disorders: the emerging role of oligomeric assemblies. *Journal of Neuroscience Research,* 01 September 2002, vol. 69 (5), 567-577

- **EVERT BO.** Inflammatory genes are upreglulated in expanded ataxin-3-expressing cell lines and spinocerebellar ataxia type 3 brains. *The Journal of Neuroscience,* 01 August 2001, vol. 21 (15), 5389-5396
- **MANN DM.** Deposition of amyloid(A4) protein within the brains of persons with dementing disorders other than Alzheimer's disease and Down's syndrome. *Neuroscience Letters,* 05 February 1990, vol. 109 (1-2), 68-75
- **PRIMAVERA J.** Brain accumulation of amyloid-β in Non-Alzheimer Neurodegeneration. *Jornal of Alzheimer's Disease,* October 1999, vol. 1 (3), 183-193
- **GIASSON BI.** Interactions of amyloidogenic proteins. *NeuroMolecular Medicine,* 2003, vol. 4 (1-2), 49-58
- **MASLIAH E.** β-amyloid peptides enhance α-synuclein accumulation and neuronal deficits in a trancgenic mouse model linking Alzheimer's disease and Parkinson's disease. *Proceeding National Academy of Science USA,* 09 October 2001, vol. 98 (21), 12245-12250
- **BARRACHINA M.** Amyloid-β deposition in the cerebral cortex in Dementia with Lewy bodies is accompanied by a relative increase in AβPP mRNA isoforms containing the Kunitz protease inhibitor. *Neurochemistry International,* February 2005, vol. 46 (3), 253-260
- **SCHMIDT ML.** Amyloid plaques in Guam amyotrophic lateral sclerosis/ parkinsonism-dementia complex contain species of Aβ similar to those found in the amyloid plaques of Alzheimer's disease and pathological aging. *Acta Neuropathologica (Berl,* February 1998, vol. 95 (2), 117-122
- **ITO H.** Demonstration of β amyloid protein-containing neurofibrillary tangles in parkinsonism-dementia complex on Guam. *Neuropathology and applied neurobiology,* October 1991, vol. 17 (5), 365-373
- **ROSSO SM.** Coexistent tau andamyloid pathology in hereditary frontotemporal dementia with tau mutations. *Annals of the New York academy of sciences,* 2000, vol. 920, 115-119
- **TOLNAY M.** Low amyloid(Aβ) plaque load and relative predominance of diffuse plaques distinguish argyrophilic grain disease from Alzheimer's disease. *Neuropathology and applied neurobiology,* August 1999, vol. 25 (4), 295-305
- **JIN LW.** Intracellular accumulation of amyloidogenic fragments of amyloid-β precursor protein in neurons with Niemann-Pick type C defects is associated with endosomal abnormalities. *American Journal-of Pathology,* March 2004, vol. 164 (3), 975-985
- **SASAKI S.** Immunoreactivity of β-amyloid precursor protein in amyotrophic lateral sclerosis. *Acta Neuropathologica(Berl,* May 1999, vol. 97 (5), 463-468
- **TAMAOKA A.** Increased amyloid β protein in the skin of patients with amyotrophic lateral sclerosis. *Journal of neurology,* August 2000, vol. 247 (8), 633-635
- **HAMILTON RL.** Alzheimer disease pathology in amyotrophic lateral sclerosis. *Acta Neuropathologica,* June 2004, vol. 107 (6), 515-522
- **TURNER BJ.** Brain P-amyloidaccumulation in transgenic mice expressing mutant superoxide dismutase 1. *Neurochemical Research,* December 2004, vol. 29 (12), 2281-2286
- **WELLER RO.** Pathology of cerebrospinal fluid and interstitial fluid of the CNS: Significance for Alzheimer disease, prion disorders and multiple sclerosis. *Journal of Neuropathology and Experimental Neurology,* October 1998, vol. 57 (10), 885-894
- **SILVERBERG GD.** Alzheimer's disease, normal-pressure hydrocephalus, and senescent changes in CSF circulatory physiology: a hypothesis. *Lancet neurology,* August 2003, vol. 2 (8), 506-511
- **WELLER RO.** Cerebral amyloid angiopathy: Accumulation of Aβ in interstitial fluid drainage pathways in Alzheimer's disease. *Annals of the New York academy of sciences,* April 2000, vol. 903, 110-117
- **YOW HY.** A role for cerebrovascular disease in determining the pattern of β-amyloid deposition in Alzheimer's disease. *Neurology and applied neurobiology,* 2002, vol. 28, 149
- **WELLER RO.** Cerebrovasculardisease is a major factor in the failure of elimination of Aβ from the aging human brain. *Annals of the New York academy of sciences,* November 2002, vol. 977, 162-168
- **SMITH MJ.** Variable phenotype of Alzheimer's disease with spastic paraparesis. *Annals of Neurology,* 2001, vol. 49 (1), 125-129
- **CROOK R.** A variant of Alzheimer's disease with spastic pararesis and unusual plaques due to deletion of exon 9 of presenilin 1. *Nature Medicine,* April 1998, vol. 4 (4), 452-455
- **ATWOOD CS.** Cerebrovascular requirement for sealant, anti-coagulant and remodeling molecules that allow for the maintenance of vascular integrity and blood supply. *Brain Research Reviews,* September 2003, vol. 43 (1), 164-78
- **LOWENSON JD.** Protein aging: Extracellular amyloid formation and intracellular repair. *Trends in cardiovascular medicine,* 1994, vol. 4 (1), 3-8
- **SINGLETON AB.** Pathology of early-onset Alzheimer's disease cases bearing the Thr113-114ins presenilin-1 mutation. *Brain,* December 2000, vol. 123 (12), 2467-2474
- **GATTAZ WF.** Platelet phospholipase A2 activity in Alzheimer's disease and mild cognitive impairment. *Journal of Neural Transmission,* May 2004, vol. 111 (5), 591-601

- **ASSINI A.** Plasma levels of amyloid β-protein 42 are increased in women with mild cognitive impariment. *Neurology,* 14 September 2004, vol. 63 (5), 828-831
- **DE MEYER GR.** Platelet phagocytosis and processing of β-amyloid precursor protein as a mechanism of macrophage activation in atherosclerosis. *Circulation Research,* 14 June 2002, vol. 90 (11), 1197-1204
- Shin Jikken Kagaku Koza. Yuki Gosei. Maruzen Co., Ltd, August 2005, vol. 14, 428-429
- **O. MITSUNOBU.** *Synthesis,* 1981, 1
- **M.M.S. GIBSON et al.** *Angew. Chem.,* 1968, vol. 80, 986
- Shin Jikken Kagaku Koza. Yuki Kagobutsu No Gosei To Hannou. Maruzen Co., Ltd, February 1978, vol. 14, 1333-1341
- Shin Jikken Kagaku Koza. Yuki Kagobutsu No Gosei To Hannou. Maruzen Co., Ltd, February 1978, vol. 14, 1380-1384
- Shin Jikken Kagaku Koza. Yuki Gosei. Maruzen Co., Ltd, August 2005, vol. 14, 1-49
- Shin Jikken Kagaku Koza. Yuki Gosei. Maruzen Co., Ltd, August 2005, vol. 14, 417-419
- Shin Jikken Kagaku Koza. Yuki Gosei. Maruzen Co., Ltd, June 1992, vol. 19, 438-446
- Shin Jikken Kagaku Koza. Yuki Gosei. Maruzen Co., Ltd, August 2005, vol. 14, 480-487
- **BAILEY, W.** *Org. Synth.,* vol. 81 (204), 121
- **D.D. DAVEY et al.** *J. Med. Chem.,* 1991, vol. 39, 2671-2677
- **S.S. CHANDRAN et al.** *Bioorg. Med. Chem. Lett.,* 2001, vol. 11, 1493-1496
- **T. OKANO et al.** *Bull. Chem. Soc. Jpn.,* 1994, vol. 67, 2329-2332
- Shin Jikken Kagaku Koza. Yuki Gosei. Maruzen Co., Ltd, February 1991, vol. 21, 2-23196-240
- Shin Jikken Kagaku Koza. Yuki Gosei. Maruzen Co., Ltd, February 1991, vol. 21, 83-85289-298
- Shin Jikken Kagaku Koza. Yuki Gosei. Maruzen Co., Ltd, June 1992, vol. 19, 363-482
- **S.L. BUCHWALD et al.** *Tetrahedron Lett.,* 1997, vol. 38, 6367-6370
- **J.F. HARTWIG et al.** *J. Am. Chem. Soc.,* 1998, vol. 120, 827-828
- **T.W. GREEN.** Protective Groups in Organic Synthesis. John Wiley & Sons, Inc, 1981
- Shin Jikken Kagaku Koza. Yuki Kagobutsu No Gosei To Hannou. Maruzen Co., Ltd, November 1977, vol. 14, 354-360
- Shin Jikken Kagaku Koza. Yuki Kagobutsu No Gosei To Hannou. Maruzen Co., Ltd, February 1978, vol. 14, 1261-1300
- **K. SAKAI et al.** *Bull. Chem. Soc. Jpn.,* 1986, vol. 59, 179-183
- Shin Jikken Kagaku Koza. Yuki Gosei. Maruzen Co., Ltd, August 2005, vol. 14, 537-542
- **K. NINOMIYA et al.** *Tetrahedron,* 1974, vol. 30, 2151
- **HANESSIAN, S. et al.** *Tetrahedron Letters,* 2000, vol. 41, 4999-5003
- **K. KURITA et al.** *Org. Synth.,* 1988, vol. VI, 715
- **KNOELKER, H.-J. et al.** *Synlett,* 1997, vol. 8, 925-928
- Shin Jikken Kagaku Koza. Yuki Gosei. Maruzen Co., Ltd, November 1992, vol. 22, 6-11
- Shin Jikken Kagaku Koza. Yuki Gosei. Maruzen Co., Ltd, November 1992, vol. 22, 1-5
- Jikken Kagaku Koza. Yuki Gosei. Maruzen Co., Ltd, June 1992, vol. 19, 57-85
- **THAVONEKHAM, B. et al.** *Synthesis,* 1997, vol. 10, 1189-1194
- **RAJU, B. et al.** *Bioorg. Med. Chem. Lett.,* vol. 8, 3043-3048
- **ATWAL, K.S. et al.** *J. Med. Chem.,* 1996, vol. 39, 304-313
- **SANTILLI, A.** *J. Org. Chem.,* 1966, vol. 31, 4268